(19) Europäisches Patentamt
European Patent Office
Office européen des brevets

(11) EP 3 394 284 B1

(12) EUROPEAN PATENT SPECIFICATION

(45) Date of publication and mention of the grant of the patent:
**12.02.2020 Bulletin 2020/07**

(21) Application number: **16825747.5**

(22) Date of filing: **22.12.2016**

(51) Int Cl.:
***C12Q 1/32*** *(2006.01)* ***G01N 33/68*** *(2006.01)*

(86) International application number:
**PCT/EP2016/082332**

(87) International publication number:
**WO 2017/109046 (29.06.2017 Gazette 2017/26)**

(54) **ENZYMATIC METHOD FOR THE EVALUATION OF XYLOSE**

ENZYMATISCHES VERFAHREN ZUR BEURTEILUNG VON XYLOSE

PROCÉDÉ ENZYMATIQUE POUR L'ÉVALUATION DE XYLOSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2015 US 201562270825 P**

(43) Date of publication of application:
**31.10.2018 Bulletin 2018/44**

(73) Proprietor: **Venter Pharma, S.L**
**28109 Madrid (ES)**

(72) Inventors:
- **FERNÁNDEZ MAYORALAS, Alfonso**
  **Madrid 28006 (ES)**
- **GARCÍA JUNCEDA, Eduardo**
  **28006 Madrid (ES)**
- **HERMIDA DIAZ, Carmen**
  **28109 Madrid (ES)**
- **MARTÍN MARTÍN, José Luis**
  **28109 Madrid (ES)**
- **SÁNCHEZ MORENO, Israel**
  **28109 Madrid (ES)**
- **MONSALVE HERNANDO, Carmen**
  **28109 Madrid (ES)**

(74) Representative: **Manuel Illescas y Asociados, S.L.U.**
**C/ Príncipe de Vergara 197, Oficina 1°A**
**28002 Madrid (ES)**

(56) References cited:
**US-A1- 2015 338 421**

- **LIANG LI ET AL: "A selective and sensitive d-xylose electrochemical biosensor based on xylose dehydrogenase displayed on the surface of bacteria and multi-walled carbon nanotubes modified electrode", BIOSENSORS AND BIOELECTRONICS, vol. 33, no. 1, 1 March 2012 (2012-03-01), pages 100-105, XP055354534, NL ISSN: 0956-5663, DOI: 10.1016/j.bios.2011.12.027**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### TECHNICAL FIELD OF THE INVENTION

The present invention relates to an *in vitro* enzymatic method for detection of D-xylose, particularly to be used after the *in vivo* evaluation of intestinal lactase activity following oral administration of Gaxilose, specifically in intestinal lactase deficiency (hypolactasia) in humans. The method of invention is based on the use of the enzyme xylose dehydrogenase (XylB) from *Caulobacter crescentus* NA1000.

### BACKGROUND OF THE INVENTION

Intestinal lactase is the enzyme responsible for lactose digestion. Lactase is located in the microvilli, which constitute the so-called brush border of the enterocytes of the small intestine. It is an integral protein of the cell membrane with its active centre directed towards the lumen of the small intestine, and its activity varies along the length of the intestine, the highest activity being found in the middle jejunum (Newcomer AD, 1966; Triadou N, 1983). Lactose is not absorbed like other disaccharides through the intestinal mucosa but must be hydrolysed by lactase into its components, galactose and glucose, which are then absorbed, this enzyme accounting for all intestinal lactase activity (Semenza G, 2006). The hydrolysis of this disaccharide is the rate limiting step in the digestion and use of lactose (Dawson DJ, 1986).

It has been known for decades that disaccharidase activities and some other intestinal enzymes and proteins vary as a consequence of intestinal mucosa damage. Some of the follow-up markers of the integrity of enterocyte cells are the alkaline phosphatase, disaccharidases (lactase, maltase and sucrase), peptidases, gamma-glutamyltransferases, glucose and fructose transporters, villin, annexins and some others. The disaccharidases are the enzymes responsible for the final step of carbohydrate digestion. Determination of lactase, maltase and sucrase enzymatic activities is usually used as marker in studies of intestinal epithelial differentiation (He Y 1993; Sato N, 1999; Fan MZ, 2001). On the other hand, it is known that intestinal lactase is the first disaccharidase to be affected and the last one to be recovered (Semenza G, 2006). This agrees with the information reported in the literature about the possibility of using lactase activity variation as a marker of the level of intestinal mucosa recovery in coeliac patients (Nieminen U, 2001; Parfenov AI, 2015). In some other cases, such as in intestinal ischemia, it has been described that alkaline phosphatase as well as intestinal lactase are missing immediately after the damage and then recovered once the ischemia is reversed (Hinnebusch BF, 2002).

If there is a lack of lactase (called hypolactasia or lactase deficiency), lactose is not hydrolysed in the small intestine, inducing a reduction in gastric emptying speed, increase in intestinal transit time, increase in osmotic pressure and retention of fluid in the intestinal cavity, giving rise to bacterial fermentation of this fluid in the colon and to the production of gasses such as hydrogen, methane and carbon dioxide and to the formation of short chain fatty acids. As a consequence of these effects, the digestion process is reduced and absorption of monosaccharides falls, giving rise to pain and abdominal cramps, flatulence, audible intestinal noise and diarrhoea. In the adult, this set of symptoms is known as lactose intolerance and is a very common genetic disorder, affecting more than a half of the human population. In the newborn, congenital deficiency of the enzyme prevents proper utilisation of lactose resulting in severe disorders such as intense diarrhoea and dehydration, derived both from the reduction of energy input and the intestinal accumulation of non-hydrolysed disaccharide, which requires early detection and a change in diet to lactose-free milk. Lactase deficiency also occurs in a secondary way in a significant number of intestinal pathologies that are accompanied by various degrees of degradation of the intestinal mucosa including celiac disease, inflammatory chronic intestinal disease (Crohn's disease and ulcerative colitis), irritable bowel syndrome, intestinal resection, cystic fibrosis, premature infants, chemotherapy treatments or as an additional disorder of old age. The evaluation of lactase activity is therefore of particular interest in gastroenterology, paediatrics and generally in pathological processes where functional integrity of intestinal mucosa or a differential diagnosis with the deficiency of this enzyme must be assessed.

Two classes of methodologies have been used in the state of the art for the diagnosis of intestinal lactase deficiency or lactose intolerance, which have a series of disadvantages that in general imply poor reliability and serious discomfort for patients and also require specialised equipment:

- Direct determination of lactase activity in a sample of intestinal mucosa obtained by biopsy using endoscopy (Newcomer AD, 1966; Semenza G, 2006; Arola H, 1994). This is an invasive method, which only indicates enzyme activity in a specific part or area of the intestine, which is generally not the area of highest occurrence (middle jejunum), as it is very difficult to take biopsies in this area. The result varies from one sample to another, therefore does not provide information about the total enzyme activity in an individual. A poor correlation has been observed between clinical symptoms of lactose intolerance and lactase activity measured in intestinal mucosa.

- Indirect determination, evaluating the metabolic consequences of enzyme deficiency after the oral lactose overload test (administration of 1-2 g of lactose per kg of body weight up to a maximum of 50 g) such as the appearance of symptoms in the subject (abdominal pain, flatulence, diarrhoea, etc.); the determination either in blood or urine of glucose or galactose levels (McGill DB, 1967; Newcomer AD, 1975); determination of the products of lactose hydrolysis by lactase, either by measurement of gasses in the breath such as, for example, $H_2$ (Arola H, 1994; Newcomer AD, 1975; Levitt MD, 1969; Metz G, 1975) or exhaled $CO_2$ (Newcomer AD, 1975; Koetse HA, 1999; Sasaki Y, 1970). The main problems with these types of indirect tests derive from the significant degree of accompanying digestive discomfort, as it is necessary to administer an oral overload of lactose in order to carry them out, this being particularly severe in infants. Also, as these are indirect tests, they do not enable the evaluation of total enzyme activity in an individual but depend on endogenous production capacity of gasses such as $H_2$ and $CO_2$ by the subject. In addition to being variable from one individual to another, this is affected by various factors beyond the quantity or activity of lactase enzyme such as smoking habit, type of diet and prior exercise, emotional state, diabetes, use of antibiotics. It must also be taken into account that there is a significant percentage of population that can be hypo or hiper hydrogen producers so there is a high proportion of false positives and false negatives, resulting in poor test reliability. Various studies have demonstrated poor correlation between clinical symptoms of lactose intolerance and the data provided by these types of tests (Davidson GP, 1985; Lifshitz CH, 1985).

In summary, indirect methods, although non-invasive, suffer from three fundamental problems:

1. serious accompanying discomfort to patients with lactase deficiency due to the high lactose doses that must be ingested in all cases;

2. need for special and unusual equipment which is not always available in all health centres;

3. relative frequency of false positives and false negatives.

These drawbacks result in diagnoses of lactase deficiency being made much less frequently than the high level of incidence in the population would lead one to expect.

For these reasons, new diagnostic methods involving little discomfort, simplicity of application and greater reliability are being developed. These are methods for the evaluation of intestinal lactase activity based on the use of specific disaccharides, structural analogues of lactose, which can function as substrates for the enzyme, and which once ingested, are transformed by the action of intestinal lactase into certain monosaccharides that are absorbed by intestinal mucosa and can be determined in the blood or urine.

Spanish patents ES478590 and ES482073 disclose methods based on the evaluation of intestinal lactase activity by oral administration of 3-O-methyl-lactose, a structural analogue of lactose, and the determination of 3-O-methyl-D-glucose in the urine. But these types of analogues have not been used in clinical practice as they imply the absorption into the bloodstream of a non-physiological compound, such as 3-O-methyl-D-glucose, and require gas chromatography or high-pressure liquid chromatography systems for the determination.

Spanish patent ES2023556 discloses the preparation of the disaccharide, 4-O-β-galactopyranosyl-D-xylose (4-GX or Gaxilose), for the evaluation of intestinal lactase activity. This disaccharide is administered orally and acts as a substrate of intestinal lactase, being hydrolysed in the intestinal tract into D-xylose and galactose (**Figure 1**). Both compounds are absorbed and a substantial part of D-xylose is eliminated in the urine where it can be directly determined by a colorimetric method. The quantity of D-xylose excreted in the urine is correlated with the levels of intestinal lactase.

Spanish patent ES2100131 describes enzymatic processes for the preparation of mixtures of galactopyranosyl-xylose disaccharides containing Gaxilose and its regioisomers, 2-O-β-galactopyranosyl-D-xylose and 3-O-β-galactopyranosyl-D-xylose, and the use of a mixture of these three regioisomers in the evaluation of intestinal lactase activity to determine the eliminated D-xylose by colorimetric analysis based on reaction with phloroglucinol and using basal urine as the blank.

Similarly, Spanish patent ES2182703 discloses an enzymatic process for obtaining Gaxilose that involves an enzymatic reaction between D-xylose and a substrate, β-D-xylopyranoside, and a subsequent phase of isolation and purification of Gaxilose. The use of Gaxilose in the preparation of compositions and solutions useful for the evaluation of intestinal lactase in humans is also described.

In the same way, Spanish patent ES2208099 discloses the use of Gaxilose in humans for the evaluation of intestinal lactase as a non-invasive diagnostic test for the deficiency of this enzyme. This patent shows that the excretion of D-xylose in urine is dependent on the dose of oral administration of Gaxilose and uses a colorimetric evaluation of D-xylose.

The colorimetric method for detection of D-xylose in biological samples (blood and urine) is based on the chemical reagent phloroglucinol, which forms a red colored complex with pentoses in an acid medium. Thus, color intensity is directly proportional to the D-xylose present in the sample.

Colorimetric reaction with phloroglucinol has been proved to be a sensitive and reliable method for detection of D-xylose in biological samples (Hermida C, 2014). However, along the implantation of this method in several clinical laboratories, some limitations have been found: the phloroglucinol reaction requires some irritating reagents (acetic and hydrochloric acids), specific laboratory instrumental such as precision balance and spectrophotometer, as well as qualified personal to carry out the reaction. These requirements could not be achieved in many clinical laboratories where the automated detection systems have become the main diagnosis method.

Nowadays, several metabolite detection methods are based on enzymatic reactions, which have been successfully adapted to automated analyzers. An attractive candidate for enzymatic detection of D-xylose is the family of the xylose dehydrogenases (XDHs). XDH enzymes catalyze the oxidation of xylose to yield xylonolactone. This reaction requires a concomitant reduction of a cofactor molecule, which can be $NAD^+$ or $NADP^+$ (**Figure 2**).

The base of this methodology is simple. A NADH molecule is synthesized per each consumed xylose molecule. As NADH can be detected and quantified by spectrophotometry at 340 nm (its molar extinction coefficient at this wavelength $\varepsilon^{340nm}$ is 6.22 $mM^{-1}cm^{-1}$), the amount of xylose consumed in a specific sample can be calculated.

XDHs are involved in the metabolism of xylose in some organisms, but pathways involving this enzyme differ from classical xylose utilization mechanisms in most of the organisms. XDHs can be classified in two types, according to the stereochemistry of the sugar substrate: D-xylose dehydrogenases (D-XDH) and L-xylose dehydrogenases (L-XDH). To the date, only one L-XDH (EC.1.1.1.113) has been identified, from the yeast *Saccharomyces cerevisiae* (Uehara K, 1962).

On the other hand, many D-XDH enzymes have been identified and studied in the last decades from different organisms: mammals (pig liver, monkey kidney. Aoki S, 2001; Asada Y, 2000), bacteria (*Arthrobacter nicotinovorans*), fungus (*Hypocrea jecorina*), and halophilic achaeon (*Haloarcula marismorui, Halofera volcanii*). Mihasan M, 2013; Berghaell S, 2007; Johnsen U, 2004; Johnsen U, 2009).

Most of them display their highest activities using $NADP^+$ as cofactor, but they are also able to utilize $NAD^+$ at acceptable activity ratios. In the same way, even though D-xylose is the main substrate for all of them, they have also showed moderate to good activities with other sugars, especially D-glucose. This fact would represent an important drawback for detection of D-xylose using D-XDH, due to the presence of significant concentrations of D-glucose in biological samples such as blood, which would interfere in the determination. Interestingly, a subgroup of D-XDH enzymes which display higher substrate specificity has also been described: the XylB enzymes group (EC.1.1.1.175). Three of these enzymes have been identified in the microorganisms *Pseudomona sp, Arthrobacter sp* and *C. crescentus* but only the last one has been isolated and intensely characterized (Stephens C, 2007; Toivari M, 2012).

XylB enzymes are much more specific for both cofactor and substrate. In fact, they are absolutely dependent on $NAD^+$ as cofactor, showing no activity when $NADP^+$ is used instead. Regarding the substrate, XylB from *C. crescentus* is highly specific for D-xylose; it is able to oxidate other sugars like D-glucose and D-arabinose, but they display a considerably lower activity than with D-xylose. Therefore, present invention is based on the use of the enzyme XylB from *C. crescentus* in an automatic new enzymatic method for specific D-xylose detection, that could replace the manual methods which use phloroglucinol, but without compromising detection accuracy. This enzyme catalyzes D-xylose oxidation to D-xylonolactone, using $NAD^+$ as cofactor.

In the last years, some D-xylose detection methods involving XylB from *C. crescentus* have been described. The enzyme has been expressed in *Escherichia coli* cells as a recombinant protein fused to the outer membrane protein INP (ice nucleation protein) (Liang B, 2012). This XylB cell-surface displaying system has been successfully used in sensitive and specific spectrophotometric D-xylose quantification by indirect measurement of the NADH formed in the enzymatic reaction. This system with XylB displayed in *Escherichia coli* surface has also been employed to build selective D-xylose biosensors by immobilization of the engineered cells on multi-walled carbon nanotube electrodes. (Li L, 2012). This electrode was subsequently improved by co-immobilization of a glucose dehydrogenase (GOD) for simultaneous voltammetric detection of D-xylose and D-glucose (Li L, 2013).

Detection of D-xylose is not the only function where XDHs have been employed. There are several examples in literature about the use of some of these enzymes in genetically modified microorganism for microbial D-xylonate, butanetriol and ethanol production using the non-food hemicelluloses hydrolysate as D-xylose source (**Figure 3**) (Toivari MH, 2012b, Valdehuesa KN, 2014). The D-XDH from *Hypocrea jecorina* has been used to modify the D-xylose metabolic pathway in engineered *S. cerevisiae* (Toivari MH, 2010) and *Kluyveromices lactis* cells (Nygård Y, 2011) in order to produce and accumulate D-xylonate. The same strategy was carried out employing XylB from *C. crescentus.* As this enzyme is more specific and efficient in D-xylose oxidation, final amounts of obtained D-xylonate were considerably improved in the modified organisms: *S. cerevisiae* (Toivari M, 2012); *Pichia kudriarzevii* (Toivari MH, 2013) and *Escherichia coli* (Liu M, 2012).

Production of D-xylonate in these XylB-modified microorganisms has been recently improved by addition of the lactonase XylC, also from *C. crescentus,* which increases the D-xylonolactone transformation (Nygård Y, 2014; Cao Y, 2013). A different version of this genetically modified bacteria has been also described for 1,2,4-butanotriol production in *Escherichia coli* (Valdehuesa KN, 2014). Production of butanotriol has also been achieved in the plant *Arabidopsis*

*taliana* genetically modified by introduction of XylB from *C. crescentus* (Abdel-Ghany SE, 2013). In addition, a XylB surface displayed bacteria constructed in an engineered *Escherichia coli* has been shown as an enzymatic biofuel cell, due to its capacity to produce ethanol from D-xylose (Xia L, 2013). There are several examples in literature about the use of XDHs in genetically modified microorganism for microbial D-xylonate, butanetriol and ethanol production using the non-food hemicelluloses hydrolysate as D-xylose source, however, the use of isolated enzymes, can reduce some of the problems associated to whole-cell catalysts, such as the diffusion limitations or the isolation and purification of final products.

There are also some additional applications of D-xylonic:
D-Xylonate has found applications as cement dispersant (Chun BW, 2006), for decreasing acrylamide contained in the food after cooking (Tornoda Y, 2004; US patent Appln. 10/742,666), in the production of copolyamides (Zamora F, 2000) and as a precursor for 1,2,4-butanetriol synthesis (Niu W, 2014). Recently, D-xylonic acid has been used as catalyst in a three-component reaction for the synthesis of pirrole and xanthene derivatives with potential pharmaceutical properties (Ma J, 2016). The enzyme XylB of *C. crescentus* used in the present invention can be used in the production of D-xylonate in a process and allows conversion of D-xylose of almost 100%. As the substrate and product of the enzyme, used for cofactor regeneration are volatile compounds, the purification of D-xylonate would only require a vacuum evaporation (rotary evaporator) or product crystallization.

The object of the present invention is to provide an *in vitro* enzymatic method for D-xylose quantification with the use of a xylose dehydrogenase, particularly the XylB from *C. crescentus* NA1000.

For the purposes of present patent description, the following terms should all be taken as synonyms: evaluation, quantification, determination, or detection.

Detection of D-xylose in biological samples (urine and blood) is currently carried out using a colorimetric method based on the chemical reagent phloroglucinol. The present invention provides an alternative sensitive and specific method for D-xylose quantification in urine and/or blood that can replace the colorimetric method without compromising product accuracy using the enzyme XylB from *C. crescentus* NA 1000. For the purposes of present patent specification, the term "blood" should be taken in its broadest meaning and it would comprise, as a way of example, analogous terms as plasma, blood serum, plasma serum, etc...

The method for evaluation of hypolactasia involves the ingestion of disaccharide 4-O-β-galactopyranosyl-D-xylose (4-GX or Gaxilose) and *in vitro* analysis of D-xylose produced by the activity of intestinal lactase, using a single XylB enzyme that can detect low levels of D-xylose. Present invention, therefore, does not need any other enzyme coupled to, or acting sequentially or simultaneously, or linked to the enzyme xylose dehydrogenase. The presence in the assay of a single enzyme offers multiple advantages: best manner of controlling enzyme activity by a single substrate, more specificity, less troubleshooting due to the different features (optimal pH, temperature, cofactors, etc...) inherent to each enzyme intervening in the assay. By means of the data shown in the present invention compared to the data obtained by the commercial phloroglucinol colorimetric test (Xylose Urine Kit, Ref. 7152 FAR Diagnostics), it is concluded that the new method of the invention is more specific for the diagnosis of hypolactasia, since unspecific background in the D-xylose detection was considerable reduced when the enzymatic method was employed. This fact was already known in clinical trials phase I and Ib when xylose cut-off detected with phloroglucinol was calculated and the doses of Gaxilose significantly different from placebo were chosen.

These results disclosed herein indicate that the enzymatic method is more accurate because of its lower unspecific background that avoids overestimation of concentration values. However, both the phloroglucinol and the enzymatic method can be indistinctly used to classify a patient as "normolactasic" or "hypolactasic" after LacTEST 0.45 g intake. LacTEST 0.45 g is a diagnostic drug for *in vivo* evaluation of hypolactasia. It is based on the oral administration of 0.45 g of Gaxilose. Gaxilose is hydrolysed by intestinal lactase, and the produced D-xylose is detected in urine. Urine is collected during 5 hours after the administration of Gaxilose. The total amount of D-xylose in excreted urine is a reflection of intestinal lactase activity. Along the present description, the terms "LacTEST®" or "Gaxilose test" will be used as synonyms or equivalents.

Additionally, the method of invention implies a simpler system which can be used in automatic detection systems or auto-analyzer devices. In addition, the method of invention avoids the use of aggressive reagents, so can be run by less qualified operators.

Finally, the evolution of xylose amount detected by the method of the invention after the administration of LacTEST® could be used as marker of the histological integrity of the intestinal mucosa in some pathologies that commonly occur with intestinal mucosa damage, such as coeliac disease and inflammatory bowel diseases. Intestinal mucosa damage is defined as any alteration in normal morphology and functionality of the intestinal mucosa. Along the present description, the expression "mucosa damage", "mucosa impairment" or "mucosa alteration" will be used as synonyms or equivalents.

## DESCRIPTION OF THE INVENTION

### Brief description of the invention

The present invention describes a highly sensitive and specific *in vitro* D-xylose detection enzymatic method based on the xylose dehydrogenase (XylB) from *C. crescentus* NA1000, for the detection of D-xylose particularly to be used after an *in vivo* diagnostic method for the diagnosis of hypolactasia.

LacTEST® is a non-invasive method which allows evaluating the overall activity of intestinal lactase *in vivo* by means of the determination of D-xylose in urine and/or blood after the oral administration of 4-O-β-D-galactopyranosyl-D-xylopyranose (Gaxilose), a disaccharide which works as a structural analogue of lactose, the physiological substrate of intestinal lactase. After its oral administration, Gaxilose is hydrolyzed by the intestinal mucosa lactase enzyme into galactose and D-xylose (**Figure 1**) and these hydrolysis products pass into the blood. The appearance of D-xylose in urine and blood is closely correlated with the levels of intestinal lactase activity.

The method is carried out following the administration of a single dose of 0.45 g of Gaxilose for the determination of the total amount of xylose excreted in urine. The concentration of xylose is measured in the total urine collected up to 5 hours after the administration of the above indicated dose of Gaxilose (0-5-hour urine).

For the effects of the present specification, the terms: reference threshold value, normal limit, lower limit of normality, cut-off point and cut-off are synonymous, all of them being defined as those values below which a patient is diagnosed as hypolactasic by the Gaxilose test The preferred embodiments of present invention are the following:

1. Method for *in vitro* quantification of D-xylose in a biological fluid consisting of adding to said biological fluid, as the only enzyme, the enzyme D-xylose dehydrogenase (XylB) or putting in contact said biological fluid with a kit containing the same, wherein XylB is intervening in said D-xylose quantification.

2. Method according to the previous embodiment, wherein the D-xylose dehydrogenase is the enzyme XylB from *C. crescentus* strain NA1000.

3. Method according to any of the previous embodiments, wherein the biological fluid is urine or blood from a mammal.

4. Method, according to embodiment 3, wherein the biological fluid is from human origin.

5. Kit for quantification of D-xylose in a biological sample which comprises the enzyme D-xylose dehydrogenase (XylB) as the only enzyme in the kit, $NAD^+$ and a suitable buffer.

6. Kit according to the previous embodiment, wherein the suitable buffer is phosphate buffer.

7. Kit according to embodiments 5 or 6, wherein the D-xylose dehydrogenase is the enzyme XylB from *C. crescentus* strain NA1000.

8. Kit according to any of embodiments 5 to 7, wherein the biological sample is a biological fluid selected from urine or blood from a mammal.

9. Non-invasive diagnostic method for evaluation of intestinal lactase deficiency comprising the following steps:

   (i) administering via oral route to the individual who is the subject of the method, a quantity of 4-O-beta-D-galactopyranosil-D-xylose (Gaxilose) between 0.1125 g and 5.4 g,

   (ii) collecting total urine excreted by the individual between the time of administration of Gaxilose and 4 or 5 hours following said administration,

   (iii) determining the total amount of D-xylose excreted in the urine collected in this period between 4 and 5 hours following administration of Gaxilose, by mixing the excreted urine with the enzyme D-xylose 1-dehydrogenase (XylB), and

   (iv) comparing the value obtained in step (iii) with a threshold reference value obtained from a population of healthy control individuals subjected to the same protocol, wherein the subject is considered to be suffering from intestinal lactase deficiency (hypolactasia), when the value of excreted D-xylose obtained is below the threshold reference value.

10. Diagnostic method according to the previous embodiment, wherein the determination of the total amount of D-xylose excreted in step (iii) is carried out after incubation of the sample with the enzyme XylB, as the only enzyme used in the evaluation method.

11. Diagnostic method according to the previous embodiment, wherein the enzyme XylB is the enzyme XylB from *C. crescentus* strain NA1000.

12. The diagnostic method according to any of embodiments 9 to 11, wherein the doses of Gaxilose administered are selected from 0.1125 g, 0.225 g, 0.45 g, 0.9 g, 2.7 g and 5.4 g.

13. The diagnostic method according to any of embodiments 9 to 11, wherein the reference threshold value of the total amount of D-xylose in urine following the administration of the dose of 0.1125 g of Gaxilose is 3.95 mg in 4-hour urine and 5.06 mg in 5-hour urine.

14. The diagnostic method according to any of embodiments 9 to 11, wherein the reference threshold value of the total amount of D-xylose in urine following the administration of the dose of 0.225 g of Gaxilose is 11.74 mg in 4-hour urine and 12.55 mg in 5-hour urine.

15. The diagnostic method according to any of embodiments 9 to 11, wherein the reference threshold value of the total amount of D-xylose in urine following the administration of the dose of 0.45 g of Gaxilose is 15.59 mg in 4-hour urine and 19.18 mg in 5-hour urine.

16. The diagnostic method according to any of embodiments 9 to 11, wherein the reference threshold value of the total amount of D-xylose in urine following the administration of the dose of 0.9 g of Gaxilose is 20.77 mg in 4-hour urine and 27.98 mg in 5-hour urine.

17. The diagnostic method according to any of embodiments 9 to 11, wherein the reference threshold value of the total amount of D-xylose in urine following the administration of the dose of 2.7 g of Gaxilose is 31.28 mg in 4-hour urine and 50.48 mg in 5-hour urine.

18. The diagnostic method according to any of embodiments 9 to 11, wherein the reference threshold value of the total amount of D-xylose in urine following the administration of the dose of 5.4 g of Gaxilose is 68.38 mg in 4-hour urine and 87.40 mg in 5-hour urine.

19. Diagnostic method according to any of embodiments 9 to 18, wherein the degree of difference of the D-xylose reference threshold value and the D-xylose quantified in the sample determines the degree of intestinal lactase deficiency (hypolactasia) of the subject.

20. Use of the method according to the embodiments 1 to 4 and/or 5 to 8 in the diagnosis of hypolactasia.

21. Use of the method according to the embodiments 1 to 4 and/or 5 to 8 in the determination of intestinal mucosa impairment.

**Description of the figures**

**Figure 1:** Scheme of Gaxilose conversion to galactose and D-xylose.

**Figure 2:** Scheme of the reaction catalyzed by D- and L-xylose dehydrogenase (XDH).

**Figure 3:** Biosynthesis pathway for the conversion of D-xylose to 1,2,4-butanetriol. XylB: D-xylose dehydrogenase; XylonDH: D-xylonate dehydratase; BenDC: benzoylformate decarboxylase; ADH: alcohol dehydrogenase (Valdehuesa KN, 2014).

**Figure 4:** Strategies designed for cloning *xyl*B and *xyl*B-6His into the vector pET28b(+). Plasmids pET-*xylB*-wt and pET-*xylB*-6His are obtained.

**Figure 5:** Xylose dehydrogenase activity assay of the four produced XylB produced: XylB-wt-1 (2 μL diluted 1/10), XylB-wt2 (2 μL diluted 1/10), XylB-6His1 (1 μL non-diluted), XylB-His62 (1 μL non-diluted) and a negative control (1-10 μL). X-axis corresponds to time in seconds and Y-axis corresponds to OD.

**Figure 6:** Xylose dehydrogenase activity in XylB-wt2 production. The assay was performed in triplicate (triangles, circles and squares). X-axis corresponds to time in seconds and Y-axis corresponds to OD.

**Figure 7:** Size-exclusion chromatogram registered in the XylB-wt2 protein purification. X-axis corresponds to the absorbance at 280 nm (mAU) of the components in the sample and Y-axis corresponds to the volume of the sample eluted (mL). The insert represents the fractions comprising the purified protein.

**Figure 8:** Substrate kinetics of purified XylB. **A.** Kinetics of the purified XylB-wt2 at different concentrations of D-xylose using an excess of $NAD^+$ (0.5 mM). **B.** Kinetics of the purified XylB-wt2 at different concentrations of D-glucose using an excess of $NAD^+$ (0.5 mM). The insert shows the plot scaled in the Y-axis. X-axis corresponds to substrate concentration (mM) and Y-axis corresponds to the specific activity (μmol· $min^{-1}$· $mg^{-1}$).

**Figure 9:** Kinetics of XylB-wt2 for the cofactor $NAD^+$ measured at eleven concentrations. X-axis corresponds to $NAD^+$ concentration (mM) and Y-axis corresponds to the specific activity (μmol· $min^{-1}$·$mg^{-1}$).

**Figure 10:** Molecular weight standard curve. The elution volume of XylB-wt2 was interpolated in a standard curve which was made using reference proteins which native molecular weight is known, in order to calculate native molecular weight of the enzyme. X-axis corresponds to molecular weight (kDa) and Y-axis corresponds to elution volume (mL).

**Figure 11:** Stability of XylB-wt2 over time. The stability of the enzyme was analyzed in three different conditions: freeze-dried stored at 4°C (full circles), freeze-dried stored at room temperature (RT: empty circles) and a rehydrated powder aliquot in pH 8.0 buffer stored at 4°C (triangles). X-axis corresponds to time (days) and Y-axis corresponds to the enzyme specific activity (U/mg).

**Figure 12:** Absorbance values obtained by measuring serial dilutions of D-xylose (0-15 mg/dL) in urine samples from healthy donors (circles) or in a buffer sample (triangles) using the enzymatic detection method. X-axis corresponds to D-xylose concentration (mg/dL) and Y-axis corresponds to absorbance increase at 340 nm (AU).

**Figure 13:** Xylose quantification reaction kinetics with the reagents (R1 and R2) of the kit. The sample is added to R1 and absorbance at 340 nm is measured after 5-minutes of incubation (A1). Then, R2 is added to the reaction and absorbance at 340 nm is measured after a 5-minutes incubation (A2). Reaction endpoint, wavelength 340 nm. Reaction must be carried out at the standard device temperature (usually 37°C). X-axis corresponds to time (minutes) and Y-axis corresponds to the absorbance at 340 nm (AU).

**Figure 14:** Absorbance values obtained by measuring the D-xylose concentrations in analyzed samples using the enzymatic detection method. Differences of absorbance at 340 nm ($\Delta A_{340\ nm}$) were determined in triplicate. **A.** Xylose concentration ranged from 0 to 19 mg/dL. No linearity is detected when absorbance values are over 2 AU (10 mg/dL). **B.** Linear regression calculated with samples under 10 mg/dL of D-xylose. X-axis corresponds to D-xylose concentration (mg/dL) and Y-axis corresponds to the absorbance at 340 nm (AU).

**Figure 15:** Linearity of absorbance increment over D-xylose concentrations in samples measured in the auto-analyzer ILab 600 (Werfen). X-axis corresponds to D-xylose concentration (mg/dL) and Y-axis corresponds to the absorbance increment at 340 nm (AU).

**Figure 16:** Linearity of absorbance increment over D-xylose concentrations in samples measured in the auto-analyzer Cobas c502 (Roche). X-axis corresponds to D-xylose concentration (mg/dL) and Y-axis corresponds to the absorbance increment at 340 nm (AU).

**Figure 17:** Linearity of absorbance increment over D-xylose concentrations in samples measured in the auto-analyzer Dimension Vista 1500 (Siemens). X-axis corresponds to D-xylose concentration (mg/dL) and Y-axis corresponds to the absorbance increment at 340 nm (AU).

**Figure 18:** Correlation of D-xylose concentrations calculated using the enzymatic and the colorimetric methods. **A** and **C.** Detection of D-xylose in urine samples measured by the enzymatic method (white), colorimetric method (black) an expected value (grey) of samples 1 (**A**) and 2 (**C**). X-axis corresponds to D-xylose concentrations added in the urine samples of healthy donors (mg/dL) and Y-axis corresponds to the D-xylose concentrations detected with each method (mg/dL). **B** and **D.** Linear correlation of D-xylose concentration between calculated values for both methods in each urine sample (sample 1: **B,** or sample 2: **D**). X-axis corresponds to D-xylose concentration (mg/dL) obtained by the phloroglucinol method and Y-axis corresponds to the D-xylose concentration (mg/dL) obtained by the enzymatic method.

**Figure 19:** Correlation between D-xylose values obtained using the enzymatic method with ILab 600 auto-analyzer and the phloroglucinol method (45 samples). **A.** Correlation between D- xylose concentration (mg/dL) calculated with the phloroglucinol assay (Y-axis) and the enzymatic assay in an auto-analyzer ILab 600 (X-axis). **B.** Correlation between total D-xylose amount (mg) calculated with the phloroglucinol assay (Y-axis) and the enzymatic assay in an auto-analyzer ILab 600 (X-axis).

**Figure 20:** Receiver operating characteristics (ROC) curve of the results obtained by the enzymatic method using as reference the results obtained by the phloroglucinol reaction using Werfen automated analyzer (45 samples). X-axis corresponds to 1-specificity and Y-axis corresponds to sensitivity.

**Figure 21:** Correlation between total D-xylose amount values (mg) obtained using the phloroglucinol method (Y-axis) and the enzymatic method with Cobas c502 automated analyzer from Roche (224 samples) (X-axis).

**Figure 22:** ROC curve of the results obtained by the enzymatic method using as reference the results obtained by the phloroglucinol reaction using Roche Cobas c502 automated analyzer (224 samples). X-axis corresponds to 1-specificity and Y-axis corresponds to sensitivity.

**Figure 23:** Corrected ROC curve of the results obtained by the enzymatic method using as reference the results obtained by the phloroglucinol reaction using Roche Cobas c502 automated analyzer (224 samples). X-axis corresponds to 1-specificity and Y-axis corresponds to sensitivity.

**Figure 24:** Classification of the samples of the assay as normolactasic (-) or hypolactasic (+) according to the colorimetric (phloroglucinol) and enzymatic methods. There are five discrepancies between the methods. The correlation between the two methods is indicated by the Kappa coefficient (95% confidence intervals are also indicated).

**Figure 25.** ROC curves of the enzymatic method results for a theoretical dose of Gaxilose of 0.45 g, generated taking as reference the phloroglucinol results in 5-hour (**A**) and 4-hour (**B**) urine samples. (AUC: area under the curve; CI: confidence interval). X-axis corresponds to 1-specificity and Y-axis corresponds to sensitivity.

**Figure 26.** ROC curves of the enzymatic method results for a theoretical dose of Gaxilose of 0.1125 g, generated taking as reference the phloroglucinol results in 5-hour (**A**) and 4-hour (**B**) urine samples (AUC: area under the curve; CI: confidence interval). X-axis corresponds to 1-specificity and Y-axis corresponds to sensitivity.

**Figure 27.** ROC curves of the enzymatic method results for a theoretical dose of Gaxilose of 0.225 g, generated taking as reference the phloroglucinol results in 5-hour (**A**) and 4-hour (**B**) urine samples (AUC: area under the curve; CI: confidence interval). X-axis corresponds to 1-specificity and Y-axis corresponds to sensitivity.

**Figure 28.** ROC curves of the enzymatic method results for a theoretical dose of Gaxilose of 0.9 g, generated taking as reference the phloroglucinol results in 5-hour (**A**) and 4-hour (**B**) urine samples (AUC: area under the curve; CI: confidence interval). X-axis corresponds to 1-specificity and Y-axis corresponds to sensitivity.

**Figure 29.** ROC curves of the enzymatic method results for a theoretical dose of Gaxilose of 2.7 g, generated taking as reference the phloroglucinol results in 5-hour (**A**) and 4-hour (**B**) urine samples (AUC: area under the curve; CI: confidence interval). X-axis corresponds to 1-specificity and Y-axis corresponds to sensitivity.

**Figure 30.** ROC curves of the enzymatic method results for a theoretical dose of Gaxilose of 5.4 g, generated taking as reference the phloroglucinol results in 5-hour (**A**) and 4-hour (**B**) urine samples (AUC: area under the curve; CI: confidence interval). X-axis corresponds to 1-specificity and Y-axis corresponds to sensitivity.

**Figure 31:** Linearity of absorbance increment over D-xylose concentration in 13 serum samples from healthy donors spiked with different concentrations of xylose (0.15 - 100 μM). A buffer sample (dotted line) was included as reference. X-axis corresponds to D-xylose concentration (μM) and Y-axis corresponds to absorbance increment at 340 nm.

**Figure 32:** Linearity of expected D-xylose concentrations over calculated D-xylose concentrations in serum samples from healthy donors analyzed by the enzymatic method (**A**) and the enzymatic method using basal serum as internal reference (**B**). This sample of serum, which does not contain D-xylose, is measured in order to establish the unspecific background in an individual blood sample. X-axis corresponds to calculated D-xylose concentration (μM) and Y-axis corresponds to expected D-xylose concentration (μM). Buffer samples are indicated with empty circles and serum samples are indicated with black circles.

## Detailed description of the invention

The present invention describes an enzymatic detection method of D-xylose with a single xylose dehydrogenase enzyme, particularly XylB from *C. crescentus* NA1000.

A preferred embodiment of the invention is a kit for the detection and quantification of xylose with a single enzyme XylB.

In another preferred embodiment of the invention is the detection and quantification of xylose with a single enzyme XylB in biological samples (urine and/or blood).

In another preferred embodiment of the invention is an *in vitro* enzymatic method to be used in the *in vivo* diagnosis of intestinal lactase deficiency (hypolactasia). The method comprising the stages of:

a) Administering via oral route to the individual who is the object of the test a unique dose of Gaxilose (0.45 g).

b) Collecting total urine excreted by the individual between the time of administration of Gaxilose and 5 hours following this administration.

c) Determining *in vitro* the total amount of xylose with an enzymatic test, based on the use of a single xylose dehydrogenase enzyme, particularly XylB from *C. crescentus* NA1000. Total amount of xylose is quantified in the excreted urine collected in this period of 5 hours following the administration of Gaxilose.

d) Comparing the *in vitro* value obtained in step c) with a threshold reference value subjected to the same protocol, below which threshold the subject is considered to be suffering from intestinal lactase deficiency (hypolactasia).

In another embodiment of the invention, the evolution of xylose amount detected by the enzymatic method disclosed herein after the administration of LacTEST® can be used as histological integrity marker of intestinal mucosa in some pathologies that commonly encompass intestinal mucosa damage, such as coeliac disease and inflammatory bowel diseases. The variation of lactase activity (measured through the variation of xylose amount detected after the administration of LacTEST®) is the variable used, not the absolute value of lactase activity. Therefore, this variation can be used as a follow-up or evolution biomarker for pathologies with histological damage and later on as a system of diet adjustment, for patients suffering from intestinal mucosa damage.

### Example 1. Production of xylose dehydrogenase from *C. crescentus* NA1000 in engineered *E. coli* strains.

The gene *xyl*B from *C. crescentus* strain NA1000 (SEQ ID NO:1, GeneBank Accession Number: ALC94329.1, reverse complementary strand) which codifies the enzyme D-xylose dehydrogenase (XylB, GeneBank Accession Number: CP001340.1, SEQ ID NO:2) was identified and analyzed. Two different cloning strategies have been followed in order to get two alternative XylB protein constructions: a wild-type enzyme (XylB-wt) and an N-terminal six histidine-tagged enzyme (XylB-6His). Expression of XylB as a His-tagged recombinant protein might allow simplifying its purification by ion metallic affinity chromatography (IMAC); however, it has been previously described in the literature that activity and stability of XylB could be affected in this kind of constructions. For this reason, the wild-type construction has also been considered as alternative.

The gene *xyl*B from *C. crescentus* strain NA1000 was codon optimized (SEQ ID NO:3) and chemically synthesized. Specific sequences for restriction endonucleases were included in the final synthetic gene (SEQ ID NO:4). This synthetic gene was cloned into the pUC57 vector by GenScript Corporation (Piscataway, NJ, USA) resulting in the recombinant plasmid pUC57-*xylB.* After optimization, the codon adaptation index (CAI) was increased from 0.73 to 0.89, where a CAI of 1.0 is considered to be perfect in the desired expression organism, and a CAI of > 0.8 is regarded as good, in terms of high gene expression level. The frequency of optimal codons was also improved in the optimized sequence, avoiding codons with less than 50 % of usage frequency in *E. coli.* In addition, GC content in the gene was adjusted from 67.87 % to 54.19 % (the ideal percentage range of GC content is between 30-70 %).

In order to express the optimized *xylB* in *E. coli* BL21(DE3), the synthetic gene from the plasmid pUC57-*xyl*B was sub-cloned in the expression vector pET28b(+) (Novagen, USA). Its use allows getting the two required XylB constructions (XylB-wt and XylB-6His) by alternative digestion with specific restriction endonucleases. To obtain the construction XylB-wt (SEQ ID NO:5), pUC57-*xyl*B is digested with the endonucleases *Nco*I/*Hin*dIII and cloned in pET28b(+), also digested with these two enzymes. The construction XylB-6His (SEQ ID NO:6) requires a double digestion of pUC57-*xylB* with *Nde*I/*Hin*dIII, and is cloned in pET28b(+) also digested with the same restriction enzymes (**Figure 4**). The two cloning strategies were followed in parallel so both XylB constructions were ready at the same time. The resulting plasmids were named pET-*xyl*B-wt (SEQ ID NO:7, encoding for the wild type enzyme) and pET-*xyl*B-6His (SEQ ID NO:8, encoding for the his-tagged enzyme). Both constructions were transformed in DH5$\alpha$ cells, purified, and analyzed by double digestion to check if genes had been successfully inserted.

The plasmids were transformed into the *E. coli* BL21(DE3) cells. Expression strains containing the plasmids pET-*xyl*B-wt (XylB-wt1 and XylB-wt2) and pET-*xyl*B-6His (XylB-6His1 and XylB-6His2) were cultured in 200 mL of LB broth containing the selection antibiotic kanamycin at 37°C with shaking. When the cultures reached an $OD_{600nm}$ of 0.5, protein expression was induced with IPTG (0.5 mM) and the temperature was dropped to 30°C. The cultures were incubated for a further period of 12h. Cells were harvested by centrifugation, washed twice and resuspended in buffer (50 mM $NaH_2PO_4$, pH 8.0). Cell suspension was disrupted by sonication and the cell lysate was centrifuged at 10000 xg for 20 min. Clear supernatant (CFE) was analyzed to determine that the two XylB constructions had been expressed.

XylB-wt displays an excellent level of over-expression in soluble fractions, even higher than initially expected. This fact might be due to the genetic engineering process used in the synthetic gene design, expression levels have been improved over 80 % of total soluble protein.

Xylose dehydrogenase activity in each CFE was evaluated. XylB catalyzes the oxidation of D-xylose with concomitant reduction of its cofactor $NAD^+$, to yield NADH. Activity can be determined by measuring the formation of NADH by spectrophotometric monitoring. Variations of absorbance at 340 nm are proportional to consumed D-xylose concentration in the reaction ($\varepsilon^{NADH}$ = 6220 $cm^{-1}M^{-1}$). Activity assays were performed at RT and pH 8.0 (**Figure 5**). Absorbance at 340 nm (OD) was spectrophotometrically recorded along the time in the presence of each CFE sample containing expressed XylB: wt1, wt2, 6His1 and 6His2. Activity assay in control CFE has also been included. Volumes of CFE samples used in the assays are shown in brackets, as well as when the sample has been diluted 10 times (dil 1/10). Slopes are proportional to enzyme activity. According to these results XylB-wt is more efficiently expressed, which results in a higher enzyme activity in soluble fractions. In addition, XylB-wt over-expression levels made of CFE fractions an excellent starting point for enzyme purification, due to the high concentration of accumulated enzyme (around 81 % of the total soluble protein). For this reason, the strain producing XylB- wt2 was chosen because of its higher enzyme production after expression (20 U/mg).

**Production of xylose dehydrogenase from *C. crescentus* NA1000 in engineered *E. coli* cells.**

Expression strain *E. coli* BL21(DE3)/pET-*xyl*B-wt2 was cultured in 1 L of LB broth containing the selection antibiotic kanamycin at 37°C with shaking. When the culture reached an $OD_{600n}$ of 0.5, protein expression was induced with IPTG (0.5 mM) and the temperature was dropped to 30°C. The culture was incubated for a further period of 12h. Cells were harvested by centrifugation, washed twice and resuspended in buffer (50 mM $NaH_2PO_4$, pH 8.0). Cell suspension was disrupted by sonication and the cell lysate was centrifuged at 10000 xg for 20 min. The resultant cell free extract (CFE), around 40 mL, was stored at -80°C in aliquots of 1 mL previously frozen with liquid nitrogen, in order to avoid protein denaturation in the process.

Produced CFE was checked by SDS-PAGE and activity assay to confirm that XylB-wt2 was expressed. SDS-PAGE analysis showed that the enzyme was over-expressed. The amount of expressed protein was analyzed by densitometry showing that XylB-wt represented 82 % of the total protein in the soluble fraction. Xylose dehydrogenase activity in CFE was also evaluated using the previously described activity assay. The thawed CFE (previously stored at -80°C) displayed an activity of 268.9 U/mL, and its protein concentration was 15.44 mg/mL. Therefore, the CFE after the freezing process retains a specific activity of 17.42 U/mg. Activity assays were performed at RT and pH 8.0. Absorbance at 340 nm (OD) was spectrophotometrically recorded along the time in the presence of 2 $\mu$L of CFE sample (diluted 1/10 in buffer) containing expressed XylB-wt2. The assay was carried out as a triplicate (**Figure 6**).

XylB-wt2 was purified by size-exclusion chromatography on a HiLoad 26/60 Superdex 200 PG column controlled using the ÄKTA-Prime Plus FPLC system (GE Healthcare Life Science). 5 mL of CFE were used in each purification, which was carried out in 50 mM $NaH_2PO_4$ buffer pH 7.2 containing NaCl (0.15 M) at a constant flow rate of 1.0 mL/min. Central fractions displaying XylB activity were taken; in order to avoid possible interfering proteins and increase the purity degree, only the fractions with the highest absorbance and activity values were taken, discarding the rest of the peak. This purification was repeated 4 times, with an excellent reproducibility. **Figure 7** shows the chromatogram for one of the XylB-wt purifications. Absorbance at 280 nm was recorded over the elution volume for protein detection after CFE loading. Only the three central fractions of the XylB-wt2 assigned peak (maximum eluted at 194 mL) were taken to continue with the purification process.

To prevent an excessive salt concentration in freeze-dried samples (Superdex columns required high concentrations of NaCl in the purification buffer to avoid unspecific protein interactions) a dialysis step was included. Fractions containing XylB-wt2 were pooled together in a dialysis membrane and incubated overnight at 4°C in 3 L of 5 mM $NaH_2PO_4$ buffer (pH 8.0), under stirring. All the purification steps were analyzed by SDS-PAGE in order to detect the purified XylB-wt2 and its purity degree by gel densitometry. The dialyzed protein was freeze-dried in glass vials (15 mL) and stored at 4°C. The XylB-wt2 samples have been found to be much more stable when freeze-dried and stored in this condition, with no activity loss in at least one year. Interestingly, the freeze-drying of the samples does not affect almost to the activity of the enzyme, which maintains 90 - 100 % of the initial activity after the process (See **Table 1**).

**Table 1.** Characteristic of XylB-wt2 freeze-dried batch.

| BATCH | Dialyzed (mL) | Freeze-dried (mg) | Ratio Dial/Powder (mg/mL) | Activity (U/mg powder) |
|---|---|---|---|---|
| **7/02/14** | 6.0 | 20.0 | 3.33 | 25.0 |
| **19/02/14** | 8.8 | 30.3 | 3.44 | 28.7 |
| **17/07/14** | 7.3 | 27.2 | 3.72 | 27.1 |
| **18/11/14** | 8.7 | 30.5 | 3.51 | 29.7 |

**Characterization of the xylose dehydrogenase XylB-wt2: substrate specificity.**

The enzyme XylB-wt2 was characterized to determine if it is possible its application in sensitive and reliable D-xylose detection and quantification. Although D-xylose is the main substrate for all Xylose dehydrogenases (XHDs), it is known that they are also able to catalyze oxidation of other sugars (e.g. D-glucose and L- arabinose), with higher or lower activity ratios depending on the enzyme and the sugar substrate. This fact would represent an important drawback for detection of D-xylose, due to the presence of these other undesirable sugars in biological samples as blood, which would interfere in the D-xylose determination. In order to confirm the substrate specificity, 9 different sugars (7 monosaccharides and 2 disaccharides) were assayed as XylB-wt substrates. **Table 2** shows the specific and relative activities of XylB-wt for each one of the substrates.

**Table 2.** XylB-wt2 specific activity measured with different mono and disaccharides.

| SUGAR | Concentration (mM) | Specific Activity (U/mg) | Relative Activity (%) |
|---|---|---|---|
| **Monosaccharide** | | | |
| D-Xylose | 5 | 30.0 | 100 |
| L-Arabinose | 5 | 4.8 | 16 |
| D-Ribose | 100 | 4.4 | 15 |
| D-Glucose | 100 | 3.9 | 13 |
| D-Galactose | 100 | 0.5 | 2 |
| D-Mannose | 100 | 0.0 | 0 |
| D-Fructose | 100 | 0.0 | 0 |
| **Disaccharides** | | | |
| Gaxilose | 100 | 0.7 | 2 |
| Maltose | 100 | 0.0 | 0 |

After this activity assay, only two of the monosaccharides showed activity at lower substrate concentrations: D-xylose, the main substrate, and L-arabinose, which displayed a 16% of relative activity. D-ribose, D-glucose, and D-galactose also showed activity but only when substrate concentration was increased in 20 times (100 mM instead 5 mM). Regarding the assayed disaccharides, only Gaxilose was found to be substrate of XylB-wt2, but only at high concentration and with a poor relative activity of 2% with respect to D-xylose. Subsequently, after conducting the substrate specificity experiment, we can conclude that D-xylose is the favorite substrate of the enzyme XylB-wt2; only L-arabinose can be considered a substrate at physiological concentrations. As this sugar is rare in biological samples, especially those from fasted patients, it should not be a problem as possible interfering agent in D-xylose detection in the methods disclosed herein.

However, even if D-glucose is not a good substrate of XylB-wt2, this sugar could be a problem due to the presence of significant concentrations of D-glucose in biological samples as blood, and would interfere in the D-xylose determination. For this reason, a more detailed specific activity study was carried out, calculating the kinetic parameters of XylB-wt2 for D-xylose and D-glucose as substrates. Steady-state kinetic assays for XylB activity were measured in triplicate at 25°C in 96-well plates (total volume of 0.3 mL). Measurements of kinetic parameters for D-xylose were performed with purified XylB-wt2 at eight different D-xylose concentrations and with saturating concentration of $NAD^+$ (0.5 mM). **Figure 8** discloses the assays to determine the kinetic parameters for D-xylose (A) and D-glucose (**B**) with purified XylB-wt2 at different concentrations of substrate. Xylose dehydrogenase activity of purified XylB-wt2 was measured at RT and pH 8.0.

Regarding the cofactor, both $NAD^+$ and $NADP^+$ were assayed as XylB-wt2 substrates, but the enzyme only displayed activity with the first one. This data is in agreement with the extreme specificity for the cofactor $NAD^+$ described in the literature for all the identified XylB enzymes. Kinetic parameters of XylB-wt2 for the cofactor $NAD^+$ were also calculated. Kinetic assays were performed in the same way as previously described for D-xylose and D-glucose, at eleven $NAD^+$ concentrations and saturating D-xylose (5 mM) (**Figure 9**). Kinetic constants of different XylB constructions are summarized in **Table 3.**

**Table 3.** Summary of the kinetic constants of different XylB constructions.

| Substrate | $V_{max}$ (U·mg$^{-1}$) | $k_{cat}$ (min$^{-1}$) | $K_M$ (mM) | $k_{cat}/K_M$ (mM$^{-1}$·min$^{-1}$) | Ref. |
|---|---|---|---|---|---|
| **XylB-wt2** | | | | | |
| D-Xylose | 30.2 ±1.0 | 812 ±35 | 0.041 ±0.005 | 19800 | [a] |
| D-Glucose | 10.7 ±0.1 | 287 ±50 | 216 ±30 | 1.32 | [a] |
| $NAD^+$ | 30.1 ±1.0 | 810 ±65 | 0.078 ±0.01 | 10385 | [a] |
| **6His-tagged XylB** | | | | | |
| D-Xylose | 27.5 | - | 0.76 | - | [b] |
| $NAD^+$ | 20.5 | - | 166 | - | [b] |
| | | | **Strep-TEV-XylB** | | |
| D-Xylose | - | 1360 ±65 | 0.08 ±0.02 | 16800 | [c] |
| $NAD^+$ | - | 1860 ±65 | 0.46 ±0.05 | 4000 | [c] |

[a]: XylB-wt2; calculated data.
[b]: N-terminal 6His-tagged XylB; data taken from: Stephens C, 2007.
[c]: N-terminal strep-TEV-XylB; data taken from: Toivari M, 2012a.

Table 3 shows that the catalytic efficiency of XylB-wt for D-xylose is much higher than that for D-glucose (19800 mM$^{-1}$·min$^{-1}$ vs. 1.32 mM$^{-1}$·min$^{-1}$). The catalytic efficiencies ($k_{cat}/K_M$) ratio D-xylose/ D-glucose is 15000, which clearly indicates a higher specificity of the enzyme for the first substrate in the assayed conditions. These results support the use of XylB-wt2 in D-xylose detection, even in samples where D-glucose is also present. Comparing the calculated parameters with the ones described in the literature for other XylB constructions, XylB-wt2 and Strep-TEV-XylB showed very similar parameters when D-xylose is used as substrate; catalytic efficiency and $K_M$ are in the same order, but $k_{cat}$ is slightly lower. This fact could be due to the pH used to perform the kinetic experiments: pH 8.0 for XylB-wt2 and pH 9.0 for Strep-TEV-XylB. The optimal pH of XylB from *C. crescentus* has been found about 7.5, but all the activity assays in XylB-wt2 analysis have been done at pH 8,0. This pH was chosen because, apart from the fact that the activity is higher when the pH is increased, preliminary storing experiments showed that long term stability of XylB-wt2 in solutions is increased when the pH is around 8.0. Thus, as the main goal was to develop a reliable and robust D-xylose detection method, a small reduction of enzyme activity at pH 8.0 was accepted in order to improve the stability.

When cofactor kinetic parameters are compared, XylB-wt2 displays better catalytic efficiency, as well as a $K_M$ value one order of magnitude lower. As it was pointed in the use of D-xylose as substrate, $k_{cat}$ is slightly higher for Strep-TEV-XylB, probably because of the pH of the assay. The construction 6His-tagged XylB displays $V_{max}$ values in the same order than XylB-wt2 enzyme, however, $K_M$ values are considerably higher. This could be due to the effect of the 6His-tag in the XylB structure, which has been previously indicated as an interfering factor in the final enzyme activity.

**Characterization of the xylose dehydrogenase XylB-wt2: quaternary structure.**

Quaternary structure of the protein XylB-wt2 was evaluated by gel filtration in native conditions. Protein was separated by size-exclusion chromatography on a HiLoad 26/60 Superdex 200 PG column at 194 mL of elution volume. This elution volume was interpolated in a standard curve which was made using reference proteins with known native molecular weight. As result of this interpolation, the calculated native molecular weight of XylB-wt2 was 168100 Da. The molecular weight of one XylB-wt subunit is 26968 Da so the gel filtration data indicate that the native protein might be a homohexamer. This result should be confirmed by other techniques as ultracentrifugation or protein crystallization, as this is the first description of the possible quaternary structure of XylB from *C. crescentus.* In fact, the calculated native molecular weight is the biggest among those of all the previously identified XylB, that have been identified as dimeric proteins of 60-90 KDa (**Figure 10**).

The calculated homohexameric quaternary structure should be considered when kinetic parameters are calculated. Kinetic constants previously summarized were calculated assuming XylB-wt is a monomer, so these values were recalculated for a hexameric protein with six active sites per enzyme (See **Table 4**).

**Table 4.** Kinetic constants of XylB-wt2, considering the enzyme as a homohexameric protein.

| Substrate | $V_{max}$ ($U \cdot mg^{-1}$) | $k_{cat}$ ($min^{-1}$) | $K_M$ (mM) | $k_{cat}/K_M$ ($mM^{-1} \cdot min^{-1}$) |
|---|---|---|---|---|
| D-Xylose | 30.2 ±1.0 | 4.872 ±35 | 0.041 ±0.005 | 118.885 |
| D-Glucose | 10.7 ±0.1 | 1.721 ±50 | 216 ±30 | 7.96 |
| $NAD^+$ | 30.1 ±1.0 | 4.858 ±65 | 0.078 ±0.01 | 62.284 |

**Characterization of the xylose dehydrogenase XylB-wt2: stability**

The enzyme is more stable when it is incubated at pH around 8.0. Initially dialyzed samples from size-exclusion purification, which had a pH of 7.2, were found very stable when stored at 4°C in solution (more than two weeks with no detectable loss of activity). This stability was improved when pH was increased to 8.0, in rehydrated freeze-dried samples. For this reason, pH 8.0 was chosen for storing and reaction default conditions, as a compromise between maximum activity (pH around 7.5) and stability.

In addition, the enzyme showed an exceptional stability when it was freeze-dried. After one year stored at 4°C, XylB-wt2 powder retained an activity comparable with the initially measured. This stability was found slightly lower when the freeze-dried enzyme was stored at RT, but a considerable activity was found after one year of storage in this condition. It is especially remarkable the high stability of the rehydrated freeze-dried enzyme in aqueous solution: when an aliquot of powder suspended in buffer pH 8.0 was stored at 4°C, the enzyme retained 85 % of its initial activity after 150 days. Activity assays were carried out at RT in aliquots of 0.6 mg of powder in 100 μL of buffer (**Figure 11**).

**Example 2. Detection of D-xylose in urine samples by an enzymatic method based in the single xylose dehydrogenase XylB.**

The enzyme XylB has been previously proved to be an efficient method for the detection of D-xylose in buffer solutions, but not in biological fluids. One objective of the enzymatic method is its use in quantification of D-xylose present in biological fluids as urine samples. Urine samples were collected from three donors in order to evaluate the enzymatic detection of D-xylose. The sample donors did not present any digestive disorder or diseases which could interfere in the normal composition of urine, such as diabetes or abnormal kidney function. The three samples were collected using the same protocol described in the LacTEST® diagnostic procedure (AEMPS, Spanish Agency of Medicines and Sanitary Products), although they did not take LacTEST®, only water, before the urine was collected.

Linearity of the analytical method was studied measuring serial dilutions of D-xylose ranging from 0 to 15 mg/dL in the urine samples. 0.024 mg/mL of enzyme was used in the reaction and absorbance at 340 nm was measured in triplicate for each D-xylose concentration in each urine sample using an auto-analyzer microplate assay. **Figure 12** shows the absorbance for each D-xylose concentration in each donor's urine sample (circles) and the concentrated sample in buffer (triangles).

The behaviour of the enzymatic assay is very similar when both urine and buffer are used for sample preparation. Linearity is not affected when urine samples are employed and linear regression parameters are very similar. Urine color in concentrated samples slightly affects, but this can be considered unspecific background not strong enough to interfere in D-xylose quantification (**Table 5**).

**Table 5:** Linear regression coefficients and detection-quantification limits obtained in a standard microplate assay with different concentrations of D-xylose in urine samples. Buffer containing D-xylose was included as control.

| Sample | $y_0$ | *a* | $R^2$ | *RSD* | LoD (mg/dL) | LoQ (mg/dL) |
|---|---|---|---|---|---|---|
| Urine 1 | 0.0083 | 0.0462 | 0.9994 | 0.0073 | 0.474 | 1.58 |
| Urine 2 | -0.0119 | 0.0473 | 0.9980 | 0.0137 | 0.868 | 2.90 |
| Urine 3 | 0.0069 | 0.0464 | 0.9994 | 0.0077 | 0.498 | 1.66 |
| Buffer | -0.0070 | 0.0475 | 0.9992 | 0.0090 | 0.568 | 1.89 |

Thus, the enzymatic detection based on the use of XylB has been proved to be a reliable method for D-xylose quantification in urine samples, with a linearity limit of at least 15 mg/dL in the assayed conditions. The enzymatic reaction displays similar results in both buffer and urine samples, which is practical for the employment of standard references in D-xylose quantification analysis.

**Example 3. Enzymatic kit for determination of D-xylose**

The enzymatic kit for xylose quantification includes the following reagents:

**Vial 1.** Phosphate buffer 50 mM, pH 8.0 (solution)
**Vial 2.** Lyophilized $NAD^+$
**Vial 3.** Lyophilized XylB
**Vial 4.** Calibrator: Standard xylose solution (3.75 mg/dL)

**Preparation of reagents:**

a) Add phosphate buffer (vial 1) in the vial 3, to dissolve the lyophilized enzyme at a final concentration of 0.24 mg/mL. Mix genteelly to avoid loss of activity in the suspended enzyme. Keep cold during use if possible. This is called **REACTIVE 2 (R2).**
b) Add phosphate buffer (vial 1) in the vial 2 (lyophilized $NAD^+$) and mix until it is totally dissolved.
c) Add the complete dissolved $NAD^+$ ($NAD^+$ + buffer) to the vial 1. Thus, the vial 1 will contain phosphate buffer plus $NAD^+$ in the appropriated concentration for the assays (3.4 mg/mL). Keep cold during use if possible. This is called **REACTIVE 1 (R1).**
d) The Standard xylose solution is ready for use.

The assay profile is shown in **Figure 13.** The sample is added to R1 and absorbance at 340 nm is measured after 5 minutes of incubation (A1). Then, R2 is added to the reaction and absorbance at 340 nm is measured after a 5-minute incubation (A2). Differences between the two absorbance values are proportional to D-xylose concentration, which is calculated using the xylose standard solution as reference.

- $\Delta$Absorbance (340 nm) = $\Delta A_{340nm}$ = A2 - A1
- $\Delta$Absorbance Standard Solution (340 nm) = $\Delta A_{340nm}$ (St. Sol) = A2 (St. Sol) - A1 (St. Sol)
- Concentration of xylose in the Standard Solution (3.75 mg/dL)

$$[\text{Sample}]\ (\text{mg/dL}) = [\Delta A_{340nm}\ (\text{sample}) / \Delta A_{340nm}\ (\text{St. sol})] \times 3.75\ \text{mg/dL}$$

$$\text{Xylose} = [\text{Sample}]\ (\text{mg/dL}) \times \text{volume sample (dL)}$$

**Example 4. Measurement of xylose with auto-analyzers using the enzymatic method and kit of the invention.**

One of the goals of the new enzymatic D-xylose detection method is its use in automated analyzers applied in clinical analysis. Therefore, the whole design of the method is based on the characteristics and methodology limitations of these detection devices. Concretely, three automated analyzers broadly distributed in clinical laboratories were used as instrumental references:

- ILab 600, from Werfen.
- Cobas c502, from Roche.
- Dimension Vista 1500, from Siemens.

These three analyzers have different operational settings that cannot be changed, such as the maximum volume of dispensed reactive-sample, total volume in measurement cuvettes, and temperature or incubation time in the assay. Considering all these particular restricting factors, a general assay protocol was optimized for D-xylose detection. This general protocol can be slightly modified in some cases to be adapted for a specific analysis device.

Initial laboratory measurement protocols were carried out using a spectrophotometer SpectraMax Plus and the analysis software SofMax Pro v4.1 (Molecular Devices). Generally, assays were performed in 96-well microplates (flat-bottomed) at room temperature (between 22°C and 26°C) but, occasionally 1 mL cuvettes and 37°C temperatures were also used with the same result.

The linearity of the analytical procedure was examined measuring serial dilutions of D-xylose ranging from 0 to 19 mg/dL. Dilutions were made using $NaH_2PO_4$ buffer at pH 8.0. Differences of absorbance at 340 nm ($\Delta A_{340\ nm}$) were determined in triplicate. When sample concentrations were plotted against their absorbance at 340 nm (**Figure 14A**), linearity was not maintained for values of D-xylose over 10 mg/dL, being the absorbance difference of around 2 AU. Linearity was displayed when data were plotted under 10 mg/dL (**Figure 14B**). An $R^2$ value of 0.9992 was obtained in the linear regression using the statistics tools of the software SigmaPlot v12.0 (Systat Software Inc). The equation of the regression line and the residual standard deviation (RSD) were also determined:

- Equation of the regression line: $y = y_0 + ax = 0.0050 + 0.2030x$
- $R^2$=0.9996
- Slope (*a*) = 0. 2030
- Residual Standard Deviation (RSD) or Standard Error of Estimate = 0.0167

Limit of detection (LoD) and limit of quantification (LoQ) of the technique were calculated from the regression parameters by the statistical formula:

- $LoD = 3 \cdot RSD/a$

- $LoQ = 10 \cdot RSD/a$

Thus, calculated LoD is 0.247 mg/dL, and LoQ is 0.823 mg/dL. As the absorbance linearity range, may vary between different models of microplate readers and auto analyzers, an additional calibration is recommended prior to establishing a new assay in every device.

**Adjustment of ILab 600 (Werfen) auto-analyzer settings.**

The linearity of the analytical method in the ILab 600 auto-analyzer was studied measuring serial dilutions of D-xylose ranging from 0 to 15 mg/dL as described in Example 3. Dilutions were made using three urine samples from healthy donors. Results show a good correlation with the expected concentration in the analyzed samples. **Figure 15** shows the linearity of absorbance increments over D-xylose concentration in samples measured in the auto-analyzer ILab 600. The samples were tested and interpolated in the previously calculated standard curve.

**Table 6:** Quantification of D-xylose (mg/dL) in an ILab 600 auto-analyzer

| | D-Xylose (mg/dL) | | | | |
|---|---|---|---|---|---|
| **Sample** | **Expected** | **Calculated** | **Mean** | **SD** | **Recovery (%)** |
| **1** | 0.375 | 0.378<br>0.384<br>0.378 | 0.380 | 0.003 | 101 |
| **2** | 2.25 | 2.178<br>2.190<br>2.178 | 2.182 | 0.007 | 97 |

(continued)

| Sample | D-Xylose (mg/dL) Expected | Calculated | Mean | SD | Recovery (%) |
|---|---|---|---|---|---|
| **3** | 5.25 | 4.989<br>4.989<br>4.968 | 4.982 | 0.012 | 95 |

**Adjustment of Cobas c502 (Roche) auto-analyzer settings.**

The linearity of the analytical method in the Cobas c502 auto-analyzer was examined by measuring serial dilutions of D-xylose ranging from 0 to 19 mg/dL as described in Example 3. Dilutions were made using $NaH_2PO_4$ buffer (pH 8.0). Concentrations of D-xylose were determined in triplicate using the Cobas c502 assay and plotted against the expected concentration. **Figure 16** shows the linearity of absorbance increments over D-xylose concentration in buffer samples measured in the auto-analyzer Cobas c502 using the described assay protocol.

**Table 7:** Linearity of absorbance over D-xylose (mg/dL) in a Cobas c502 auto-analyzer

| Sample | $y_0$ | *a* | $R^2$ | *RSD* |
|---|---|---|---|---|
| Buffer + D-xylose | -0.0134 | 0.9965 | 0.9999 | 0.0469 |

**Adjustment of Dimension Vista 1500 (Siemens) auto-analyzer settings.**

The linearity of the analytical method in the Dimension Vista 1500 auto-analyzer was studied measuring serial dilutions of D-xylose ranging from 15 mg/dL as described in Example 3. **Figure 17** shows the linearity of absorbance increments over D-xylose concentration in buffer samples measured in the auto-analyzer Dimension Vista 1500. A serial dilution of D-xylose in $NaH_2PO_4$ buffer (pH 8.0) was used.

**Table 8:** Linearity of absorbance increments over D-xylose concentration (mg/dL) in a Dimension Vista 1500 auto-analyzer

| Sample | $y_0$ | *a* | $R^2$ | *RSD* | LoD (mg/dL) | LoQ (mg/dL) |
|---|---|---|---|---|---|---|
| Buffer + D-xylose | 0.0572 | 0.0330 | 0.9995 | 0.0051 | 0.46 | 1.55 |

In conclusion, determination of D-xylose using the kit or methods disclosed herein is possible in all auto-analyzers.

**Example 5. Measurement of xylose: correlation among the enzymatic and colorimetric methods**

Detection of D-xylose in biological samples (blood and urine) is currently carried out using a colorimetric method based on the chemical reagent phloroglucinol (Xylose Urine Kit, Ref. 7152 FAR Diagnostics). This colorimetric method has been certified and authorized for D-xylose quantification after the oral administration of Gaxilose for clinical diagnosis of hypolactasia, so it was used as reference technique to validate the enzymatic detection of D-xylose based on XylB.

As a step of the full validation of the enzymatic method, some correlation experiments were carried out. In these experiments two urine samples from healthy donors were taken and used for D-xylose serial dilution. Concentrations of D-xylose (0, 0.75 mg/dL, 1.5 mg/dL, 2.25 mg/dL, 3.75 mg/dL, 5.25 mg/dL, 7.5 mg/dL and 15 mg/dL) in urine samples were tested in parallel by both the enzymatic and the colorimetric methods in order to compare them. 0.024 mg/ml of XylB is used in the enzymatic method. **Figure 18** shows the correlation of D-xylose concentrations calculated using both methods. Plots A (sample 1) and C (sample 2) compare D-xylose values calculated with the enzymatic method (white), the colorimetric method (black) and the expected D-xylose concentration (grey). Plots B and D show the linear correlation between the calculated values for both methods in each one of the samples, respectively. The expected D-xylose concentration corresponds to the theoretical xylose concentration added to the sample.

When D-xylose concentration values calculated with each method are compared, a linear correlation is found. Nevertheless, it is remarkable that the unspecific background in D-xylose detection was considerably reduced when the enzymatic method was employed, due to the lower effect that urine yellow color produces in this assay. These results indicate that the enzymatic method is more accurate than the colorimetric method because of its lower unspecific background, which avoids xylose levels overestimation. This color interference also slightly affects the enzymatic method, providing a basal value different to zero in the samples without D-xylose (see samples "basal" in Figure 18A and Figure 18C), but this effect is much lower than the basal value detected in colorimetric test.

**Example 6. Cut-off point of the enzymatic method**

One of the main goals of the present invention is to provide a new method to quantify D-xylose in urine samples (and therefore to classify subjects suspected to be lactose intolerants (hypolactasic)) after the administration of LacTEST®. In order to know whether the new method provides the same classification as the authorized assay (phloroglucinol colorimetric method, Xylose Urine Kit, Ref. 7152 FAR Diagnostics), we analyzed:

a) The correlation of the colorimetric and enzymatic methods in urine samples doped with different xylose concentrations.
b) Concordance between both methods with the doped urine samples.
c) Correlation of both methods in 224 urine samples of patients to whom LacTEST® 0.45g had been previously prescribed by the gastroenterologist.
d) Concordance of both methods with the 224 urine samples of patients to whom LacTEST® 0.45g had been previously prescribed
e) Patient classification according to the results obtained by both methods.
f) Calculation of the Kappa coefficient.

**a) Correlation of the methods with doped urine samples**

Comparison between the colorimetric and the enzymatic methods was performed with 9 basal urine samples (the subjects took water instead of Gaxilose as LacTEST® method indicates) collected under the conditions detailed in LacTEST 0.45g SmPC protocol (Summary of Product Characteristics, AEMPS, Spanish Agency of Medicines and Sanitary Products). The subjects fasted from 10 hours before the collection of urine, which was collected during 5 hours. Part of each urine sample was stored without adding xylose, in order to measure the background. Different D-xylose concentrations (1.5, 3.75, 7.5, 11.25 and 15.0 mg/dL) were added to the samples.

The same samples were tested using both the colorimetric and enzymatic methods. Results were expressed in concentration of D-xylose in each sample (mg/dL) and total amount of D-xylose expressed in total mg. When 9 basal urine samples (without xylose) were tested, the unspecific background detected with the phloroglucinol method ranged between 3.31 mg/dL and 7.87 mg/dL (mean value 5.24 mg/dL). However, when the same samples were assayed using the enzymatic method, lower concentration of xylose (0.26 mg/dL - 0.80 mg/dL) was found as unspecific background (mean value 0.51 mg/dL) (**Table 9**).

**Table 9:** Accumulated xylose in urine samples analyzed by the colorimetric (phloroglucinol) and enzymatic methods.

| | ***Phloroglucinol method*** | ***Enzymatic method*** |
|---|---|---|
| ***Sample (mg/dL)*** | ***(total mg)*** | ***(total mg)*** |
| 1-1 (1.5) | 46.62 | 18.47 |
| 1-2 (3.75) | 71.79 | 41.28 |
| 1-3 (7.5) | 114.09 | 79.51 |
| 1-4 (11.25) | 146.61 | 117.01 |
| 1-5 (15.0) | 183.68 | 154.96 |
| 2-1 (1.5) | 37.26 | 12.91 |
| 2-2 (3.75) | 56.49 | 29.94 |
| 2-3 (7.5) | 88.80 | 57.50 |
| 2-4 (11.25) | 119.31 | 85.17 |
| 2-5 (15.0) | 149.05 | 109.97 |
| 3-1 (1.5) | 35.21 | 11.57 |

(continued)

| Sample (mg/dL) | *Phloroglucinol method (total mg)* | *Enzymatic method (total mg)* |
|---|---|---|
| 3-2 (3.75) | 47.39 | 24.60 |
| 3-3 (7.5) | 66.48 | 46.01 |
| 3-4 (11.25) | 89.24 | 68.45 |
| 3-5 (15.0) | 107.96 | 90.55 |
| 4-1 (1.5) | 28.07 | 9.02 |
| 4-2 (3.75) | 33.91 | **18.56** |
| 4-3 (7.5) | 54.59 | 35.09 |
| 4-4 (11.25) | 63.28 | 50.77 |
| 4-5 (15.0) | 68.53 | 68.08 |
| 5-1 (1.5) | 40.90 | **12.33** |
| 5-2 (3.75) | 52.05 | 26.52 |
| 5-3 (7.5) | 86.41 | 51.16 |
| 5-4 (11.25) | 108.94 | 72.18 |
| 5-5 (15.0) | 126.35 | 99.12 |
| 6-1 (1.5) | 25.42 | 6.63 |
| 6-2 (3.75) | 33.35 | 13.12 |
| 6-3 (7.5) | 40.86 | 22.43 |
| 6-4 (11.25) | 58.02 | 34.36 |
| 6-5 (15.0) | 61.58 | 44.11 |
| 7-1 (1.5) | 28.23 | 7.16 |
| 7-2 (3.75) | 34.00 | 14.36 |
| 7-3 (7.5) | 41.02 | 26.42 |
| 7-4 (11.25) | 58.34 | 39.11 |
| 7-5 (15.0) | 72.00 | 50.98 |
| 8-1 (1.5) | 29.94 | 7.95 |
| 8-2 (3.75) | 36.33 | 15.64 |
| 8-3 (7.5) | 49.10 | 28.48 |
| 8-4 (11.25) | 55.60 | 41.38 |
| 8-5 (15.0) | 60.33 | 54.05 |
| 9-1 (1.5) | 40.76 | **11.17** |
| 9-2 (3.75) | 50.23 | 23.42 |
| 9-3 (7.5) | 75.81 | 46.32 |
| 9-4 (11.25) | 95.35 | 68.33 |
| 9-5 (15.0) | 121.74 | 86.66 |
| - | - | - |

As Table 9 shows, the values obtained using the enzymatic method are systematically lower than those found with the colorimetric method. The rationale behind these results is based on the higher specificity of the enzymatic method in urine samples. These results are also consistent with the results found in the LacTEST® Phase I Clinical Trial (EudraCT: 2005-001899-12), in which the phloroglucinol method was used. In this trial, the presence of a xylose background variable within the patient urine samples was found with the colorimetric method.

There are three different Gaxilose-tests available in the prior art, which have been used in clinical trials: 4-hour urine test, 5-hour urine test and blood test. The calculated xylose cut-off values for normal lactase activity when xylose is quantified by the phloroglucinol colorimetric method are 27.58 and 37.87 mg for the 4- and 5-hour urine tests respectively, and 0.97 mg/dL for the blood test. These cut-offs were calculated through two clinical trials with healthy volunteers and were also confirmed in a third clinical trial with patients who presented clinical symptoms suggestive of lactose intolerance (Hermida C, 2013; Aragón JJ, 2014).

Furthermore, the correlation analysis of the results of **Table 9** was performed (**Figure 19**). The coefficients of determination ($R^2$) were calculated both with mg/dL (left panel) and total mg (right panel), being 0.90 and 0.96 respectively. The corresponding coefficients of correlation together with their confidence intervals (CI) found both with mg/dL and total mg were 0.9511 (95%CI: 0.9122-0.9730) and 0.9800 (95% CI: 0.9637-0.9890), respectively. The enzymatic method was performed in an ILab 600 auto-analyzer.

The D-xylose cut-off value for lactase activity when xylose is detected with the phloroglucinol colorimetric method is 37.87 mg in the 5-hour urine test. This value allows classification of subjects as normolactasic or negative (xylose ≥ 37.87 mg) or hypolactasic or positive (xylose < 37.87 mg). The value was calculated from two clinical trials with healthy volunteers, using excreted xylose amount mean value in 5-hour urine ± 1.96 times the standard deviation, and has also been confirmed in a third clinical trial with patients who presented clinical symptoms suggestive of lactose intolerance. Xylose results obtained in the 45 samples analyzed above were classified as positive or negative according to the reference value of the phloroglucinol method: if the value is ≥ 37.87 mg the patient is classified as normolactasic, and *vice versa.*

From the equation calculated with the total mg (**Figure 19 B**), the value equivalent in the enzymatic method to the phloroglucinol cut-off was calculated, obtaining a value of 16.47 mg. The samples were classified as positive or negative according to this value and 3 discrepancies were found in the theoretical classification of the samples with both methods as "normolactasic" and "hypolactasic" (bold values in **Table 9**).

**b) Concordance between both methods with the doped urine samples.**

Using these values as reference, the receiver operating characteristic (ROC) curve was generated (**Figure 20**) in order to compare phloroglucinol (colorimetric method) results with the results obtained using the enzymatic method. This comparison analyzes if both xylose quantification methods have diagnostic similarity.

The area under the curve (AUC) was calculated from the values obtained by the enzymatic method, using as reference the data obtained with the colorimetric method. The AUC value obtained was 0.966 (CI. 95%: 0.918- 1.000), which is a good preliminary result. From the same curve, a xylose cut-off value for the enzymatic method of 22.43 mg was obtained, giving only 3 discrepancies between both methods.

The degree of agreement between the methods in the classification of the 45 samples as normolactasic or hypolactasic was evaluated calculating the Kappa coefficient. We obtained a Kappa coefficient of 0.8258 (CI 95%: 0.6383-1.0000), a fair coefficient considering the low number of observations, which is reflected in the wide range of the confidence interval. This result indicates that the enzymatic method is reliable and allows a classification of the samples as accurate as that of the colorimetric method.

**c) Correlation of both methods with 224 urine samples of patients to whom LacTEST® 0.45g had been previously prescribed by the gastroenterologist.**

Taking into consideration these preliminary results, and as part of the validation process of the new enzymatic method, an additional study was performed with a higher number of samples from patients who had visited the gastroenterologist and to whom LacTEST 0.45g had been prescribed as diagnostic test. The study was performed with 224 urine samples from 10 different hospitals. Each hospital provided an aliquot of the patient's collected urine sample, with the consent of the laboratory authorizing the exclusive xylose measurement for the comparison of both methods. Xylose was quantified in each sample at the same laboratory using both methods: the colorimetric method (phloroglucinol) and the enzymatic method, using a Cobas c502 analyzer from Roche, as described above. Then the results of both methods were compared. Sample testing was performed independently of the patient diagnosis performed in each hospital. **Figure 21** shows the correlation of the xylose amount detected by the colorimetric and enzymatic methods.

As it has been disclosed previously, LacTEST® 0.45g is a diagnostic drug authorized to provide a "positive" or "negative" diagnosis of hypolactasia. Interpolating the phloroglucinol xylose cut-off value (37.87 mg) in the correlation linear regression, a xylose cut-off value for the enzymatic assay of 22.07 mg was obtained. The classification of the 224 samples according to this new value resulted in 18 discrepancies with regard to the diagnosis previously made by the medical doctors. Consequently, we considered advisable to calculate this cut-off value generating the ROC curve, as performed in section b) with the doped urine samples

**d) Concordance of both methods with the 224 urine samples of patients to whom LacTEST® 0.45g had been previously prescribed**

In order to calculate the equivalence between the results of the phloroglucinol method and the enzymatic method, the ROC curve was generated with the values of xylose obtained with the 224 urine samples, taking the phloroglucinol method as reference (authorized method when the analysis was conducted) (**Figure 22**).

According to the data obtained from the ROC curve, the cut-off value for the enzymatic method of the invention was defined as "Normolactasic if ≥ 19.18 mg". In this case, the AUC of the ROC curve was 0.989 (CI 95%: 0.979-0.998) (**Figure 22**). As it’s observed, the lower limit of the confidence interval is very high, which indicates a robust result due to the very much higher number of observances (224 samples), reflected in the minimum range of the confidence interval. These data show two methods essentially equivalent.

In order to re-evaluate the robustness of the equivalence of these methods, the Kappa coefficient was calculated. The most agreed criteria used to interpret the evaluated agreement degree using the Kappa coefficient are summarized in **Table 10.**

**Table 10.** Criteria to interpret the agreement degree evaluated with the Kappa coefficient.

| **Criteria for interpreting the degree of agreement** | | | |
|---|---|---|---|
| **Kappa** | **Agreement** | **Kappa** | **Agreement** |
| < 0.20 | Slight | <0.40 | Poor |
| 0.21-0.40 | Fair | 0.40-0.75 | Fair to good |
| 0.41-0.60 | Moderate | 0.76-1.00 | Excellent |
| 0.61-0.80 | Substantial | | |
| 0.81-1.00 | Almost perfect | | |
| (Landis, 1977) | | (Fleiss, 1981) | |

The value of the Kappa coefficient for the 224 samples analyzed using both methods is 0.9347 (CI 95%: 0.8871-0.9822). The CI lower limit was 0.8871, being considerably higher than 0.81, considered by Landis and Koch as an excellent agreement value. Thus, the equivalence of both methods is fully demonstrated. In the light of all these data analyzed, it can be concluded that both methods can classify a patient as "normolactasic" or "hypolactasic" after LacTEST 0.45g intake.

**e) Patient classification according to the results obtained by both methods.**

There were seven discrepancies between both methods (See **Table 11**).

**Table 11.** Discrepancies observed using the enzymatic method and the phloroglucinol method.

| **Patient** | **Phloroglucinol Method (mg/dL)** | **Phloroglucinol Method (total mg)** | | **Enzymatic Method (mg/dL)** | **Enzymatic Method (total mg)** | | **Urine Volume (mL)** |
|---|---|---|---|---|---|---|---|
| 6 | 5.96 | 35.74 | + | 4.07 | 23.28 | - | 600 |
| 8 | 4.83 | 24.14 | + | 4.10 | 20.65 | - | 500 |
| 62 | 3.00 | 54.08 | - | 0.88 | 15.75 | + | 1800 |
| 125 | 6.01 | 36.08 | + | 3.90 | 23.40 | - | 120 |
| 145 | 3.57 | 35.68 | + | 2.29 | 22.85 | - | 200 |
| 173 | 4.77 | 34.85 | + | 3.90 | 28.43 | - | 730 |

(continued)

| Patient | Phloroglucinol Method (mg/dL) | Phloroglucinol Method (total mg) | | Enzymatic Method (mg/dL) | Enzymatic Method (total mg) | | Urine Volume (mL) |
|---|---|---|---|---|---|---|---|
| 207 | 2.93 | 29.25 | + | 2.38 | 23.75 | - | 200 |
| + Hypolactasic - Normolactasic<br>Phloroglucinol Total mg (Normolactasic if ≥37.87 mg)<br>Enzymatic Total mg (Normolactasic if ≥19.18 mg) | | | | | | | |

Even though the unspecific background detected by the phloroglucinol is generally high, some urine samples presented a very small background. If the background of a urine sample is very small, due to the specific characteristics of the sample, the amount of xylose detected by the phloroglucinol method would be underestimated in its comparison with the cut-off value obtained with the different urine samples of the previous clinical trials (phase I, Ib and IIb-III clinical trials). For some patients (8, 173 and 207), xylose values (mg/dL) obtained with the phloroglucinol and the enzymatic methods are similar (differences smaller than 1 mg/dL between them). It would be concluded that these urine samples present a very low background that does not interfere with the phloroglucinol method. This could be interpreted as the xylose values obtained by the phloroglucinol method being unusually low, and therefore being underestimated in front of the cut-off. If these urine samples had a normal background, the amount of xylose detected by the phloroglucinol method would be higher than the cut-off, changing the classification of these patients (at least patients 173 and 207) obtained by phloroglucinol method to the classification obtained by the enzymatic method.

On the other hand, the amount of xylose in the urine samples of discrepant patients 6, 125 and 145 is very close to the phloroglucinol cut-off value, taking into the account that the range of measurement error of the phloroglucinol method is 0.48-6.45% (indicated in LacTEST 0.45 g SmPC). The difference in percentage between the amount of xylose in the urine samples of patients 6, 125 and 145 and the phloroglucinol cut-off is 5.62%, 4.73% and 5.78%, respectively. This suggests that some of these patients could have been wrongly classified using the phloroglucinol method. In these cases, it would be recommended for the physician to advice these patients that their lactase deficiency is very mild.

Finally, sample number 62 showed a big difference between both methods, presenting a positive result using the enzymatic method and, on the contrary, a negative result using the phloroglucinol method. This might be possibly due to the excessive volume of urine collected after 5 hours (1800 mL), which could amplify a minimum interference in the urine when xylose is measured with the phloroglucinol method and not in the case of the enzymatic measurement. The discrepancies analyzed are summarized in **Table 12.**

**Table 12.** First analysis of the discrepancies.

| Patient | Phloroglucinol Method (mg/dL) | Phloroglucinol Method (total mg) | Enzymatic Method (mg/dL) | Enzymatic Method (total mg) | Urine Volume (mL) | Discrepancies |
|---|---|---|---|---|---|---|
| 8 | 4.83 | 24.14 | 4.10 | 20.65 | 500 | Similar xylose values (mg/dL) obtained by both methods |
| 173 | 4.77 | 34.85 | 3.90 | 28.43 | 730 | |
| 207 | 2.93 | 29.25 | 2.38 | 23.75 | 200 | |
| 6 | 5.96 | 35.74 | 4.07 | 23.28 | 600 | Phloroglucinol values very close to the cut-off |
| 125 | 6.01 | 36.08 | 3.90 | 23.40 | 120 | |
| 145 | 3.57 | 35.68 | 2.29 | 22.85 | 200 | |
| 62 | 3.00 | 54.08 | 0.88 | 15.75 | 1800 | Excessive urine volume |

To better clarify these discrepancies, the different physicians who prescribed LacTEST 0.45 g to the discrepant patients (6, 8, 62, 125, 145, 173 and 207) were contacted according to Regulatory Authorities, in order to be informed about each patient follow up. Concerning the samples of patients 6 and 8 (from Hospital 1) these urine samples presented a brick-red colored pellet when they were thawed prior to xylose measurement. For this reason, the determination of the amount of xylose in these two samples was repeated using the colorimetric and the enzymatic methods, in order to confirm if the red pellet could have interfered in the previous xylose measurement. It must be noted that the samples were not contaminated.

As it can be observed in **Table 13,** the results obtained by the enzymatic method provided both a normolactasic diagnosis for the two patients, and the obtained values were very similar among repetitions. However, xylose values obtained by the phloroglucinol method in the Hospital 2 were lower than the cut-off, indicating hypolactasia in both samples, being very different between them for patient 8.

**Table 13.** Xylose values of patients 6 and 8 measured in two hospitals and by both analytical methods.

| **Patient** | **Phloroglucinol Hospital 2** Measurement 1 **(mg/5 hours)** | **Phloroglucinol Hospital 2** Measurement 2 **(mg/5 hours)** | **Enzymatic Hospital 2** Measurement 1 **(mg/5 hours)** | **Enzymatic Hospital 2** Measurement 2 **(mg/5 hours)** | **Phloroglucinol Hospital 1 (mg/ 5 hours)** |
|---|---|---|---|---|---|
| **6** | 33.21 | 35.74 | 24.42 | 23.28 | 39.5 |
| **8** | 33.98 | 24.14 | 20.50 | 20.65 | 52.3 |

According to the information provided by the responsible physician (Hospital 1), these patients were previously classified as normolactasic with phloroglucinol xylose values of 39.5 mg and 52.3 mg for patients 6 and 8 respectively. Therefore, some problem may have occurred during the handling of these samples. However, we do not consider this problem as related to the freezing and thawing process, since it was demonstrated in the validation of the phloroglucinol method that urine samples could resist 3 freezing and thawing cycles.

In this case, the physician classified both patients as normolactasic, in agreement with the results obtained later with the new enzymatic method. According to the physician, these patients feel currently well, without symptoms of lactose intolerance. Taking into account the medical judgment and the follow-up of the two patients, we estimate that xylose values obtained by the phloroglucinol method in the Hospital 1 were the wright ones. Therefore, after a more complete analysis and taking into consideration the judgment of the physician, we consider that the classification of both patients should change to normolactasic, being no longer discrepant to calculate the diagnostic performance of the new enzymatic method, as it is mentioned below.

Concerning these discrepancies, the classification of patients 6 and 8 has been corrected taking into account the criteria described previously considering these patients as normolactasic, assuming that the values obtained by the enzymatic method are right (see **Table 14**).

**Table 14.** Calculation of the cut-off with the 224 urine samples after correcting the two discrepancies, where the cut-off is written in bold font.

| Test | Sensit. | Specif. | PPV | NPV | LR+ | LR- | TP | TN | FP | FN | Sensit+Specif. | Acc. |
|---|---|---|---|---|---|---|---|---|---|---|---|---|
| 18.150 | 0.921 | 0.993 | 0.988 | 0.950 | 124.382 | 0.079 | 82 | 134 | 1 | 7 | 1.914 | 0.964 |
| 18.300 | 0.933 | 0.993 | 0.988 | 0.957 | 125.899 | 0.068 | 83 | 134 | 1 | 6 | 1.925 | 0.969 |
| 18.530 | 0.944 | 0.993 | 0.988 | 0.964 | 127.416 | 0.057 | 84 | 134 | 1 | 5 | 1.936 | 0.973 |
| **19.180** | **0.955** | **0.993** | **0.988** | **0.971** | **128.933** | **0.045** | **85** | **134** | **1** | **4** | **1.948** | **0.978** |
| 19.200 | 0.955 | 0.985 | 0.977 | 0.971 | 64.466 | 0.046 | 85 | 133 | 2 | 4 | 1.940 | 0.973 |
| 19.553 | 0.955 | 0.978 | 0.966 | 0.971 | 42.978 | 0.046 | 85 | 132 | 3 | 4 | 1.933 | 0.969 |
| 19.710 | 0.955 | 0.970 | 0.955 | 0.970 | 32.233 | 0.046 | 85 | 131 | 4 | 4 | 1.925 | 0.964 |

Note: The cut-off from the ROC curve is 19.18 mg of xylose. Superior or equal values to 19.18 mg of xylose are considered to be normolactasic patients. Minor values than 19.18 mg of xylose are considered to be hypolactasic patients.
Sensit = Sensitivity, Specif. = Specificity, PPV = Positive predictive value, NPV = Negative predictive value, LR+ = Positive likelihood ratio. LR- = Negative likelihood ratio, TP = True positive, TN = True negative, FP = False positive, FN = False negative, Acc = Accuracy.

Assuming this analysis of the discrepancies, the ROC curve was recalculated with the 224 samples, correcting the diagnostic classification (hypolactasic/normolactasic) of patients 6 and 8 (**Figure 23**). The values of sensitivity, specificity, positive and negative predictive values, positive and negative likelihood ratios and accuracy obtained from the ROC curve are shown in **Table 14.** These values are obtained taking the phloroglucinol method as reference.

**f) Calculation of the Kappa coefficient**

As it was expected, after correcting the two discrepancies mentioned above, the AUC changed from 0.989 to 0.991. An increase is also observed in the value of the Kappa coefficient, that changed from 0.9347 (I.C. 95%: 0.8871-0.9822) to 0.9531 (I.C. 95% 0.9125-0.9937), being the lower limit of the confidence interval of 0.9125 higher than 0.81, considered by Landis and Koch as a value of excellent agreement. Both methods are able to classify as hypolactasic 85 patients from the 224 analyzed, and 134 as normolactasic. There are only four discrepancies in the patients classified as hypolactasic with the colorimetric method, which the enzymatic method considers normolactasic, and one patient classified as hypolactasic with the enzymatic method and normolactasic with the colorimetric method. The discrepancies between the two methods are 5 of 224 samples analyzed (2.32%) (**Figure 24**).

In the light of all the data previously analyzed, it can be concluded that the phloroglucinol and the enzymatic methods are equivalent methods and can be indistinctly used to classify patients as "normolactasic" or "hypolactasic" after LacTEST 0.45 g intake. However, the enzymatic method is more accurate because of its lower unspecific background that avoids overestimation of xylose concentration.

**Example 7. Determination of xylose cut-off values for different Gaxilose doses using the enzymatic method.**

The xylose cut-off value with which it is determined if a patient is hypolactasic or not depends on the administered dose of Gaxilose and the xylose quantification method used. **Table 15** discloses the cut-off values after ingestion of 0.1125 g, 0.225 g, 0.45 g, 0.9 g, 2.7 g and 5.4 g of Gaxilose when xylose is quantified by the phloroglucinol method in 4-hour or 5-hour urine.

**Table 15:** Xylose cut-off values after Gaxilose ingestion using the colorimetric method.

| Gaxilose dose | 5-hour urine | 4-hour urine |
|---|---|---|
| **0.1125 g** | 16.72 mg | 11.88 mg |
| **0.225 g** | 28.08 mg | 20.62 mg |
| **0.45 g** | 37.87 mg | 27.58 mg |
| **0.9 g** | 41.35 mg | 33.04 mg |
| **2.7 g** | 69.75 mg | 45.58 mg |
| **5.4 g** | 110.12 mg | 87.96 mg |

The objective of this example is to calculate xylose reference values in 5-hour and 4-hour urine quantifying this monosaccharide by the new enzymatic method described herein, for Gaxilose doses of 0.1125 g, 0.225 g, 0.45 g, 0.9 g, 2.7 g and 5.4 g. Samples were analyzed by the new enzymatic xylose quantification method and by the phloroglucinol colorimetric reaction and xylose cut-off values for the new enzymatic technique were obtained from the ROC curves generated taking as reference the phloroglucinol results, with the phloroglucinol xylose cut-off value for each Gaxilose dose as diagnostic reference.

The samples used in the study were prepared doping basal urine samples from healthy donors with different amounts of xylose. For that purpose, total urine from 12 healthy donors was collected, but taking water instead of the diagnostic drug (Gaxilose). Total excreted urine was collected during the following 5 hours, measuring its volume 4 and 5 hours after beginning the collection. This urine is considered total basal urine. Aliquots of the 12 basal urines were stored at -20°C until use.

In order to calculate the cut-off value for the different Gaxilose doses, basal urine samples doped with xylose were measured by the phloroglucinol colorimetric reaction and by the enzymatic method. Since the cut-off values are expressed in total amount of xylose in 5-hour urine (mg), aliquots of 1.2 mL of each basal urine were doped with the corresponding xylose amount, taking into consideration the volume of urine excreted in each case, to obtain the final xylose amounts summarized in **Table 16.** These amounts were chosen based on preliminary non-published laboratory results.

**Table 16.** Amounts of xylose (mg) in total 5-hour urine used to calculate xylose cut-off values for each theoretical Gaxilose dose.

| Theoretical dose of Gaxilose | 0.1125 g | 0.225 g | 0.45 g | 0.9 g | 2.7 g | 5.4 g |
|---|---|---|---|---|---|---|
| Total amount of xylose (mg) | 0.00 | 0.00 | 0.00 | 0.00 | 0.00 | 0.00 |
| | 1.00 | 5.00 | 5.00 | 15.00 | 30.00 | 70.00 |
| | 1.50 | 6.00 | 10.00 | 20.00 | 35.00 | 76.00 |
| | 2.00 | 7.50 | 15.00 | 22.00 | 40.00 | 80.00 |
| | 2.50 | 8.00 | 17.00 | 24.00 | 42.00 | 82.00 |
| | 2.75 | 8.50 | 17.50 | 26.00 | 43.50 | 85.00 |
| | 3.00 | 9.00 | 18.00 | 28.00 | 45.00 | 87.00 |
| | 3.25 | 9.50 | 18.25 | 30.00 | 47.50 | 88.00 |
| | 3.50 | 10.00 | 18.50 | 31.00 | 49.00 | 89.00 |
| | 3.75 | 10.50 | 18.75 | 32.00 | 50.00 | 90.00 |
| | 4.00 | 10.75 | 19.00 | 33.00 | 51.00 | 91.00 |
| | 4.25 | 11.00 | 19.25 | 33.50 | 52.00 | 92.00 |
| | 4.50 | 11.25 | 19.50 | 33.75 | 53.00 | 93.00 |
| | 4.75 | 11.50 | 19.75 | 34.00 | 54.00 | 94.00 |
| | 5.00 | 12.00 | 20.00 | 34.25 | 55.00 | 95.00 |
| | 5.25 | 12.50 | 20.50 | 34.50 | 56.00 | 96.00 |
| | 5.50 | 13.00 | 21.00 | 34.75 | 57.00 | 97.00 |
| | 5.75 | 13.50 | 21.50 | 35.00 | 58.00 | 98.00 |
| | 6.00 | 14.00 | 22.00 | 35.25 | 59.00 | 99.00 |
| | 6.50 | 15.00 | 23.00 | 35.50 | 60.00 | 100.00 |
| | 8.00 | 16.00 | 24.00 | 36.00 | 62.00 | 102.00 |
| | 10.00 | 17.00 | 26.00 | 36.50 | 64.00 | 104.00 |
| | 15.00 | 18.00 | 28.00 | 37.00 | 66.00 | 106.00 |
| | - | 19.00 | 30.00 | 38.00 | 68.00 | 108.00 |
| | - | 20.00 | 32.00 | 39.00 | 70.00 | 110.00 |
| | - | 21.00 | 34.00 | 40.00 | 72.00 | 112.00 |
| | - | 22.00 | 36.00 | 42.00 | 74.00 | 115.00 |
| | - | 23.00 | 38.00 | 45.00 | 76.00 | 120.00 |
| | - | 24.00 | 40.00 | 47.50 | 78.00 | 125.00 |
| | - | 25.00 | 45.00 | 50.00 | 80.00 | 130.00 |

For each one of the xylose amounts, three samples were prepared separately with three different basal urines (except for the samples containing 0 mg of xylose, which were prepared separately from the 12 collected urines). To determine the distribution of the urine samples, the 12 urines were randomized in 32 sets of 12 variables per set, and distributed following the order obtained as indicated in **Table 17.**

**Table 17.** Urine samples used to prepare each final amount of xylose in 5-hour urine.

| Xylose total amount in 5-hour urine (mg) | Urine samples | | | Xylose total amount in 5-hour urine (mg) | Urine samples | | |
|---|---|---|---|---|---|---|---|
| 0.00 | all of them | | | 17.50 | C | I | G |
| 1.00 | J | C | I | 18.00 | D | A | B |
| 1.50 | E | B | D | 18.25 | D | C | E |
| 2.00 | A | L | K | 18.50 | K | I | F |
| 2.50 | H | G | F | 18.75 | J | L | A |
| 2.75 | A | J | L | 19.00 | H | B | G |
| 3.00 | C | E | B | 19.25 | L | B | I |
| 3.25 | K | H | I | 19.50 | D | F | H |
| 3.50 | G | D | F | 19.75 | A | G | E |
| 3.75 | L | D | J | 20.00 | C | K | J |
| 4.00 | H | F | B | 20.50 | K | C | B |
| 4.25 | G | C | I | 21.00 | H | A | L |
| 4.50 | A | K | E | 21.50 | E | I | F |
| 4.75 | H | E | K | 22.00 | G | D | J |
| 5.00 | I | A | D | 23.00 | D | G | I |
| 5.25 | F | C | J | 24.00 | J | H | K |
| 5.50 | G | B | L | 25.00 | L | B | E |
| 5.75 | H | E | F | 26.00 | C | F | A |
| 6.00 | J | L | I | 28.00 | E | F | H |
| 6.50 | C | A | K | 30.00 | K | C | J |
| 7.50 | D | B | G | 31.00 | A | D | L |
| 8.00 | D | I | H | 32.00 | I | B | G |
| 8.50 | E | J | C | 33.00 | C | D | B |
| 9.00 | F | B | A | 33.50 | H | L | E |
| 9.50 | L | K | G | 33.75 | G | I | F |
| 10.00 | K | I | J | 34.00 | A | K | J |
| 10.50 | C | L | H | 34.25 | B | I | H |
| 10.75 | E | D | F | 34.50 | D | F | C |
| 11.00 | B | G | A | 34.75 | E | K | L |
| 11.25 | C | K | B | 35.00 | A | J | G |
| 11.50 | I | H | J | 35.25 | F | I | A |
| 12.00 | A | D | E | 35.50 | J | H | B |
| 12.50 | L | G | F | 36.00 | K | D | G |
| 13.00 | L | E | J | 36.50 | E | L | C |
| 13.50 | K | D | H | 37.00 | K | C | A |
| 14.00 | I | C | B | 38.00 | J | D | B |
| 15.00 | A | F | G | 39.00 | H | L | F |

(continued)

| Xylose total amount in 5-hour urine (mg) | Urine samples | | | Xylose total amount in 5-hour urine (mg) | Urine samples | | |
|---|---|---|---|---|---|---|---|
| 16.00 | F | H | L | 40.00 | E | I | G |
| 17.00 | E | K | J | 42.00 | I | G | B |

**Table 17 (continued).** Urine samples used to prepare each final amount of xylose in 5-hour urine.

| Xylose total amount in 5-hour urine (mg) | Urine samples | | | Xylose total amount in 5-hour urine (mg) | Urine samples | | |
|---|---|---|---|---|---|---|---|
| 43.50 | J | C | A | 85.00 | D | J | G |
| 45.00 | L | E | H | 87.00 | L | F | E |
| 47.50 | F | D | K | 88.00 | D | I | A |
| 49.00 | C | I | F | 89.00 | H | G | C |
| 50.00 | D | K | G | 90.00 | B | K | J |
| 51.00 | E | B | A | 91.00 | L | H | D |
| 52.00 | J | L | H | 92.00 | B | I | G |
| 53.00 | G | B | K | 93.00 | F | E | K |
| 54.00 | F | I | A | 94.00 | A | C | J |
| 55.00 | L | C | E | 95.00 | F | D | A |
| 56.00 | D | J | H | 96.00 | B | I | J |
| 57.00 | H | A | G | 97.00 | E | H | L |
| 58.00 | C | I | J | 98.00 | G | C | K |
| 59.00 | E | F | D | 99.00 | B | D | J |
| 60.00 | B | L | K | 100.00 | K | H | G |
| 62.00 | L | I | B | 102.00 | F | L | A |
| 64.00 | J | K | C | 104.00 | C | E | I |
| 66.00 | A | G | H | 106.00 | I | D | C |
| 68.00 | F | D | E | 108.00 | E | K | L |
| 70.00 | G | J | C | 110.00 | F | B | G |
| 72.00 | F | K | D | 112.00 | J | A | H |
| 74.00 | L | I | B | 115.00 | D | H | A |
| 76.00 | A | E | H | 120.00 | F | G | E |
| 78.00 | C | I | A | 125.00 | B | L | J |
| 80.00 | H | F | E | 130.00 | C | K | I |
| 82.00 | K | B | L | | | | |

The colorimetric detection of xylose with phloroglucinol was carried out as explained in LacTEST 0.45 g summary of product characteristics, and the xylose quantification by the enzymatic method was performed as detailed in LacTEST 0.45 g summary of product characteristics, adjusting the instrumental application to the ILab Aries automated analyzer, from Werfen.

After quantifying xylose in the 5-hour and 4-hour urine samples by both methods, the xylose cut-offs of the enzymatic method were determined for each theoretical Gaxilose dose with the ROC curve, using the phloroglucinol results as reference. For each theoretical Gaxilose dose, samples were classified with the phloroglucinol cut-off values previously reported in patent US Patent Application 13/350,097. ROC curves were obtained using the XLSTAT software (Addinsoft).

The volumes of total urine excreted during 4 and 5 hours by the 12 healthy volunteers are indicated in **Table 18.**

**Table 18.** Volume of total urine excreted during 4 and 5 hours by each healthy donor.

| Urine | 4-hour urine volume (mL) | 5-hour urine volume (mL) |
|---|---|---|
| A | 450 | 710 |
| B | 360 | 637 |
| C | 410 | 410 |
| D | 550 | 672 |
| E | 300 | 490 |
| F | 575 | 770 |
| G | 920 | 1090 |
| H | 700 | 810 |
| I | 715 | 890 |
| J | 920 | 1107 |
| K | 465 | 465 |
| L | 336 | 336 |

Basal urine samples doped with different amounts of xylose were analyzed by both the phloroglucinol reaction and the enzymatic method to determine total xylose amount in 5-hour urine. After that, total amount of xylose in 4-hour urine was calculated for both analytical techniques. ROC curves for each assay are depicted in **Figures 25 to 30** for each Gaxilose dose.

The xylose cut-off of the enzymatic method in 5-hour urine for the 0.45 g Gaxilose dose was 20.93 mg. This cut-off had already been determined in the concordance study with the 224 urine samples from patients to whom LacTEST 0.45 g had been prescribed (Example 6d). After analyzing the samples by both analytical procedures, the obtained cut-off value was 19.18 mg. Both values are very close and the difference between them is lower than the measurement error of the method indicated in LacTEST 0.45 g summary of product characteristics. Since the previous analysis in Example 6 was directly performed with samples from real patients, the value of **19.18 mg** shall remain as the cut-off value for 0.45 g Gaxilose dose in 5-hour urine. However, the similarity between both values indicates that the analysis of basal urine samples doped with different xylose amounts is also valid to calculate the remaining cut-off values. The xylose cut-off in 4-hour urine for a Gaxilose dose of 0.45 g was calculated from the corresponding ROC curve (**Figure 25B**), and was found to be **15.89 mg.**

For the Gaxilose dose of 0.1125 g, it should be taken into account that samples with xylose concentration lower than 0.5 mg/dL cannot be analyzed, which is the limit of quantification of the phloroglucinol reaction, as indicated in LacTEST 0.45 g summary of product characteristics. Therefore, the calculation of xylose cut-off values was carried out without including these samples. Xylose cut-off values were found to be **5.06 mg** and **3.95 mg** in 5-hour and 4-hour urine, respectively, for a Gaxilose dose of 0.1125 g **(Figure 26**).

Xylose cut-off values were found to be **12.55 mg** and **11.74 mg** in 5-hour and 4-hour urine, respectively, for a Gaxilose dose of 0.225 g **(Figure 27**).

Xylose cut-off values were found to be **27.98 mg** and **20.77 mg** in 5-hour and 4-hour urine, respectively, for a Gaxilose dose of 0.9 g **(Figure 28**).

Xylose cut-off values were found to be **50.48 mg** and **31.28 mg** in 5-hour and 4-hour urine, respectively, for a Gaxilose dose of 2.7 g **(Figure 29**).

Xylose cut-off values were found to be **87.40 mg** and **68.38 mg** in 5-hour and 4-hour urine, respectively, for a Gaxilose dose of 5.4 g **(Figure 30**).

The xylose cut-off values of the enzymatic xylose quantification method in 5-hour and 4-hour urine for the different theoretical Gaxilose doses are summarized in **Table 19,** together with the reference values obtained for the phloroglucinol method.

**Table 19.** Xylose cut-off values in 5-hour and 4-hour urine for the six theoretical Gaxilose doses, for the enzymatic method and the phloroglucinol reaction.

| Gaxilose Dose | Xylose cut-off (mg) | | | |
|---|---|---|---|---|
| | Phloroglucinol reaction (5h) | Enzymatic method (5h) | Phloroglucinol reaction (4h) | Enzymatic method (4h) |
| 0.1125 g | 16.72 | 5.06 | 11.88 | 3.95 |
| 0.225 g | 28.08 | 12.55 | 20.62 | 11.74 |
| 0.45 g | 37.87 | 19.18 | 27.58 | 15.59 |
| 0.9 g | 41.35 | 27.98 | 33.04 | 20.77 |
| 2.7 g | 69.75 | 50.48 | 45.58 | 31.28 |
| 5.4 g | 110.12 | 87.40 | 87.96 | 68.38 |

**Example 8. D-xylose detection by absorbance in blood samples by the enzymatic method of invention based in a single xylose dehydrogenase enzyme XylB.**

Detection and quantification of xylose in blood serum samples using the XylB enzymatic system was performed in 96-wells microtiter plates. Due to the fact that xylose concentration in blood is much lower than in urine, the amount of serum sample required in the enzymatic test must be increased in order to accomplish an accurate detection. Thus, the optimal amount of serum sample in the enzymatic assay was established in 100 μL, in a final reaction volume of 300 μL (dilution 1:3). Reactions were carried out in 50 mM phosphate buffer pH 8.0 in the presence of the cofactor $NAD^+$ at 1 mM (xylose concentrations in blood samples are lower so concentration of $NAD^+$ was decreased from 5 to 1 mM). The mixture of buffer, $NAD^+$ and sample must be initially incubated after addition of XylB (0.045 mg of enzyme). Differences of absorbance before and after the reaction initiated by XylB are proportional to the concentration of xylose present in the analyzed sample. Using these reaction conditions, 13 blood sera from healthy donors spiked with different concentration of xylose were analyzed. Every individual serum was aliquoted and spiked with xylose concentrations ranging from 0.15 μM to 100 μM. All of the analyzed serum serial dilutions showed a good response and linearity when variations of absorbance were recorded and plotted against their xylose concentration **(Figure 31**).

The enzymatic method of xylose determination based on XylB is very effective when urine samples are measured, since the behavior of the enzyme in this medium is identical to that presented in buffered aqueous media. However, xylose detection is different when samples are blood serum. In this case, it has been observed that the behavior of the enzyme is slightly different than that presented in sample buffer (**Figure 31**). In all the analyzed cases, it has been observed that the absorbance differences obtained are generally lower when serum samples are used instead of buffer samples. Thus, to analyze samples with increasing concentrations of xylose in both buffer and serum, linear behavior is observed in both cases but the lines are not parallel neither have the same intercept. Several studies in our laboratory suggest that this effect is due to side reactions which could interfere with the NADH generated in the reaction, and the immediate effect is that the use of reference samples of buffer concentration does not allow effective direct quantification in a serum sample. In addition, it has been observed that different sera also show different behaviors; even if their regression lines have greater similarity between them than with samples prepared in buffer, they are different between them. This prevents the efficient use of serum samples of known xylose concentration for determining the concentration of xylose in a different serum.

Despite these drawbacks, determination of xylose in blood serum samples is possible in the described reaction conditions (in 96-well microtiter plates) when basal serum samples are used as internal reference. In this method, a basal sample of serum which does not contain any xylose sample is measured in order to stablish the unspecific background in an individual blood sample. After that, basal serum is spiked with a known xylose concentration which will be used as reference point. Using the two absorbance values obtained from basal and spiked serum samples as reference curve, it is possible to calculate xylose concentration in samples from the referenced individual. This method has been successfully used in serum samples from different individuals. **Figure 32** shows the linearity of expected D-xylose concentrations over calculated D-xylose concentrations with the enzymatic method (A), and the enzymatic method using the basal serum as internal reference (B).

## REFERENCES

Abdel-Ghany SE, Day I, Heuberger AL, Broeckling CD, Reddy AS. "Metabolic engineering of Arabidopsis for butanetriol production using bacterial genes". Metab Eng (2013) 20, 109-20.

Aoki S, Ishikura S, Asada Y, Usami N, Hara A. "Identity of dimeric dihydrodiol dehydrogenase as NADP+-dependent D-xylose dehydrogenase in pig liver". Chem Biol Interact (2001) 130-132, 775-84.

Aragón JJ, Hermida C, Martinez-Costa OH, Sanchez V, Martin I, Sanchez JJ, Codoceo R, Cano JM, Cano A, Crespo L, Torres Y, Garcia FJ, Fernández-Mayoralas A, Solera J, Martinez P. "Noninvasive diagnosis of hypolactasia with 4-Galactosylxylose (Gaxilose): a multicentre, open-label, phase IIB-III nonrandomized trial". J Clin Gastroenterol (2014) 48, 29-36.

Arola H. "Diagnosis of hypolactasia and lactose malabsorption". Scand J Gastroenterol (1994) 202 (29 Suppl) 22-35.

Asada Y, Aoki S, Ishikura S, Usami N, Hara A. "Roles of His-79 and Tyr-180 of - xylose/dihydrodiol dehydrogenase in catalytic function". Biochem Biophys Res Commun (2000) 278, 333-7.

Berghäll S, Hilditch S, Penttilä M, Richard P. "Identification in the mould Hypocrea jecorina of a gene encoding an NADP+: D-xylose dehydrogenase". FEMS Microbiol Lett (2007) 277, 249-53.

Cao Y, Xian M, Zou H, Zhang H. "Metabolic engineering of Escherichia coli for the production of xylonate". PLoS ONE (2013) 8 (7) e67305.

Chun BW, Dair B, Macuch PJ, Wiebe D, Porteneuve C, Jeknavorian A. "The development of cement and concrete additive: based on xylonic acid derived via bioconversion of xylose". Appl Biochem Biotech (2006) 131: 645-58.

Davidson GP, Robb TA. "Value of breath hydrogen analysis in management of diarrheal illness in childhood: Comparison with duodenal biopsy". J Pediatr Gastroenterol Nutr (1985) 4,381-7.

Dawson DJ, Lobley RW, Burrows PC, Miller V, Holmes R. "Lactose digestion by human jejunal biopsies: the relationship between hydrolysis and absorption". Gut (1986) 27, 521-7.

ES478590. Process for obtaining 3-methil-lactose which can be used for evaluation of intestinal lactase.

ES482073. Improvements in the main patent Process for obtaining 3-methil-lactose which can be used for evaluation of intestinal lactase.

ES2023556. Process for obtaining 4-O-β-D-galactopyranosyl-D-xylose which can be used for the diagnostic evaluation of intestinal lactase.

ES2100131. Use of beta -D-galactopyranosyl-D-xyloses for the preparation of compositions and solutions intended to the evaluation of intestinal lactase, and production process.

ES2182703. Enzymatic Method of producing 4-0-β-D galactopyranosyl-D-xylose, 4-0-β-D galactopyranosyl-D-Xylose, obtained using said method, compositions containing same and the use thereof in evaluating intestinal lactase.

ES2208099. Use of 4-galactosyl-xylose in humans for the *in vivo* evaluation of intestinal lactase as a non-invasive diagnostic test of the deficiency of said enzyme.

Fan MZ, Stoll B, Jiang R, Burrin DG. "Enterocyte digestive enzyme activity along the crypt-villus and longitudinal axes in the neonatal pig small intestine". J Anim Sci (2001) 79,371-81.

Fleiss JL. "Statistical methods for rates and proportions". 2nd ed. (New York: John Wiley) pp. 38-46.

He Y, Chus S, Walker W. "A Nucleotide supplements alter proliferation and differentiation of cultured human (Caco-2) and rat (IEC-6) intestinal epithelial cells". J Nutr (1993) 123, 1017-17.

Hermida C, Guerra P, Martinez-Costa OH, Sanchez V, Sanchez JJ, Solera J, Fernández-Mayoralas A, Codoceo R, Frias J, Aragón JJ. "Phase I and phase IB clinical trials for the noninvasive evaluation of intestinal lactase with 4-galactosylxylose (gaxilose)". J Clin Gastroenterol (2013) 47, 501-8.

Hermida C, Martinez-Costa OH, Corrales G, Teruel C, Sanchez V, Sanchez JJ, Sarrion D, Ariza MJ, Codoceo R, Calvo I, Fernández-Mayoralas A, Aragón JJ. "Improvement and validation of D-Xylose determination in urine and serum as a new tool for the noninvasive evaluation of lactase activity in humans". J Clin Lab Anal (2014) 28: 478-86.

Hinnebusch BF, Ma Q, Henderson JW, Siddique A, Archer SY, Hodin RA. "Enterocyte response to ischemia is dependent on differentiation state". J Gastrointest Surg (2002) 6, 403-9.

Johnsen U, Schonheit P. "Novel xylose dehydrogenase in the halophilic archaeon Haloarcula marismortui". J Bacteriol (2004) 186, 6198-207.

Johnsen U, Dambeck M, Zaiss H, Fuhrer T, Soppa J, Sauer U, Schonheit P. "D-Xylose degradation pathway in the halophilic archaeon Haloferax volcanii". J Biol Chem (2009) 284, 27290- 303.

Koetse HA, Stellaard F, Bijleveld CM, Elzinga H, Boverhof R, Van der Meer R, Vonk RJ, Sauer PJ. "Non-invasive detection of low intestinal lactase activity in children by use of a combined 13CO2/H2 breath Test. Scand". J Gastroenterol (1999) 34, 35-40.

Landis JR, Koch GG. "The measurement of observer agreement for categorical data". Biometrics (1977) 33;159-74.

Levitt MD. "Production and excretion of hydrogen gas in man" N Engl J Med (1969) 281, 122-7.

Li L, Liang B, Shi J, Li F, Mascini M, Liu A. "A selective and sensitive D-xylose electrochemical biosensor based on xylose dehydrogenase displayed on the surface of bacteria and multi-walled carbon nanotubes modified electrode". Biosens Bioelectron (2012) 33, 100-5.

Li L, Li F, Shi J, Mascini M, Lang Q, Liu A. "Co-immobilization of glucose oxidase and xylose dehydrogenase displayed whole cell on multiwalled carbon nanotube nanocomposite films modified electrode for simultaneous voltammetric detection of d-glucose and D-xylose". Biosens Bioelectron (2013) 42, 156-62.

Liang B, Li L, Mascin M, Liu A. "Construction of xylose dehydrogenase displayed on the surface of bacteria using ice nucleation protein for sensitive D-Xylose detection". Anal Chem (2012) 84;275-82.

Liu M., Valdehuesa KN, Nisola GM, Ramos KR, Chung WJ. "High yield production of D-xylonic acid from D-xylose using engineered Escherichia coli". Bioresour Technol (2012) 115,244-8.

Lifshitz CH, Bautista A, Gapalachrishna GS, Stuff J, Garza C. "Absorption and tolerance of lactose in infants recovering from severe diarrhea". J Pediatr Gastroenterol Nut. (1985) 4, 942-8.

Ma J, Zhong L, Peng X, Sun R. "D-Xylonic acid: a solvent and an effective biocatalyst for a three-component reaction" Green Chem (2016) 18, 1738-50.

McGill DB, Newcomer AD. "Comparison of venous and capillary blood samples in lactose tolerance testing". Gastroenterology (1967) 53, 371-4.

Metz G, Jenkins DJ, Peters TJ, Newman A, Blendis LM. "Breath hydrogen as a diagnostic method for hypolactasia". Lancet (1975) 1, 1155-7.

Mihasan M, Stefan M, Hritcu L, Artenie V, Brandsch R. "Evidence of a plasmid-encoded oxidative xylose-catabolic pathway in Arthrobacter nicotinovorans pAO1". Res Microbiol (2013) 164, 22-30.

Newcomer AD, McGill DB. "Distribution of disaccharidase activity in the small bowel of normal and lactase-deficient subjects". Gastroenterology (1966) 51, 481-8.

Newcomer AD, McGill DB, Thomas PJ, Hofmann AF. "Prospective comparison of indirect methods for detecting lactase deficiency". N Engl J Med (1975) 293, 1232-6.

Nieminen U, Kahri A, Savilahti E, Färkkilä MA. "Duodenal disaccharidase activities in the follow-up of villous atrophy in coeliac disease". Scand J Gastroenterol (2001) 36, 507-10.

Niu W, Molefe MN, Frost JW. "Microbial synthesis of the energetic material precursor 1,2,4-butanetriol". J Am Chem Soc (2003) 125, 12998-9.

Nygård Y, Toivari MH, Penttilä M, Ruohonen L, Wiebe MG. "Bioconversion of D-xylose to D-xylonate with Kluyveromyces lactis". Metab Eng (2011) 13, 383-91.

Nygård Y, Maaheimo H, Mojzita D, Toivari M, Wiebe M, Resnekov O, Gustavo Pesce C, Ruohonen L, Penttila M. "Single cell and in vivo analyses elucidate the effect of xylC lactonase during production of D-xylonate in Saccharomyces cerevisiae". Metab Eng (2014) 25, 238-47.

Parfenov AI, Akhmadullina OV, Sabeln'nikova EA, Belostoskii NI, Gudkova RB, Khomeriki SG. "Carbohydrase activities may serve as a marker for small intestinal mucosal recovery in patients with celiac disease". Ter Arkh (2015) 87, 24-9.

Sasaki Y, Iio M, Kameda H, Ueda H, Aoyagi T. "Measurement of C-lactose absorption in the diagnosis of lactase deficiency" J Lab Clin Med (1970) 76, 824-35.

Sato N, Nakano T, Kawakami H, Idota T. "In vitro and in vivo effects of exogenous nucleotides on the proliferation and maturation of intestinal epithelial cells". J Nutr Sci Vitaminol (Tokyo) (1999) 45, 107-18.

Semenza G, Auricchio S, Mantei N. "The Online Metabolic and Molecular Bases of Inherited Disease". McGraw-Hill (2006) Part 7: Carbohydrates. Chapter 75: Small-Intestinal Disaccharidases pp 1-62.

Semenza G, Auricchio S, Mantei N. "Small-intestinal disaccharidases [Review]. In: Valle D, Beaudet AL, Vogelstein B, Kinzler KW, Antonarakis SE, Ballabio A, Scriver CR, Sly WS, Childs B, eds. Scriver's Online Metabolic and Molecular Bases of Inherited Disease. New York; McGraw-Hill; 2006.

Stephens C, Christen, B, Fuchs T, Sundaram V, Watanabe K, Jenal U. "Genetic analysis of a novel pathway for D-Xylose metabolism in C. crescentus". J Bacteriol (2007) 189, 2181-5.

Toivari MH, Ruohonen L, Richard P, Penttilä M, Wiebe MG. "Saccharomyces cerevisiae engineered to produce D-xylonate" Appl Microbiol Biotechnol (2010) 88, 751-60.

Toivari M, Nygård Y, Kumpula EP, Vehkomäki ML, Benčina M, Valkonen M., Maaheimo H., Andberg M, Koivula A, Ruohonen L, Penttilä M, Wiebe MG. "Metabolic engineering of Saccharomyces cerevisiae for bioconversion of D-xylose to D-xylonate". Metab Eng (2012a) 14, 427-36.

Toivari MH, Nygård Y, Penttilä M, Ruohonen L, Wiebe MG. "Microbial D-xylonate production". Appl Microbiol Biotechnol (2012b) 96, 1-8.

Toivari MH, Vehkomäki ML, Nygård Y, Penttilä M, Ruohonen L, Wiebe MG. "Low pH D-xylonate production with Pichia kudriavzevii". Bioresource Technology (2013) 133, 555-62.

Triadou N, Bataille J, Schmitz J. "Longitudinal study of the human intestinal brush border membrane proteins distribution of the main disaccharidases and peptidases". Gastroenterology (1983) 85, 1326-32.

Uehara K, Takeda M. "L-Xylose dehydrogenase in baker's yeast". J Biochem. (1962) 52, 461-3.

US patent Appln. 10/742,666. Method of decreasing acrylamide in food cooked under heat.

US Patent Application 13/350,097. Non-invasive diagnostic method for the evaluation of intestinal lactase deficiency (hypolactasia).

Valdehuesa KNG, Liu H, Ramos KRM, Park SJ, Nisola W, Lee K, Chung J. "Direct bioconversion of D-xylose to 1, -2, -4-butanetriol in an engineered Escherichia coli". Process Biochem (2014) 49, 25-32.

Xia L, Liang B, Li L, Tang X, Palchetti I, Mascini M, Liu A. "Direct energy conversion from xylose using xylose dehydrogenase surface displayed bacteria based enzymatic biofuel cell". Biosens Bioelectron (2013) 44, 160-3.

Zamora F, Bueno M, Molina I, Iribarren JI, Munoz-Guerra S, Galbis JA. "Macromolecules" (2000) 33, 2030-38.

## Claims

1. Method for *in vitro* quantification of D-xylose in a biological fluid consisting of adding to said biological fluid, as the only enzyme, the enzyme D-xylose dehydrogenase (XylB), wherein said D-xylose is detected by XylB and wherein XylB comprises the sequence SEQ ID NO: 5 and retained an activity comparable with the initial activity after 270 days stored freeze-dried at 4°C.

2. Method according to claim 1 wherein XylB consists of sequence SEQ ID NO: 5.

3. Method according to any of the claims 1 or 2, wherein the biological fluid is urine or blood from a mammal.

**4.** Method according to claim 3, wherein the biological fluid is from human origin.

**5.** Kit for quantification of D-xylose in a biological sample which comprises the enzyme D-xylose dehydrogenase (XylB) as the only enzyme in the kit, $NAD^+$ and a suitable buffer, wherein XylB comprises the sequence SEQ ID NO: 5 and retained an activity comparable with the initial activity after 270 days stored freeze-dried at 4°C.

**6.** Kit according to claim 5, wherein the suitable buffer is phosphate buffer.

**7.** Kit according to claims 5 or 6, wherein XylB consists of sequence SEQ ID NO: 5.

**8.** *In vitro* diagnostic method for non-invasive evaluation of intestinal lactase deficiency comprising the following steps:

(i) collecting total urine excreted by a subject between the time of an oral intake of a dose of 4-O-beta-D-galactopyranosil-D-xylose (Gaxilose) by the subject and 4 or 5 hours following said time, wherein said dose is between 0.1125 g and 5.4 g,
(ii) mixing said total urine with the enzyme D-xylose dehydrogenase (XylB) comprising the sequence SEQ ID NO: 5, detecting the total amount of D-xylose by said enzyme XylB comprising the sequence SEQ ID NO: 5, thereby determining the total amount of D-xylose in said total excreted urine, and
(iii) comparing the value obtained in step (ii) with a threshold reference value obtained from total urine samples from a population of healthy control individuals, wherein said total urine samples are subjected to the same protocol as the total urine sample from the subject, and wherein the subject is considered to be suffering from intestinal lactase deficiency (hypolactasia), when the value of excreted D-xylose obtained is below the threshold reference value.

**9.** *In vitro* diagnostic method according to claim 8, wherein the enzyme XylB comprising the sequence SEQ ID NO: 5 is the only enzyme used in the method.

**10.** *In vitro* diagnostic method according to claim 9, wherein XylB consists of sequence SEQ ID NO: 5.

**11.** *In vitro* diagnostic method according to any of the claims 8 to 10, wherein the doses of Gaxilose are selected from the group consisting of 0.1125g, 0.225 g, 0.45 g, 0.9 g, 2.7 g and 5.4 g.

**12.** *In vitro* diagnostic method according to any of the claims 8 to 10, wherein the reference threshold value of the total amount of D-xylose in urine is selected from the group consisting of 3.95 mg in 4-hour urine and 5.06 mg in 5-hour urine, when the dose of Gaxilose is 0.1125 g; 11.74 mg in 4-hour urine and 12.55 mg in 5-hour urine, when the dose of Gaxilose is 0.225 g; 15.59 mg in 4-hour urine and 19.18 mg in 5-hour urine, when the dose of Gaxilose is 0.45 g; 20.77 mg in 4-hour urine and 27.98 mg in 5-hour urine, when the dose of Gaxilose is 0.9 g; 31.28 mg in 4-hour urine and 50.48 mg in 5-hour urine, when the dose of Gaxilose is 2.7 g; and 68.38 mg in 4-hour urine and 87.40 mg in 5-hour urine, when the dose of Gaxilose is 5.4 g.

**13.** *In vitro* diagnostic method according to any of the claims 8 to 12, wherein the degree of difference of the D-xylose reference threshold value and the value of excreted D-xylose determines the degree of intestinal lactase deficiency (hypolactasia) of the subject.

**14.** Use of the method of claims 1 to 4 and/or of the kit of claims 5 to 7, in the *in vitro* diagnosis of hypolactasia.

**15.** Use of the method of claims 1 to 4 and/or of the kit of claims 5 to 7, in the *in vitro* determination of intestinal mucosa impairment.

**Patentansprüche**

**1.** Verfahren für eine *in vitro*-Quantifizierung der D-Xylose in einer biologischen Flüssigkeit, bestehend aus dem Hinzufügen der genannten biologischen Flüssigkeit, als einziges Enzym, das Enzym D-Xylose-Dehydrogenase (XylB), wobei die genannte D-Xylose von XylB erkannt wird und wobei XylB die Sequenz SEQ ID NO: 5 umfasst und eine Aktivität vergleichbar der anfänglichen Aktivität nach 270 Tagen gefriergetrockneter Lagerung bei 4° C zurückgehalten wird.

2. Verfahren nach Anspruch 1, wobei XylB aus der Sequenz SEQ ID NO: 5 besteht.

3. Verfahren nach einem der Ansprüche 1 oder 2, wobei die biologische Flüssigkeit Urin oder Blut von einem Säugetier ist.

4. Verfahren nach Anspruch 3, wobei die biologische Flüssigkeit menschlichen Ursprungs ist.

5. Set zur Quantifizierung von D-Xylose in einer biologischen Probe, welche das Enzym D-Xylose-Dehydrogenase (XylB) als einziges Enzym in dem Set umfasst, NAD+ und einen geeigneten Puffer, wobei XylB die Sequenz SEQ ID NO: 5 umfasst und eine Aktivität vergleichbar der anfänglichen Aktivität nach 270 Tagen gefriergetrockneter Lagerung bei 4° C zurückgehalten wird.

6. Set nach Anspruch 5, wobei der geeignete Puffer ein Phoshatpuffer ist.

7. Set nach Anspruch 5 oder 6, wobei XylB aus der Sequenz SEQ ID NO: 5 besteht.

8. In-vitro-Diagnose-Verfahren für die nicht-invasive Evaluierung der intestinalen Laktoseintoleranz, bestehend aus den folgenden Schritten:

(i) Aufnahme einer gesamten Urinprobe, die von einer Versuchsperson in der Zeit zwischen der oralen Einnahme einer Dosis von 4-O-beta-D-Galactopyranosil-D-Xylose (Gaxilose) von der Versuchsperson und 4 oder 5 Stunden nach dem genannten Zeitpunkt ausgeschieden wird, wobei die besagte Dosis zwischen 0,1125 g und 5,4 g liegt, wobei
(ii) die gesamte Urinprobe mit dem Enzym D-Xylose-Dehydrogenase (XylB) vermischt wird, welche die Sequenz SEQ ID NO: 5 umfasst, wobei die Gesamtmenge von D-Xylose von dem genannten Enzym XylB, welches die Sequenz SEQ ID NO: 5 umfasst, festgestellt wird, und wodurch die Gesamtmenge der D-Xylose in der Gesamtmenge des ausgeschiedenen Urins bestimmt wird, und
(iii) beim Vergleich des Wertes, der bei Schritt (ii) erlangt wird, mit einem Schwellenreferenzwert, den man von der Gesamtheit aller Urinproben einer Population mit gesunden Kontrollpersonen erhält, wird die genannte Gesamtheit aller Urinproben dem gleichen Protokoll unterworfen wie die gesamte Urinprobe der Versuchsperson, und geschlussfolgert wird, dass die Versuchsperson an der der intestinalen Laktoseintoleranz (Hypolaktasie) leidet, wenn der erhaltende Wert der ausgeschiedenen D-Xylose unter dem Schwellenreferenzwert liegt.

9. In-vitro-Diagnose-Verfahren nach Anspruch 8, wobei das Enzym XylB, welches die SEQ ID NO: 5 umfasst, das einzige Enzym ist, das bei dem Verfahren verwendet wird.

10. In-vitro-Diagnose-Verfahren nach Anspruch 9, wobei XylB aus der Sequenz SEQ ID NO: 5 besteht.

11. In-vitro-Diagnose-Verfahren nach einem der Ansprüche 8 bis 10, wobei die Dosen von Gaxilose aus der Gruppe ausgewählt werden, bestehend aus 0,1125 g, 0,225 g, 0,45 g, 0,9 g, 2,7 g und 5,4 g.

12. In-vitro-Diagnose-Verfahren nach einem der Ansprüche 8 bis 10, wobei der Referenzschwellenwert der Gesamtmenge von D-Xylose im Urin ausgewählt wird aus der Gruppe, bestehend aus 3,95 mg beim 4-Stunden-Urin und 5,06 mg beim 5-Stunden-Urin, wobei die Dosis von Gaxilose 0,1125 g beträgt; 11,74 mg beim 4-Stunden-Urin und 12,55 mg beim 5-Stunden-Urin, wenn die Dosis von Gaxilose 0,225 g beträgt; 15,59 mg beim 4-Stunden-Urin und 19,18 mg beim 5-Stunden-Urin, wenn die Dosis von Gaxilose 0,45 g beträgt; 20,77 mg beim 4-Stunden-Urin und 27,98 mg beim 5-Stunden-Urin, wenn die Dosis von Gaxilose 0,9 g beträgt; 31,28 mg beim 4-Stunden-Urin und 50,48 mg beim 5-Stunden-Urin, wenn die Dosis von Gaxilose 2,7 g beträgt; und 68,38 mg beim 4-Stunden-Urin und 87,40 mg beim 5-Stunden-Urin, wenn die Dosis von Gaxilose 5,4 g beträgt.

13. In-vitro-Diagnose-Verfahren nach einem der Ansprüche 8 bis 12, wobei der Grad des Unterschieds zwischen dem D-Xylose-Referenzschwellenwert und dem Wert der ausgeschiedenen D-Xylose den Grad der intestinalen Laktoseintoleranz (Hypolaktasie) der Versuchsperson bestimmt.

14. Anwendung des Verfahrens der Ansprüche 1 bis 4 und/oder des Sets der Ansprüche 5 bis 7 bei der In-vitro-Diagnose von Hypolaktasie.

15. Anwendung des Verfahrens der Ansprüche 1 bis 4 und/oder des Sets der Ansprüche 5 bis 7 bei der *in vitro*-Bestimmung einer Darmschleimhautschädigung.

**Revendications**

1. Méthode pour la quantification *in vitro* du D-xylose dans un liquide biologique consistant à ajouter audit liquide biologique, en tant qu'unique enzyme, l'enzyme D-xylose déshydrogénase (XylB), dans lequel ledit D-xylose est détecté par XylB et dans lequel XylB comprend la séquence SEQ ID NO: 5 et a conservé une activité comparable à l'activité initiale après avoir été stockée pendant 270 jours sous la forme lyophilisée à 4 °C.

2. Méthode selon la revendication 1, dans lequel XylB consiste en la séquence SEQ ID NO: 5.

3. Méthode selon l'une des revendications 1 ou 2, dans lequel le liquide biologique est de l'urine ou du sang d'un mammifère.

4. Méthode selon la revendication 3, dans lequel le liquide biologique est d'origine humaine.

5. Kit pour la quantification du D-xylose dans un échantillon biologique qui comprend l'enzyme D-xylose déshydrogénase (XylB) en tant qu'unique enzyme dans le kit, NAD+ et un tampon adapté, dans lequel XylB comprend la séquence SEQ ID NO: 5 et a conservé une activité comparable à l'activité initiale après avoir été stockée pendant 270 jours sous la forme lyophilisée à 4 °C.

6. Kit selon la revendication 5, dans lequel le tampon adapté est un tampon phosphate.

7. Kit selon les revendications 5 ou 6, dans lequel XylB comprend la séquence SEQ ID NO: 5.

8. Méthode de diagnostic *in vitro* pour l'évaluation non invasive du déficit en lactase intestinale comprenant les étapes suivantes :

   (i) recueil de la totalité de l'urine excrétée par un sujet durant la période comprise entre le moment d'une absorption par voie orale d'une dose de 4-O-bêta-D-galactopyranosil-D-xylose (Gaxilose) par le sujet et les 4 ou 5 heures suivant ledit moment, dans laquelle ladite dose est comprise entre 0,1125 g et 5,4 g,
   (ii) mélange de ladite totalité de l'urine avec l'enzyme D-xylose déshydrogénase (XylB) comprenant la séquence SEQ ID NO: 5, détection de la quantité totale de D-xylose par ladite enzyme XylB comprenant la séquence SEQ ID NO: 5, détermination par ce moyen de la quantité totale de D-xylose dans ladite totalité d'urine excrétée, et
   (iii) comparaison de la valeur obtenue dans l'étape (ii) avec une valeur seuil de référence obtenue à partir d'échantillons de totalité d'urine provenant d'une population d'individus témoins en bonne santé, dans laquelle lesdits échantillons de totalité d'urine sont soumis au même protocole que l'échantillon de totalité d'urine du sujet, et dans laquelle le sujet est considéré comme souffrant d'un déficit en lactase intestinale (hypolactasie), lorsque la valeur de D-xylose excrétée obtenue est inférieure à la valeur seuil de référence.

9. Méthode de diagnostic *in vitro* selon la revendication 8, dans laquelle l'enzyme XylB comprenant la séquence SEQ ID NO: 5 est l'unique enzyme utilisée dans la méthode.

10. Méthode de diagnostic *in vitro* selon la revendication 9, dans laquelle XylB comprend la séquence SEQ ID NO: 5.

11. Méthode de diagnostic *in vitro* selon l'une des revendications 8 à 10, dans laquelle les doses de Gaxilose sont choisies dans le groupe comprenant 0,1125 g, 0,225 g, 0,45 g, 0,9 g, 2,7 g et 5,4 g.

12. Méthode de diagnostic *in vitro* selon l'une des revendications 8 à 10, dans laquelle la valeur seuil de référence de la quantité totale de D-xylose dans l'urine est choisie dans le groupe comprenant 3,95 mg dans l'urine de 4 heures et 5,06 mg dans l'urine de 5 heures, lorsque la dose de Gaxilose est de 0,1125 g ; 11,74 mg dans l'urine de 4 heures et 12,55 mg dans l'urine de 5 heures, lorsque la dose de Gaxilose est de 0,225 g ; 15,59 mg dans l'urine de 4 heures et 19,18 mg dans l'urine de 5 heures, lorsque la dose de Gaxilose est de 0,45 g ; 20,77 mg dans l'urine de 4 heures et 27,98 mg dans l'urine de 5 heures, lorsque la dose de Gaxilose est de 0,9 g ; 31,28 mg dans l'urine de 4 heures et 50,48 mg dans l'urine de 5 heures, lorsque la dose de Gaxilose est de 2,7 g ; et 68,38 mg dans l'urine de 4 heures et 87,40 mg dans l'urine de 5 heures, lorsque la dose de Gaxilose est de 5,4 g.

**13.** Méthode de diagnostic *in vitro* selon l'une des revendications 8 à 12, dans laquelle le degré de différence de la valeur seuil de référence de D-xylose et la valeur de D-xylose excrété détermine le degré de déficit en lactase intestinale (hypolactasie) du sujet.

**14.** Utilisation de la méthode des revendications 1 à 4 et/ou du kit des revendications 5 à 7, dans le diagnostic *in vitro* de l'hypolactasie.

**15.** Utilisation de la méthode des revendications 1 à 4 et/ou du kit des revendications 5 à 7, dans la détermination *in vitro* d'une déficience de la muqueuse intestinale.

**Figure 1**

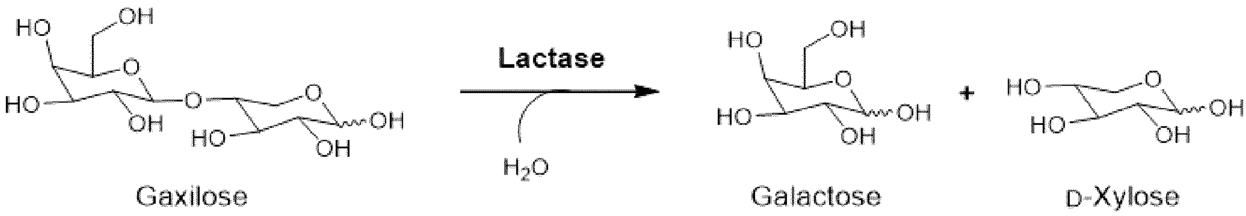


Gaxilose Galactose D-Xylose

**Figure 2**

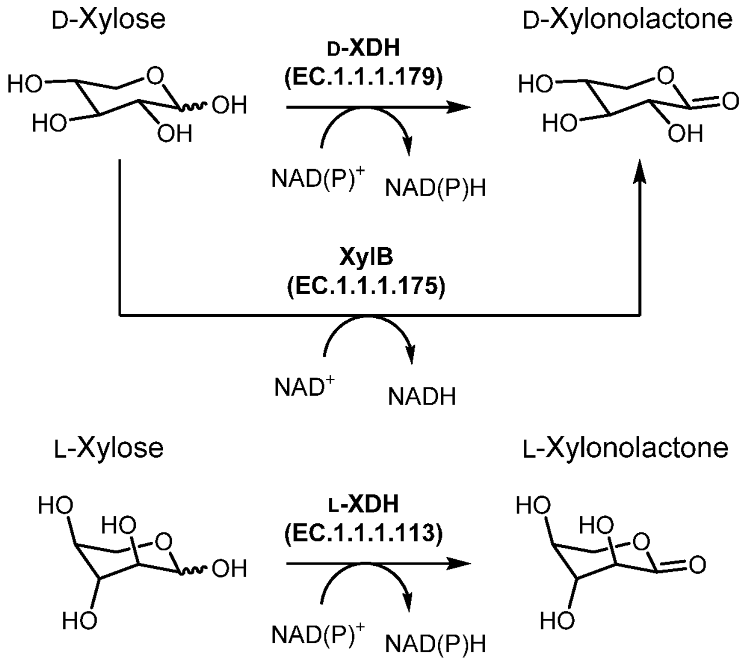

**Figure 3**

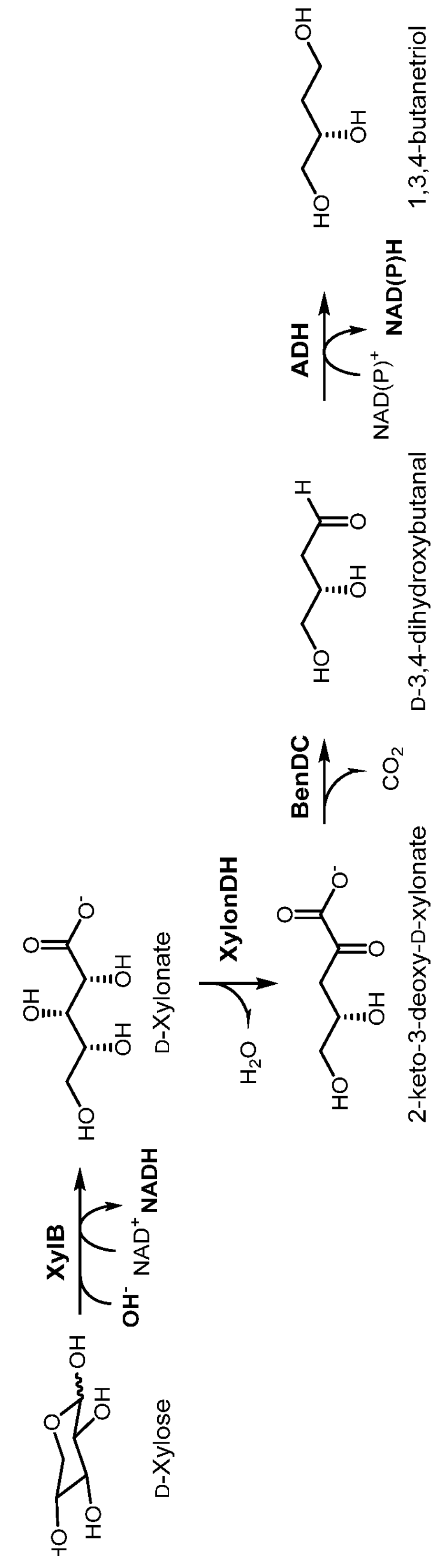
D-Xylose
XylB
OH-
NAD+
NADH
D-Xylonate
XylonDH
H2O
2-keto-3-deoxy-D-xylonate
BenDC
CO2
D-3,4-dihydroxybutanal
ADH
NAD(P)+
NAD(P)H
1,3,4-butanetriol

Figure 4

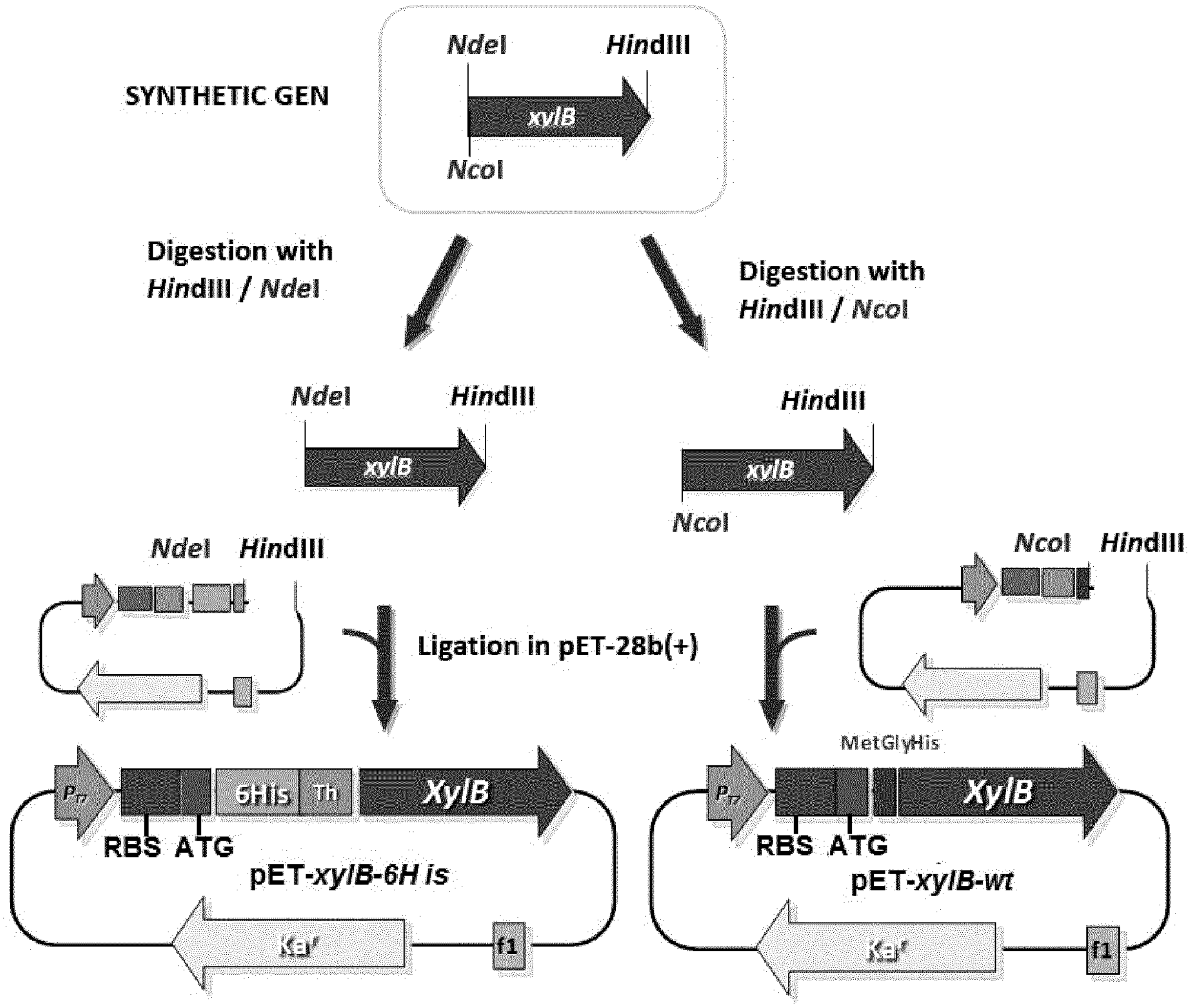
SYNTHETIC GEN
NdeI
HindIII
xylB
NcoI
Digestion with
HindIII / NdeI
Digestion with
HindIII / NcoI
NdeI
HindIII
xylB
HindIII
xylB
NcoI
NdeI
HindIII
Ligation in pET-28b(+)
NcoI
HindIII
MetGlyHis
6His
Th
XylB
RBS
ATG
pET-xylB-6H is
Kar
f1
XylB
RBS
ATG
pET-xylB-wt
Kar
f1

Figure 5

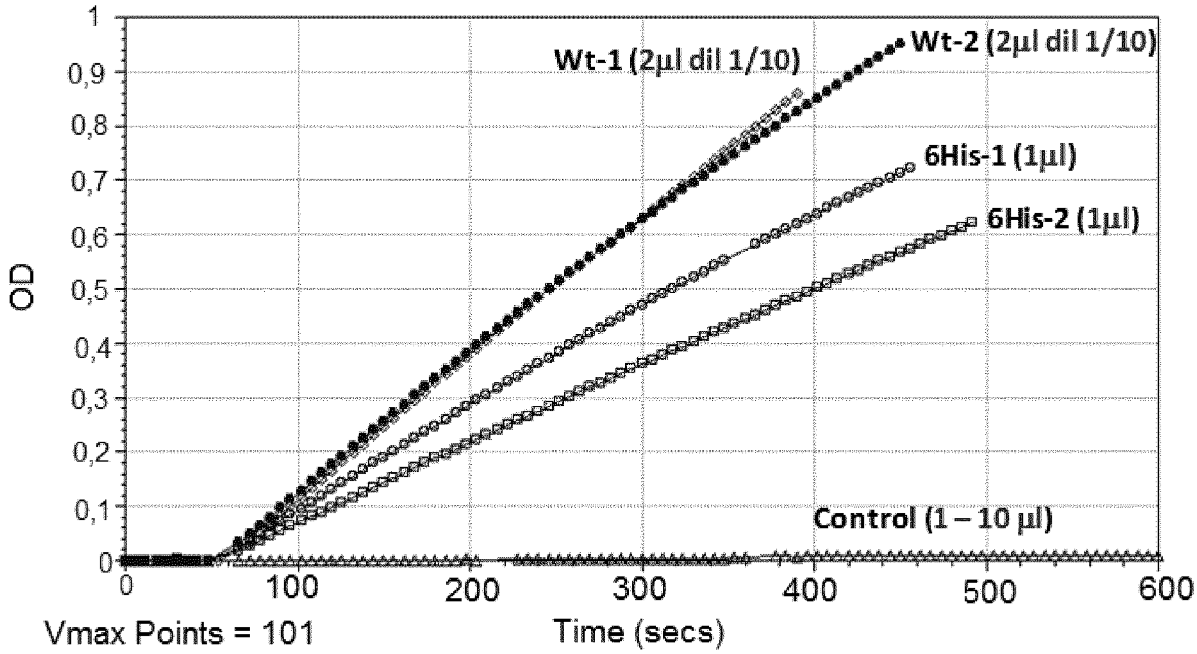


Figure 6

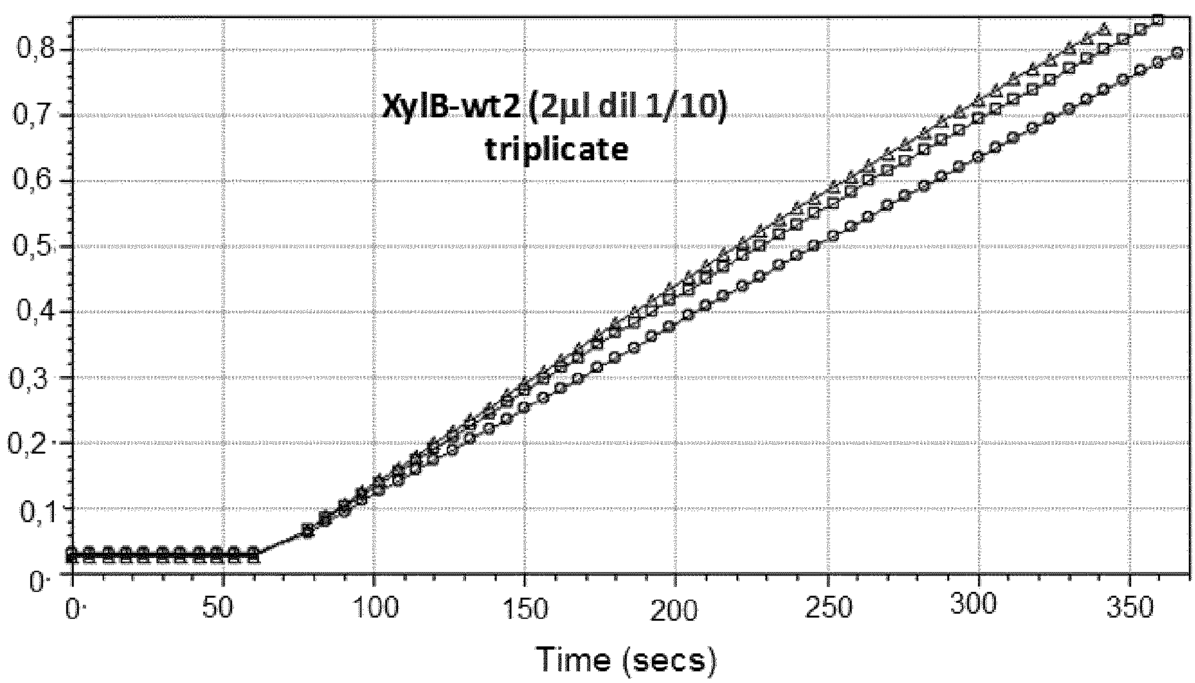

Figure 7

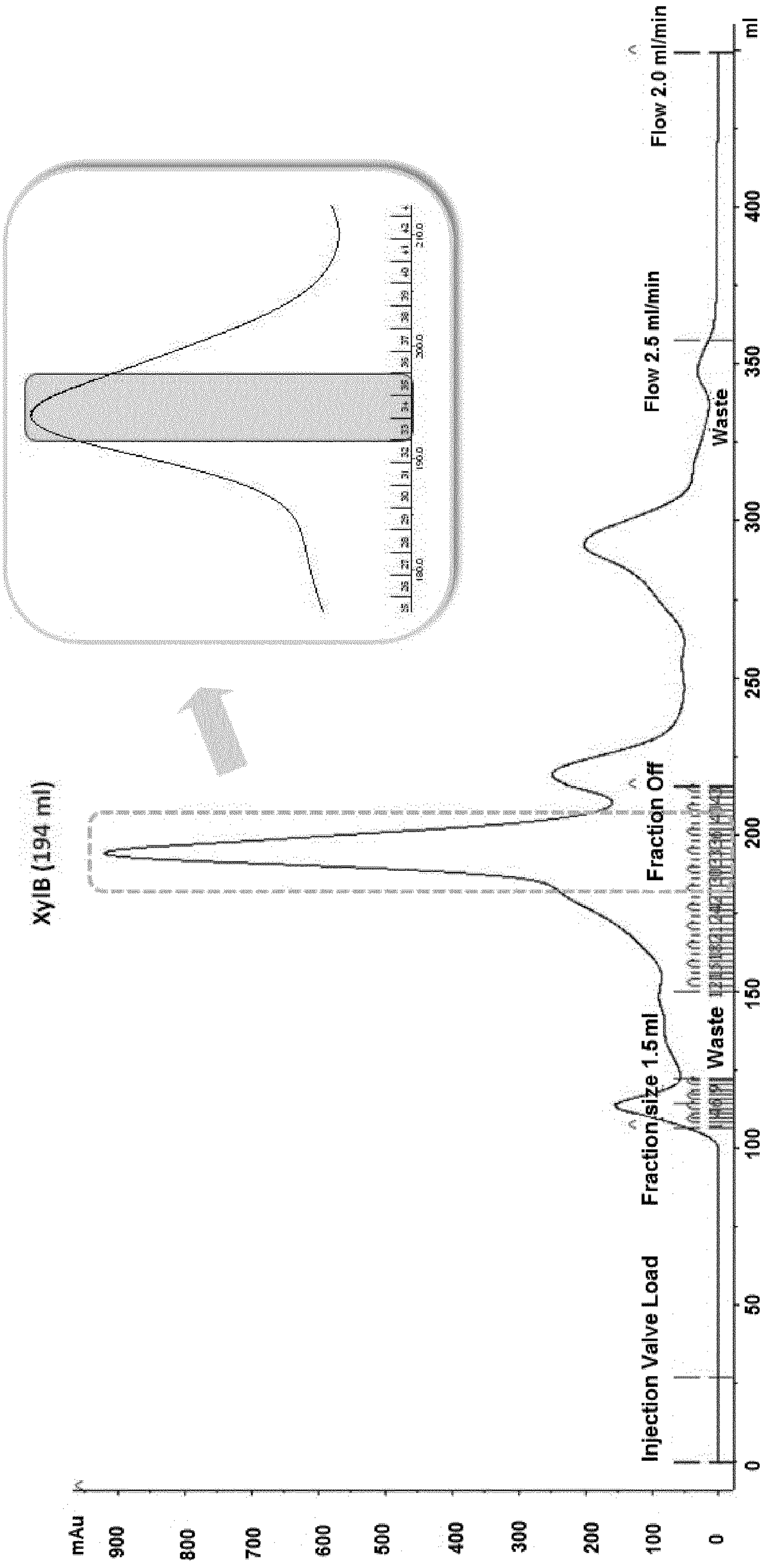
XylB (194 ml)
Injection Valve Load
Fraction size 1.5 ml
Waste
Fraction Off
Flow 2.5 ml/min
Waste
Flow 2.0 ml/min
mAu
ml

Figure 8

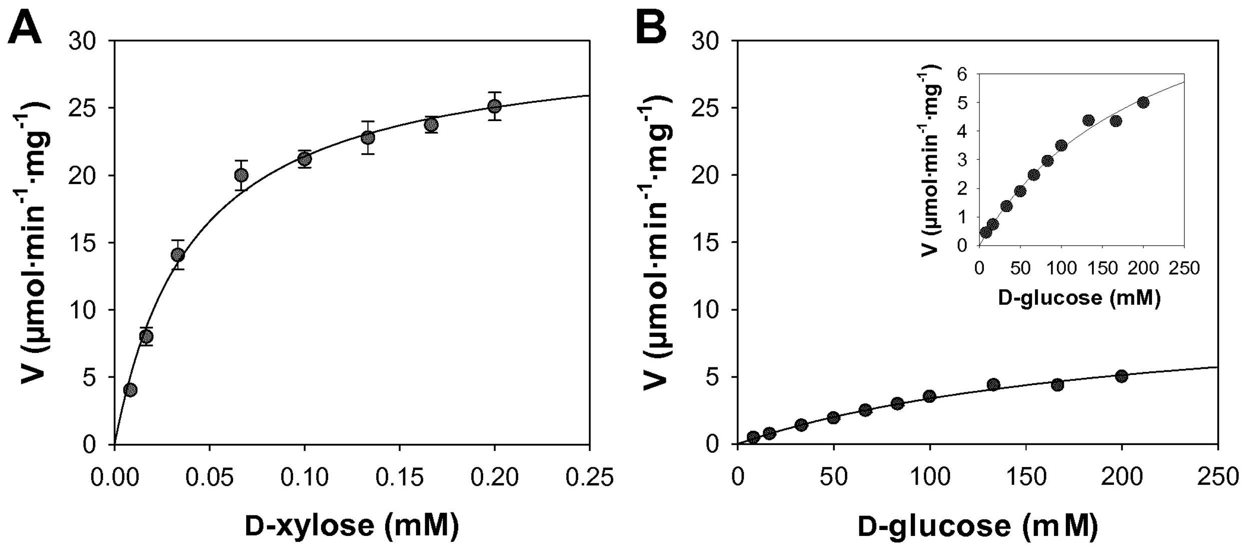


Figure 9

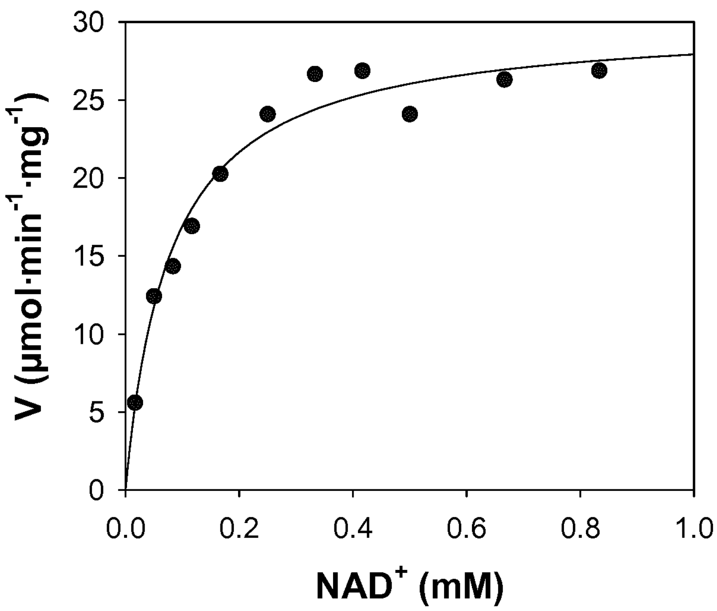

Figure 10

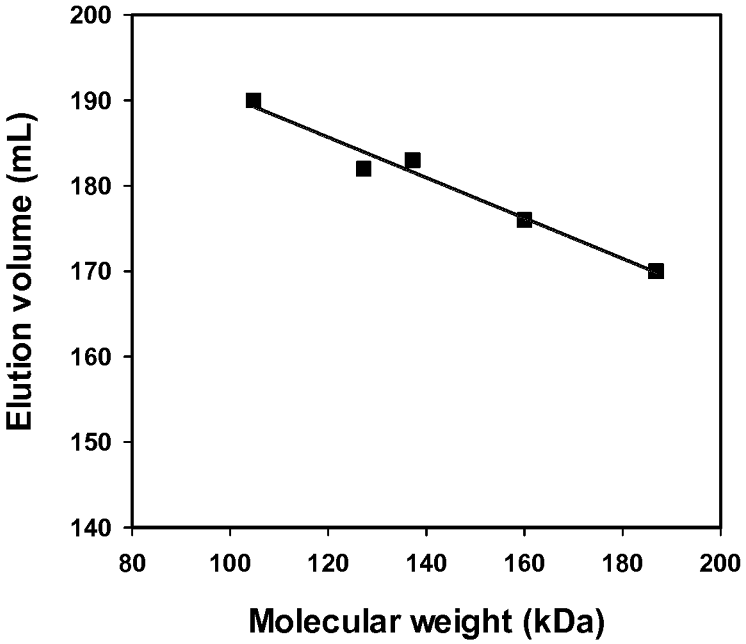


Figure 11

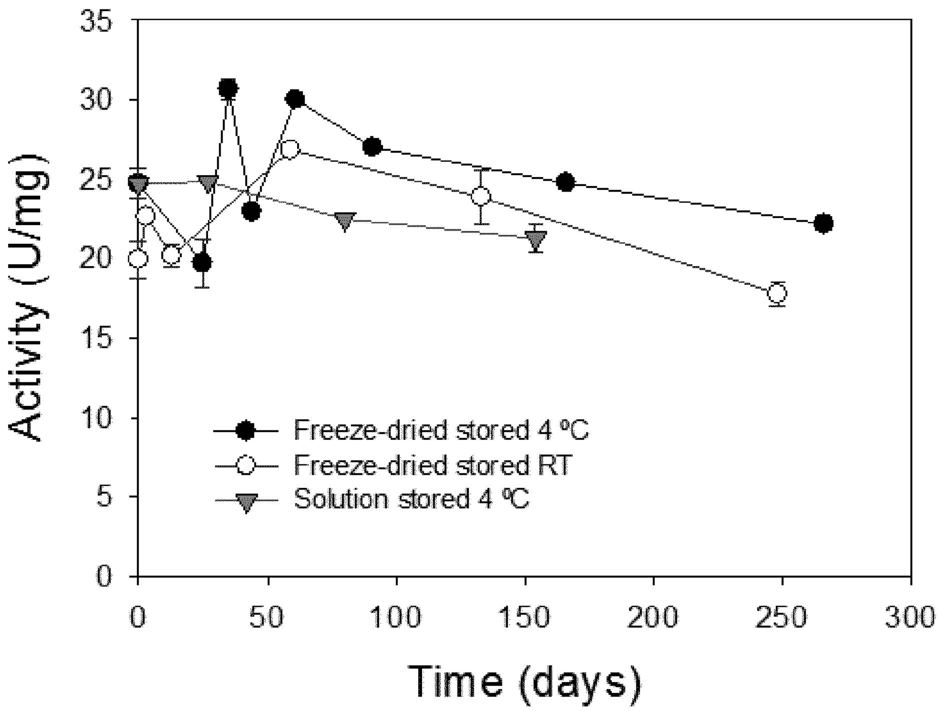

Figure 12

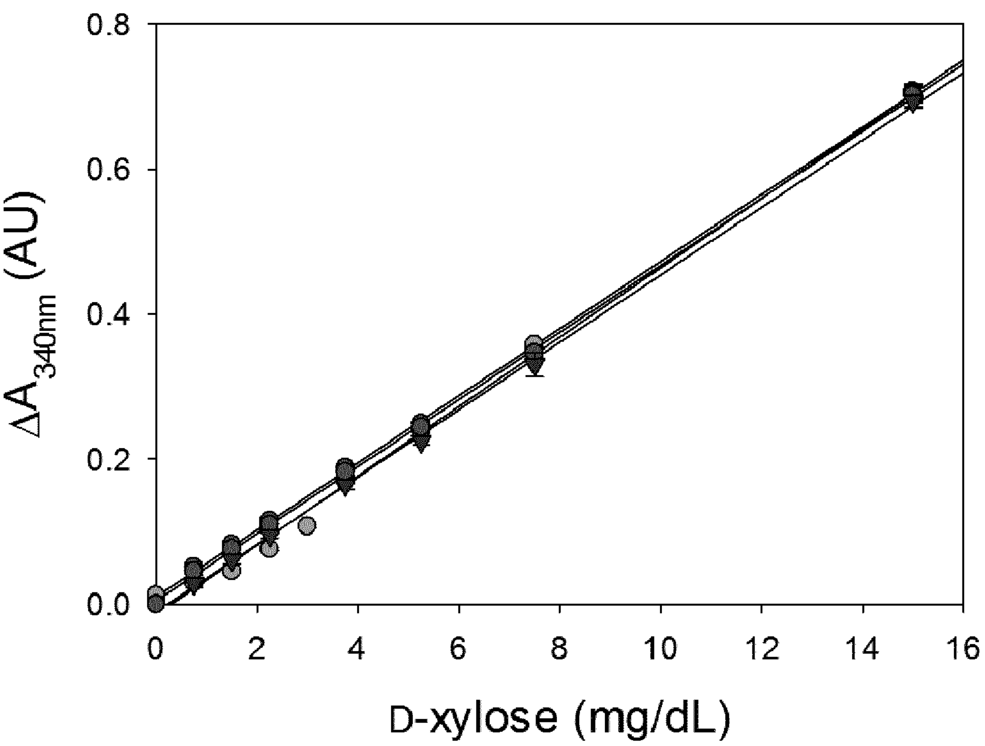


Figure 13

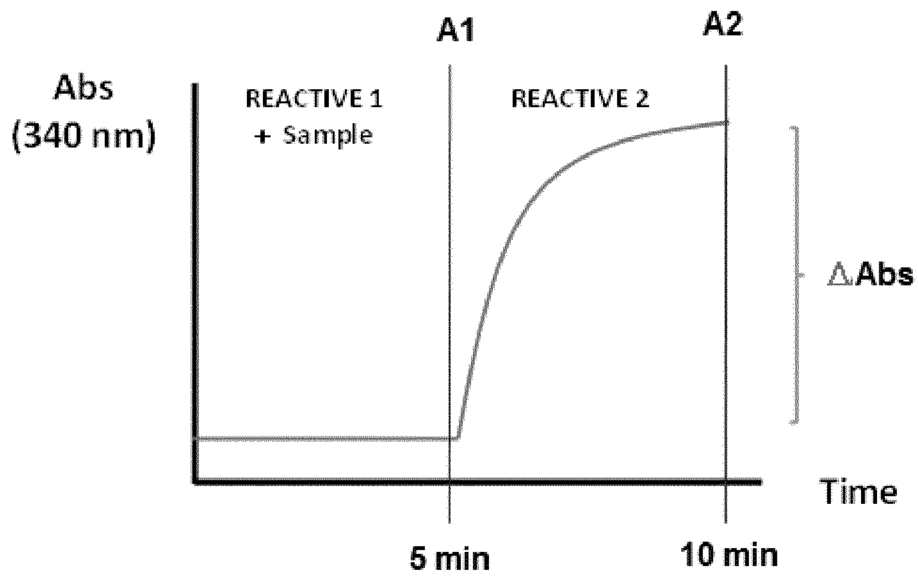

**Figure 14**

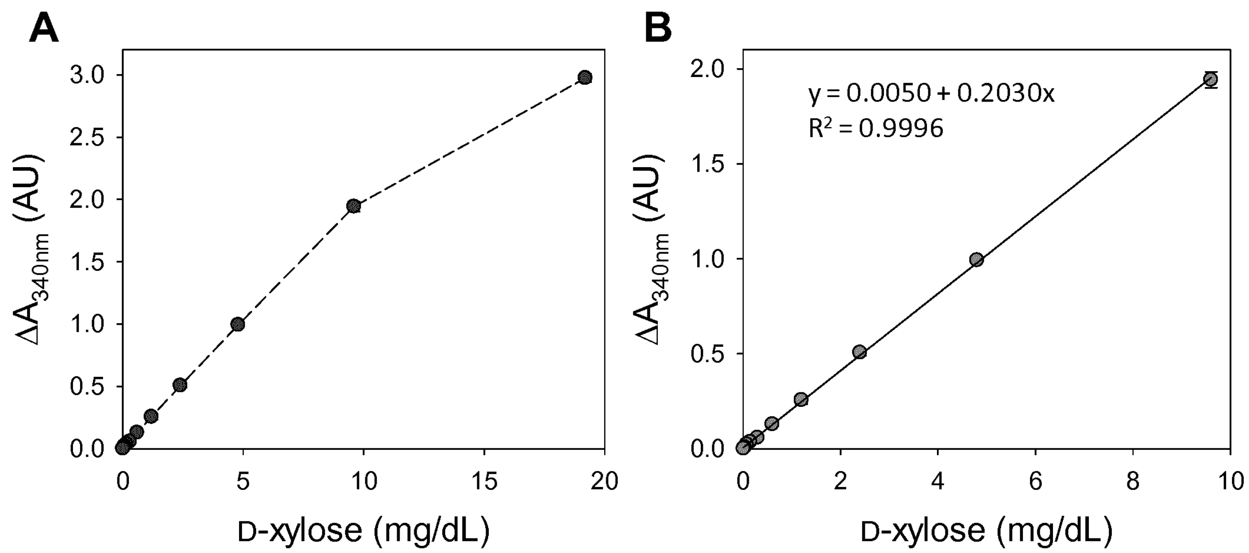


**Figure 15**

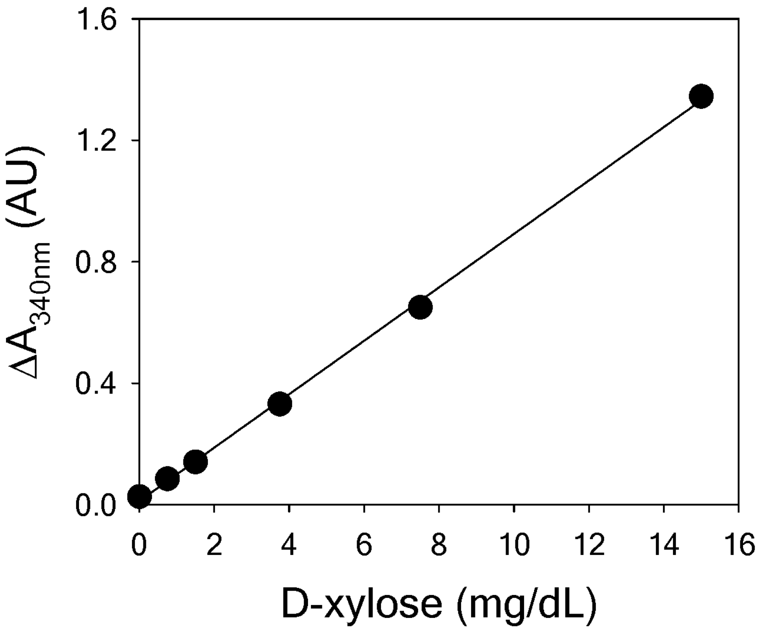

**Figure 16**

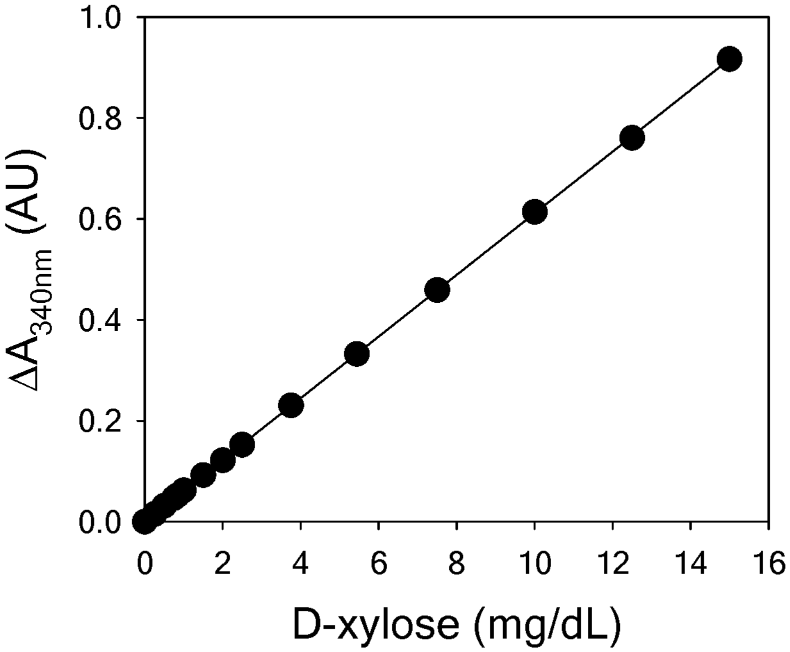


**Figure 17**

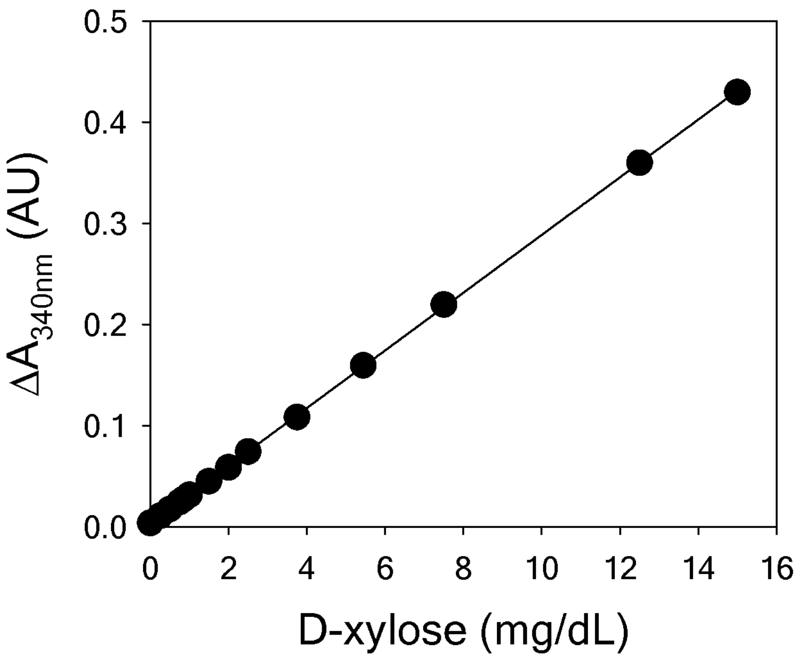

Figure 18

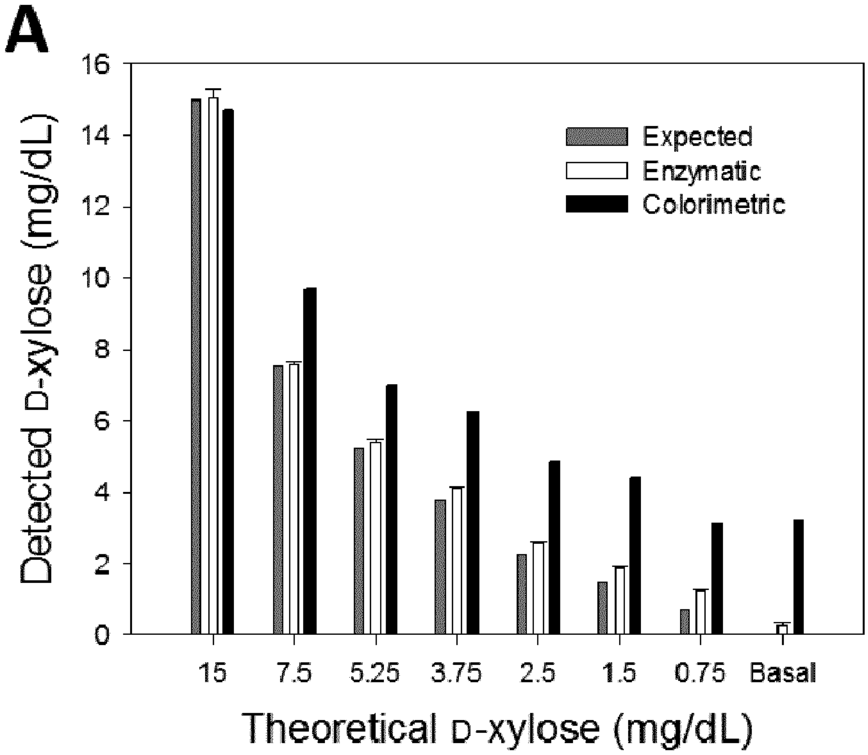
A
Detected D-xylose (mg/dL)
16
14
12
10
8
6
4
2
0
Expected
Enzymatic
Colorimetric
15
7.5
5.25
3.75
2.5
1.5
0.75
Basal
Theoretical D-xylose (mg/dL)

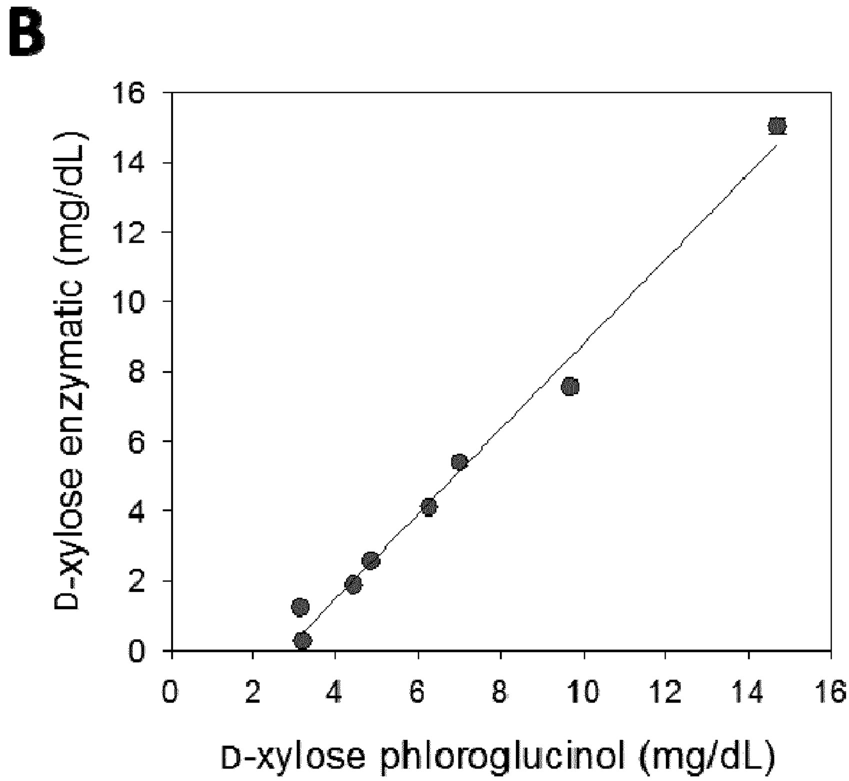
B
D-xylose enzymatic (mg/dL)
16
14
12
10
8
6
4
2
0
0
2
4
6
8
10
12
14
16
D-xylose phloroglucinol (mg/dL)

Figure 18 (Cont.)

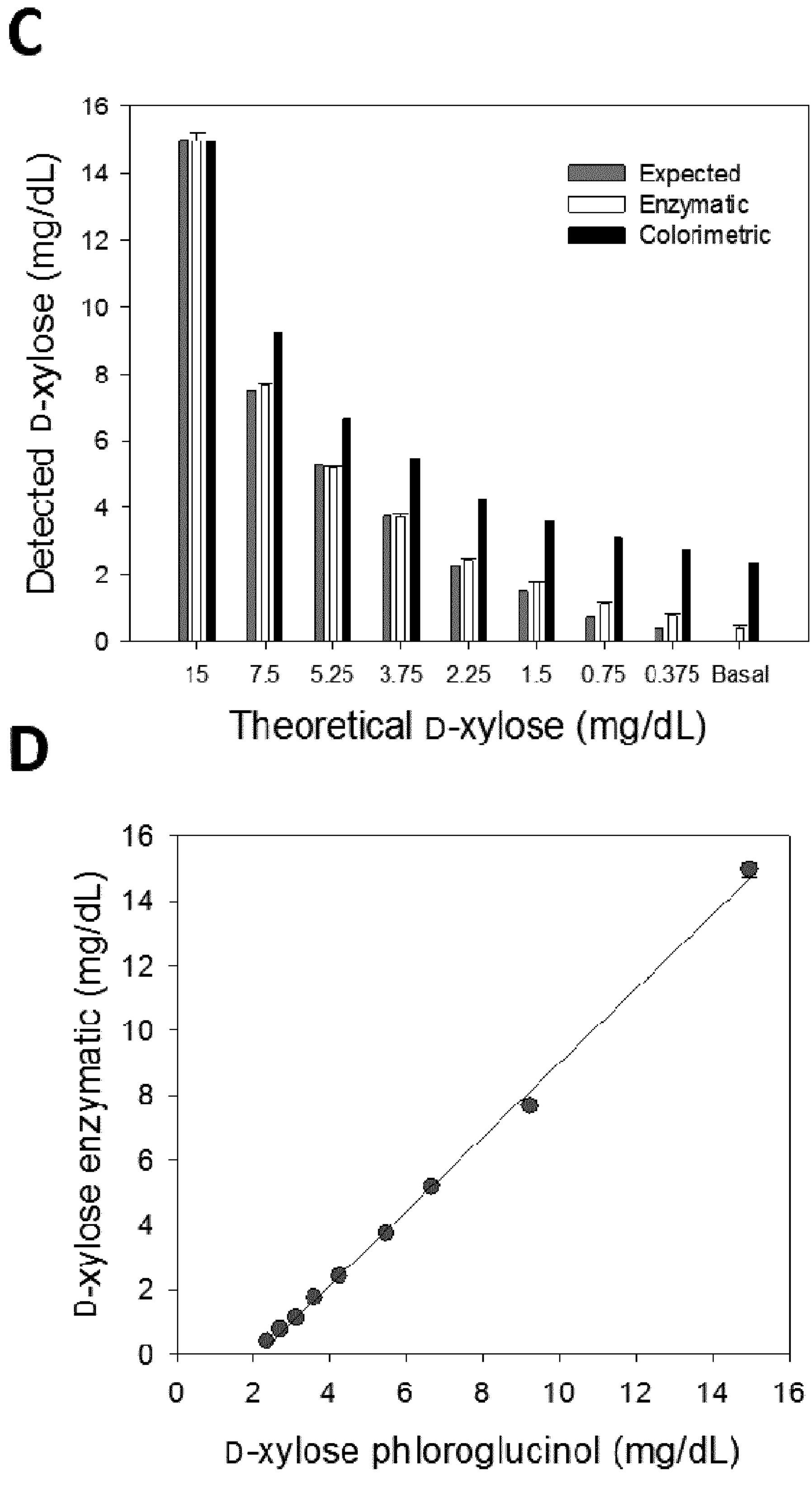

Figure 19

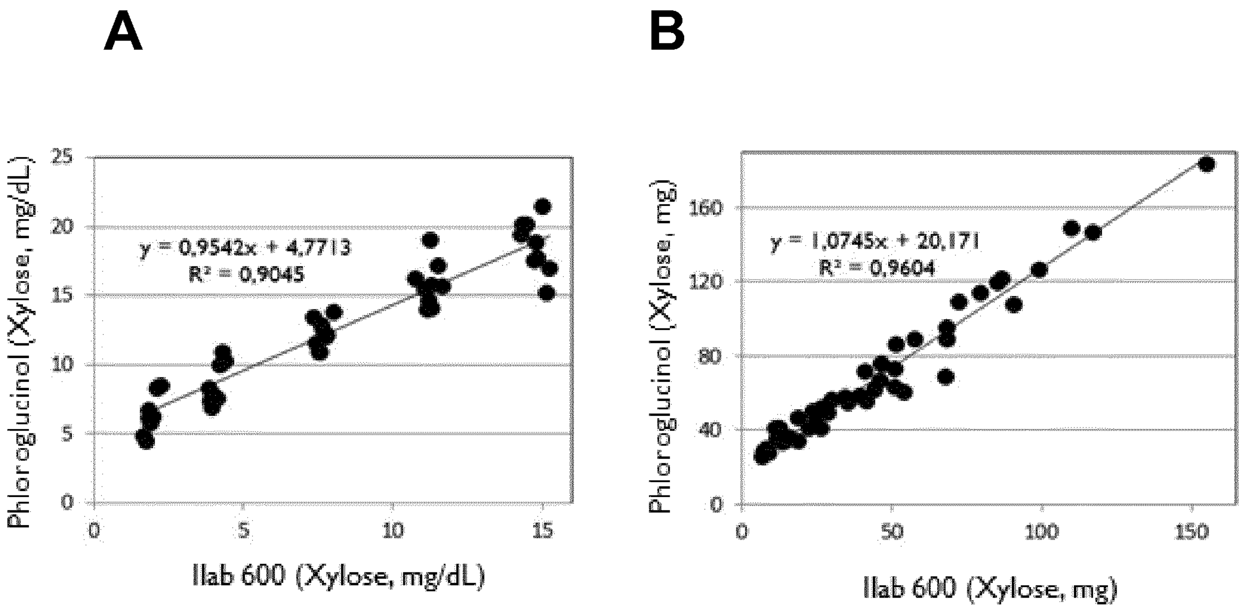


Figure 20

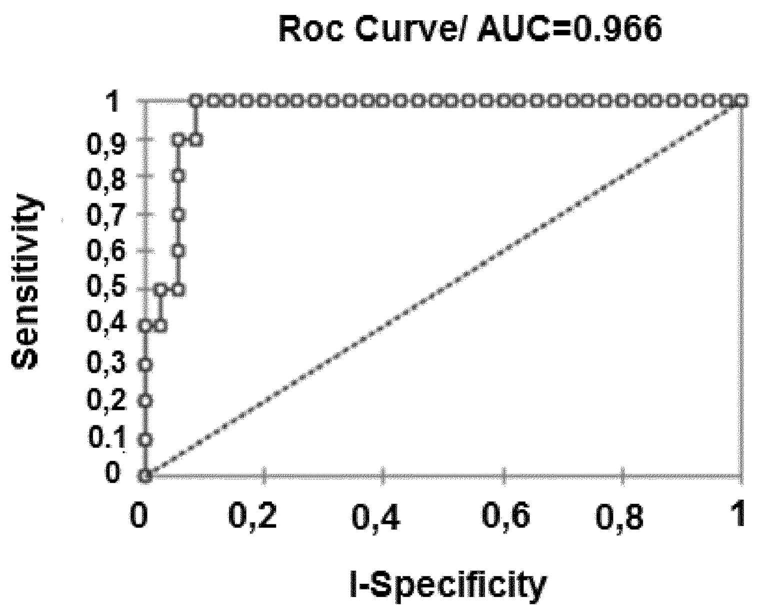

Figure 21

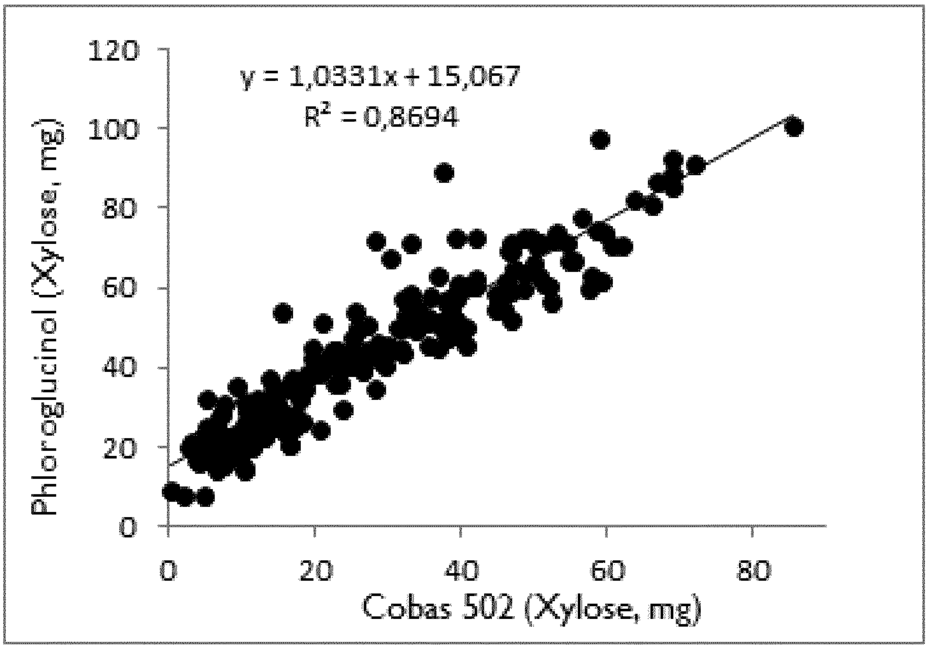


Figure 22

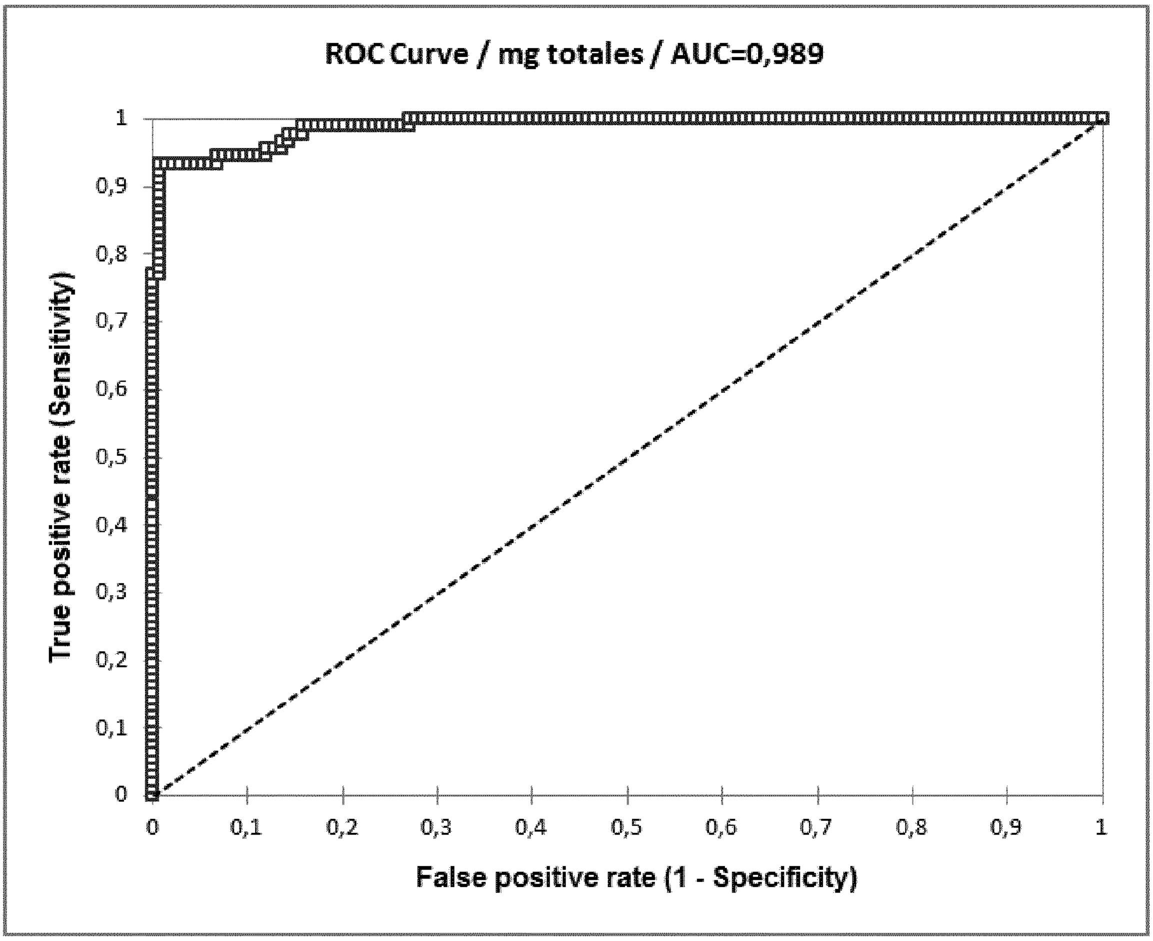

Figure 23

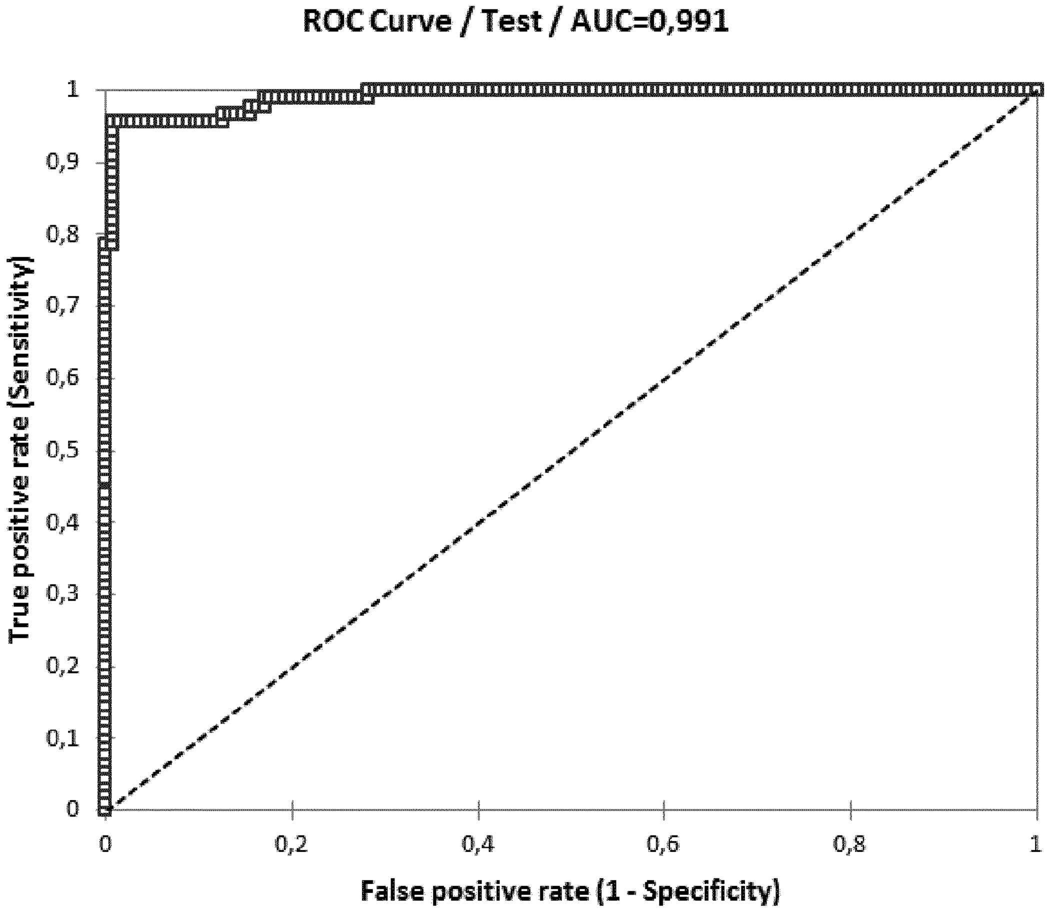


Figure 24

| | | ENZYMATIC + | ENZYMATIC - |
|---|---|---|---|
| PHLOROGLUCINOL | + | 85 | 4 |
| | - | 1 | 134 |

Kappa: 0.9531 (I.C. 95%: 0.9125 - 0.9937)

Figure 25

A

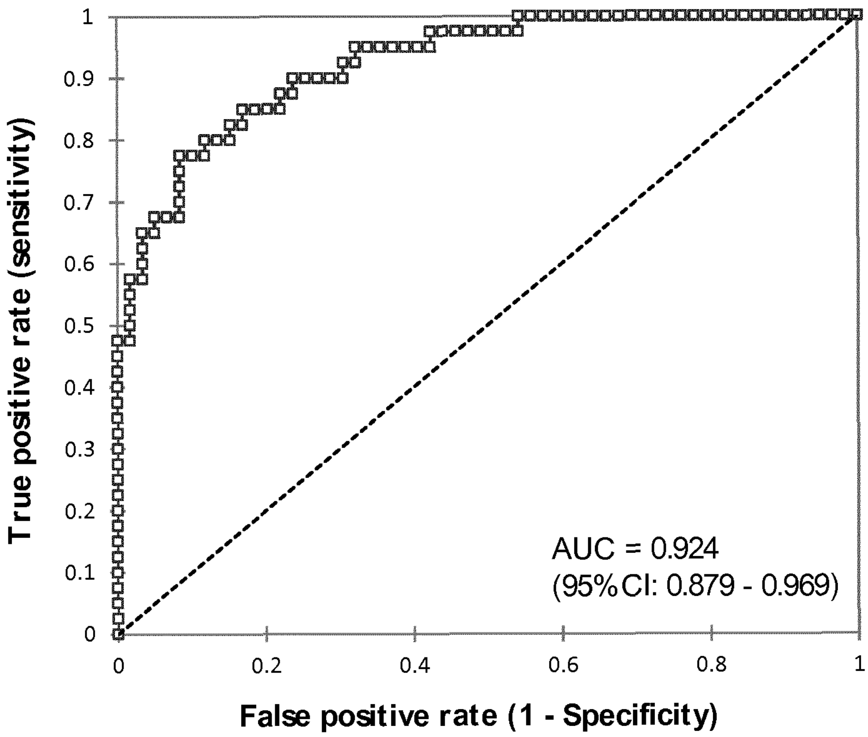
1
0.9
0.8
0.7
0.6
0.5
0.4
0.3
0.2
0.1
0
0
0.2
0.4
0.6
0.8
1
True positive rate (sensitivity)
False positive rate (1 - Specificity)
AUC = 0.924
(95% CI: 0.879 - 0.969)

B

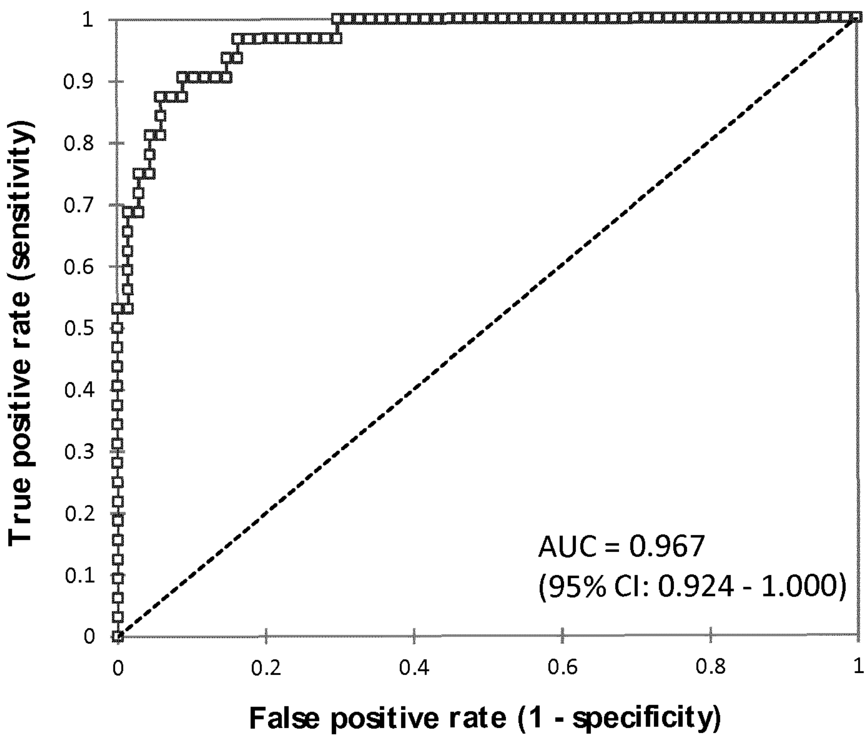
1
0.9
0.8
0.7
0.6
0.5
0.4
0.3
0.2
0.1
0
0
0.2
0.4
0.6
0.8
1
True positive rate (sensitivity)
False positive rate (1 - specificity)
AUC = 0.967
(95% CI: 0.924 - 1.000)

Figure 26

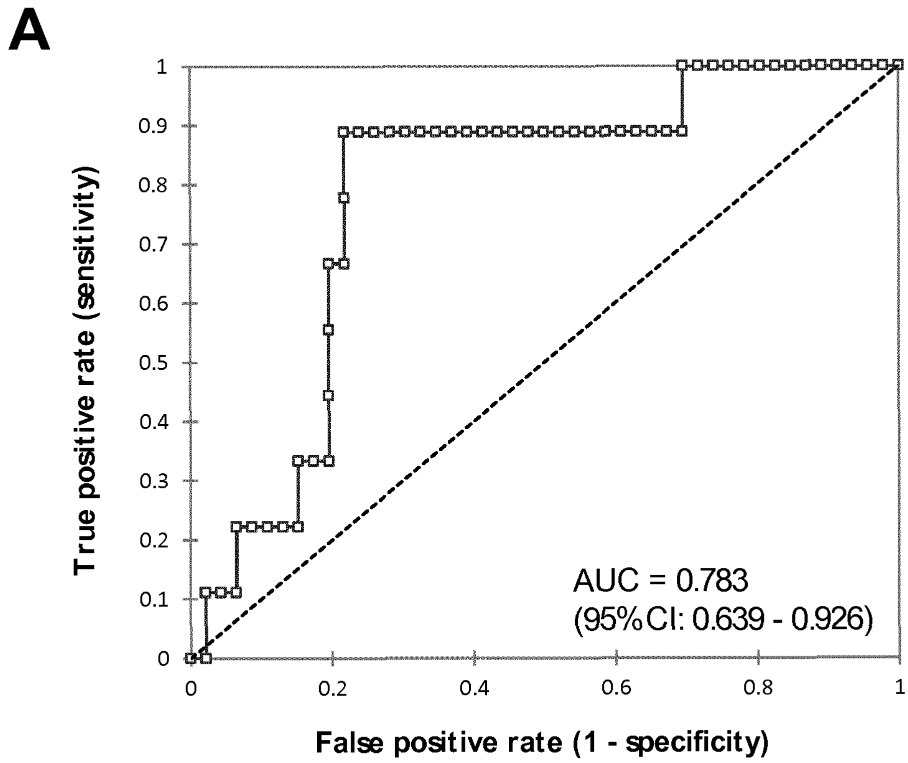
A
1
0.9
0.8
0.7
0.6
0.5
0.4
0.3
0.2
0.1
0
True positive rate (sensitivity)
0
0.2
0.4
0.6
0.8
1
False positive rate (1 - specificity)
AUC = 0.783
(95%CI: 0.639 - 0.926)

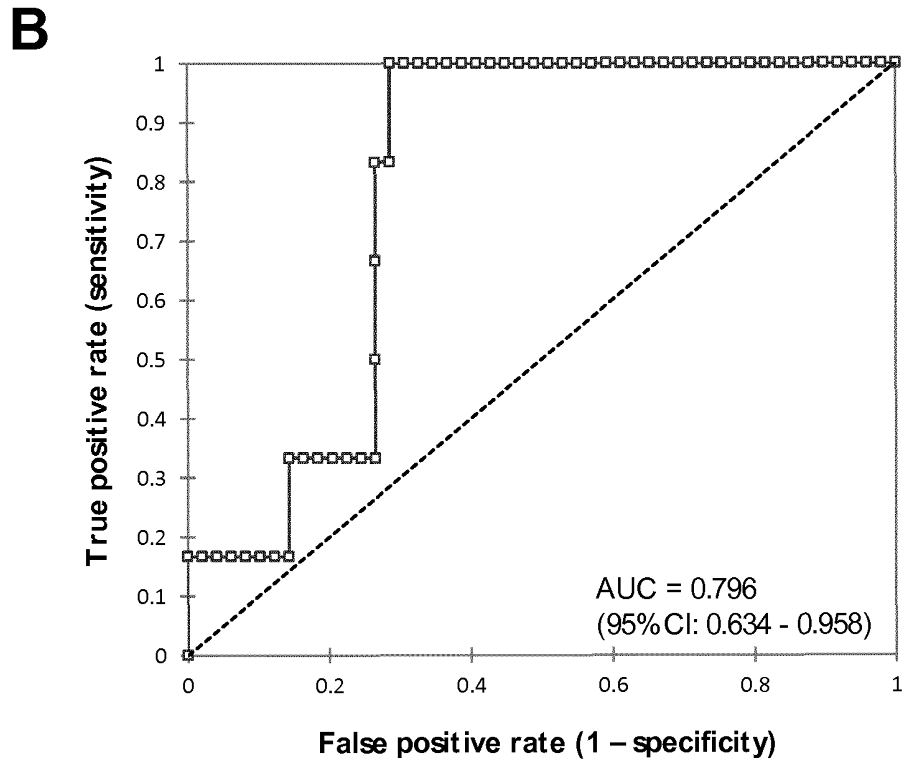
B
1
0.9
0.8
0.7
0.6
0.5
0.4
0.3
0.2
0.1
0
True positive rate (sensitivity)
0
0.2
0.4
0.6
0.8
1
False positive rate (1 – specificity)
AUC = 0.796
(95%CI: 0.634 - 0.958)

Figure 27

A

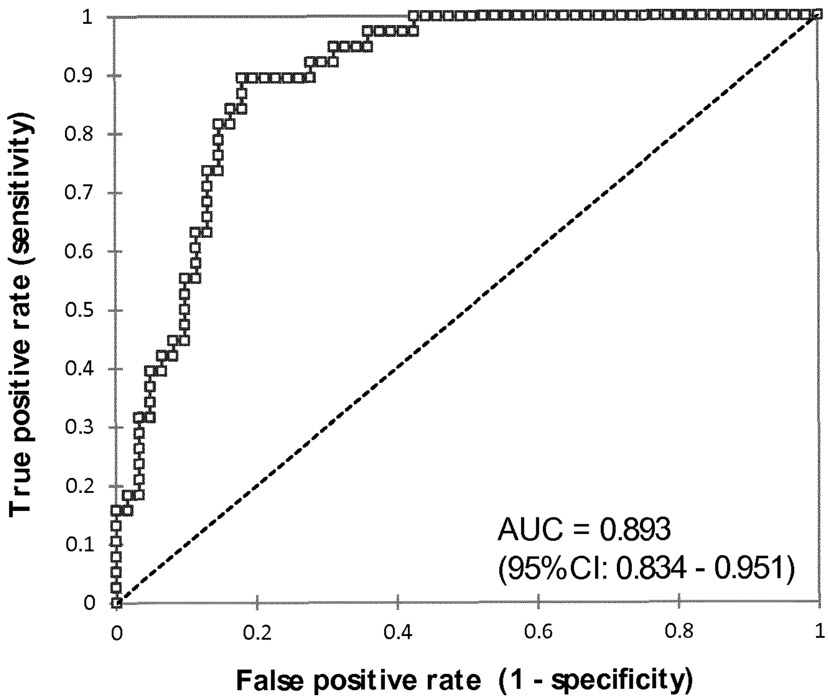
True positive rate (sensitivity)
False positive rate (1 - specificity)
AUC = 0.893
(95%CI: 0.834 - 0.951)

B

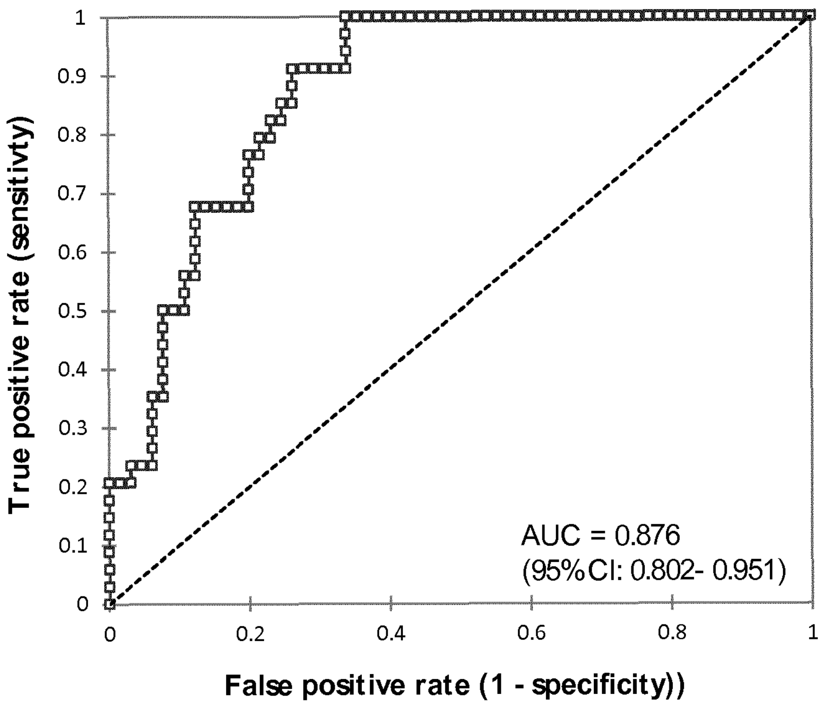
True positive rate (sensitivty)
False positive rate (1 - specificity))
AUC = 0.876
(95%CI: 0.802- 0.951)

Figure 28

A

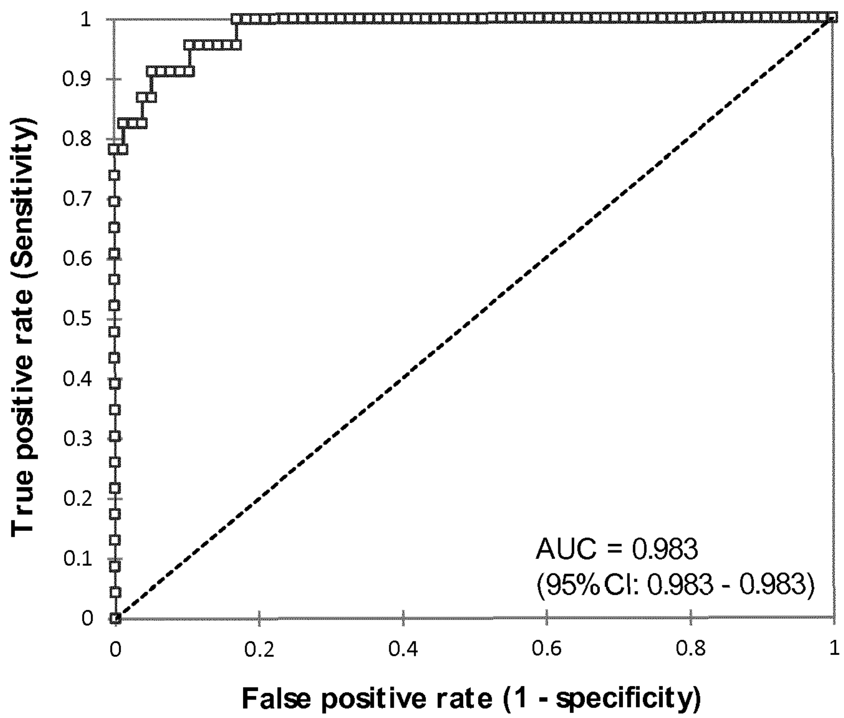
True positive rate (Sensitivity)
False positive rate (1 - specificity)
AUC = 0.983
(95% CI: 0.983 - 0.983)
0
0.1
0.2
0.3
0.4
0.5
0.6
0.7
0.8
0.9
1
0
0.2
0.4
0.6
0.8
1

B

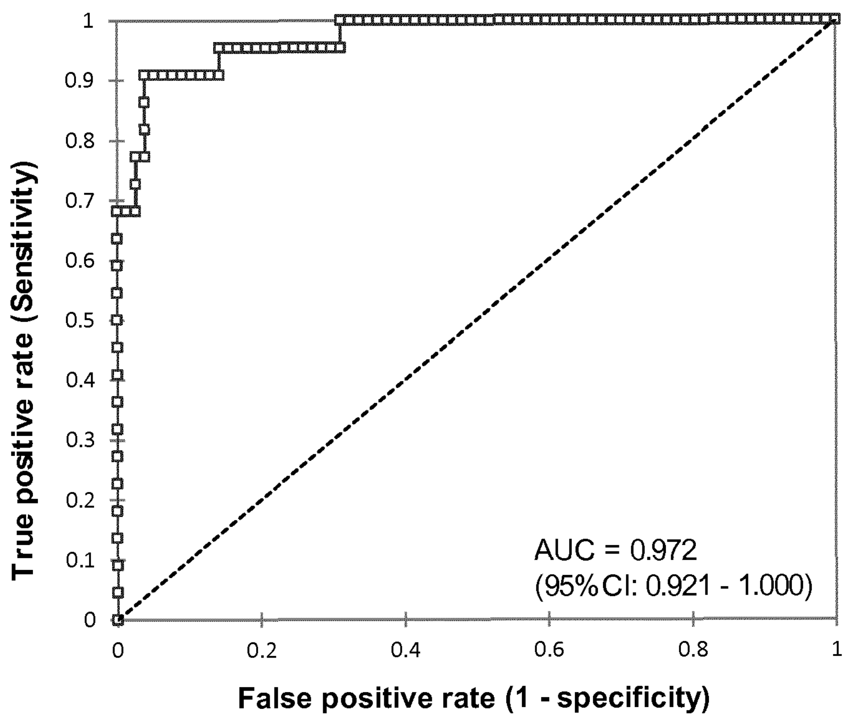
True positive rate (Sensitivity)
False positive rate (1 - specificity)
AUC = 0.972
(95% CI: 0.921 - 1.000)
0
0.1
0.2
0.3
0.4
0.5
0.6
0.7
0.8
0.9
1
0
0.2
0.4
0.6
0.8
1

Figure 29

A

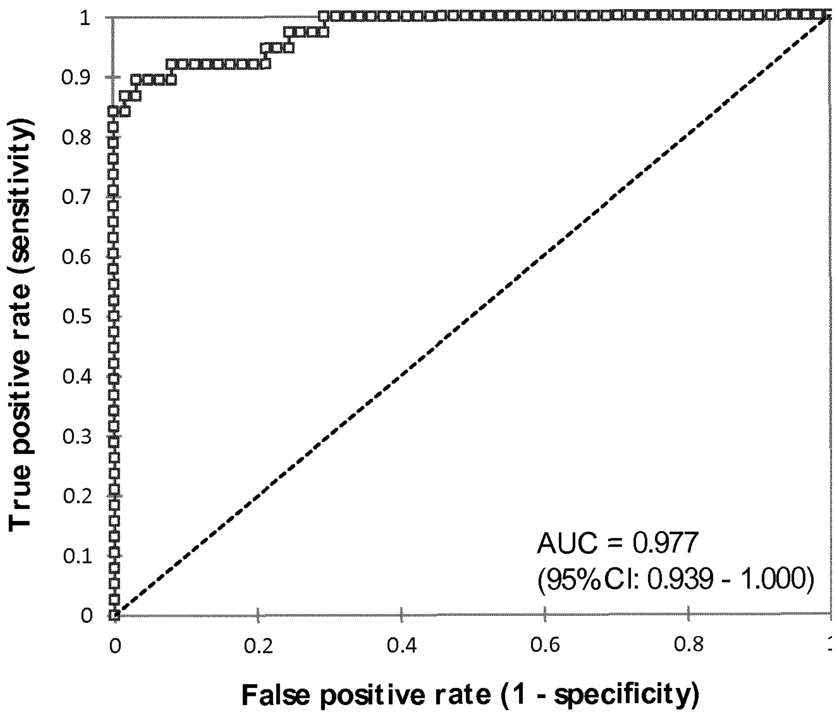
True positive rate (sensitivity)
False positive rate (1 - specificity)
AUC = 0.977
(95%CI: 0.939 - 1.000)
0
0.1
0.2
0.3
0.4
0.5
0.6
0.7
0.8
0.9
1
0
0.2
0.4
0.6
0.8

B

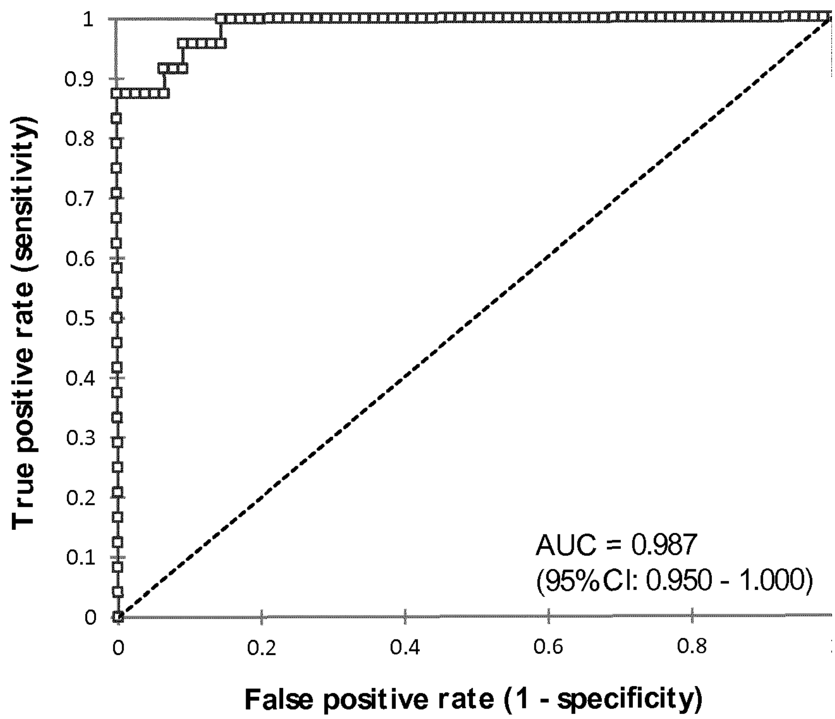
True positive rate (sensitivity)
False positive rate (1 - specificity)
AUC = 0.987
(95%CI: 0.950 - 1.000)
0
0.1
0.2
0.3
0.4
0.5
0.6
0.7
0.8
0.9
1
0
0.2
0.4
0.6
0.8

Figure 30

A

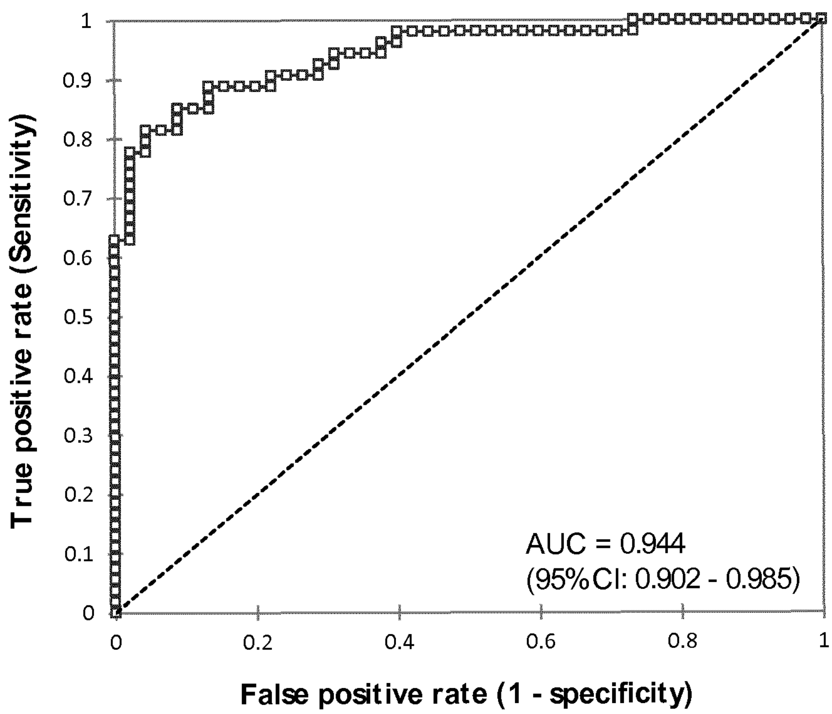
1
0.9
0.8
0.7
0.6
0.5
0.4
0.3
0.2
0.1
0
True positive rate (Sensitivity)
AUC = 0.944
(95% CI: 0.902 - 0.985)
0
0.2
0.4
0.6
0.8
1
False positive rate (1 - specificity)

B

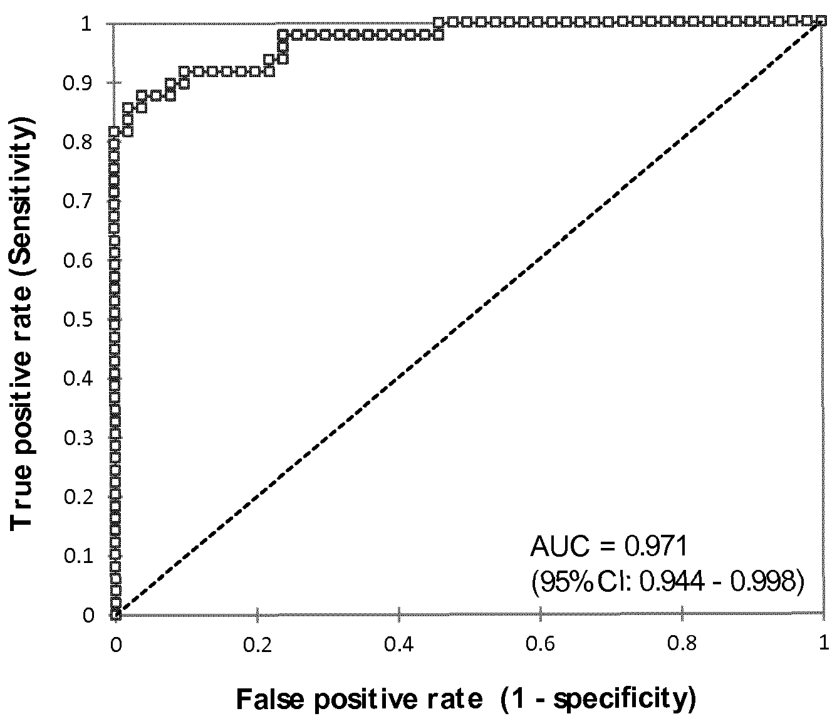
1
0.9
0.8
0.7
0.6
0.5
0.4
0.3
0.2
0.1
0
True positive rate (Sensitivity)
AUC = 0.971
(95% CI: 0.944 - 0.998)
0
0.2
0.4
0.6
0.8
1
False positive rate (1 - specificity)

Figure 31

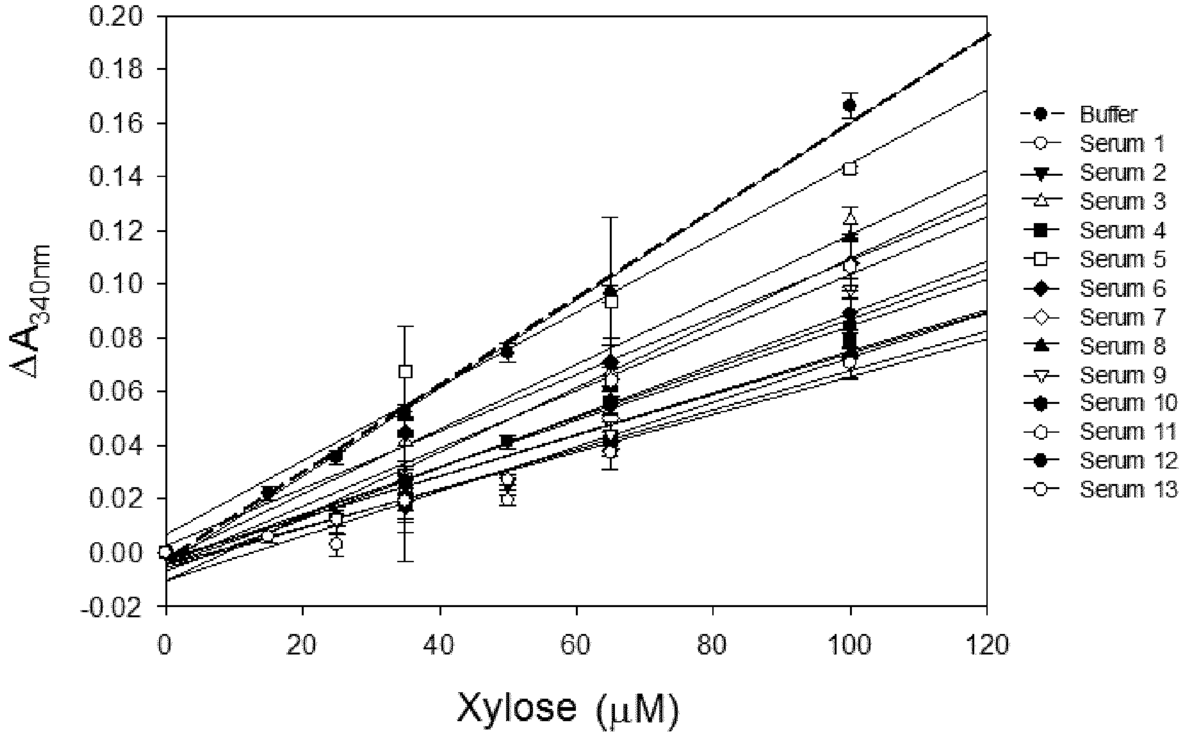
0.20
0.18
0.16
0.14
0.12
0.10
0.08
0.06
0.04
0.02
0.00
-0.02
$\Delta A_{340nm}$
0
20
40
60
80
100
120
Xylose (μM)
Buffer
Serum 1
Serum 2
Serum 3
Serum 4
Serum 5
Serum 6
Serum 7
Serum 8
Serum 9
Serum 10
Serum 11
Serum 12
Serum 13

Figure 32

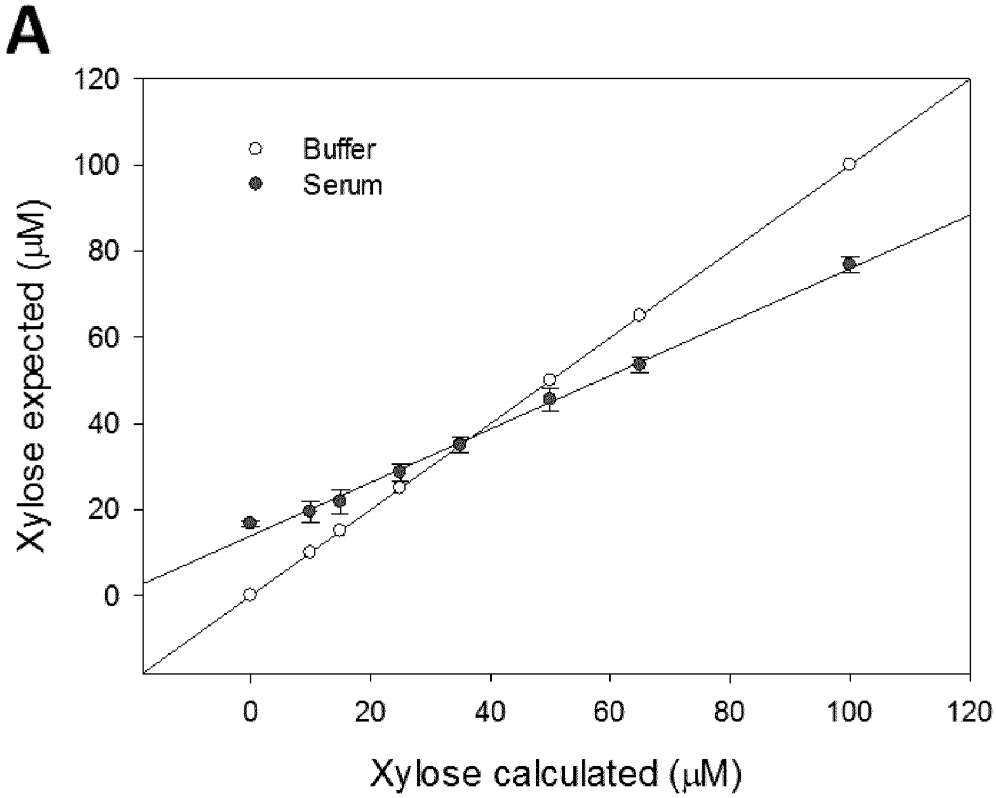
A
Buffer
Serum
Xylose expected (μM)
Xylose calculated (μM)
120
100
80
60
40
20
0
0
20
40
60
80
100
120

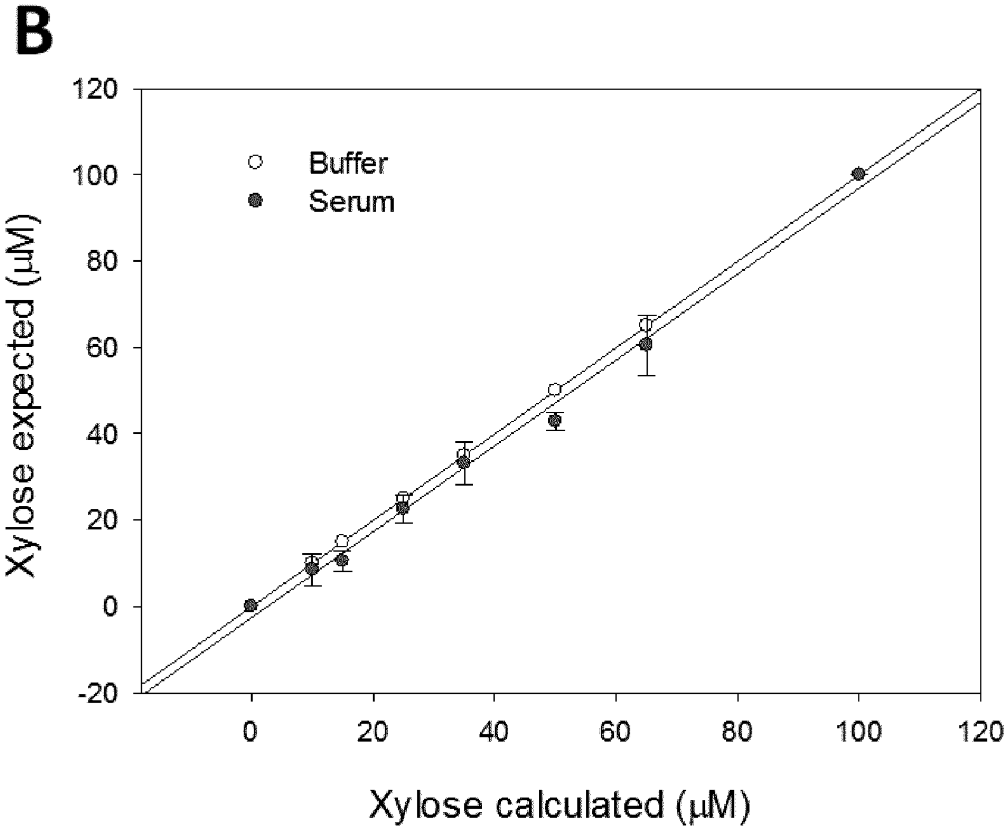
B
Buffer
Serum
Xylose expected (μM)
Xylose calculated (μM)
120
100
80
60
40
20
0
-20
0
20
40
60
80
100
120

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- ES 478590
- ES 482073
- ES 2023556
- ES 2100131
- ES 2182703
- ES 2208099
- US 742666
- US 350097
- US 62270825 W


### Non-patent literature cited in the description


- **ABDEL-GHANY SE ; DAY I ; HEUBERGER AL ; BROECKLING CD ; REDDY AS.** Metabolic engineering of Arabidopsis for butanetriol production using bacterial genes. *Metab Eng,* 2013, vol. 20, 109-20
- **AOKI S ; ISHIKURA S ; ASADA Y ; USAMI N ; HARA A.** Identity of dimeric dihydrodiol dehydrogenase as NADP+-dependent D-xylose dehydrogenase in pig liver. *Chem Biol Interact,* 2001, vol. 130-132, 775-84
- **ARAGÓN JJ ; HERMIDA C ; MARTINEZ-COSTA OH ; SANCHEZ V ; MARTIN I ; SANCHEZ JJ ; CODOCEO R ; CANO JM ; CANO A ; CRESPO L.** Noninvasive diagnosis of hypolactasia with 4-Galactosylxylose (Gaxilose): a multicentre, open-label, phase IIB-III nonrandomized trial. *J Clin Gastroenterol,* 2014, vol. 48, 29-36
- **AROLA H.** Diagnosis of hypolactasia and lactose malabsorption. *Scand J Gastroenterol,* 1994, vol. 202 (29), 22-35
- **ASADA Y ; AOKI S ; ISHIKURA S ; USAMI N ; HARA A.** Roles of His-79 and Tyr-180 of - xylose/dihydrodiol dehydrogenase in catalytic function. *Biochem Biophys Res Commun,* 2000, vol. 278, 333-7
- **BERGHÄLL S ; HILDITCH S ; PENTTILÄ M ; RICHARD P.** Identification in the mould Hypocrea jecorina of a gene encoding an NADP+: D-xylose dehydrogenase. *FEMS Microbiol Lett,* 2007, vol. 277, 249-53
- **CAO Y ; XIAN M ; ZOU H ; ZHANG H.** Metabolic engineering of Escherichia coli for the production of xylonate. *PLoS ONE,* 2013, vol. 8 (7), e67305
- **CHUN BW ; DAIR B ; MACUCH PJ ; WIEBE D ; PORTENEUVE C ; JEKNAVORIAN A.** The development of cement and concrete additive: based on xylonic acid derived via bioconversion of xylose. *Appl Biochem Biotech,* 2006, vol. 131, 645-58
- **DAVIDSON GP, ROBB TA.** Value of breath hydrogen analysis in management of diarrheal illness in childhood: Comparison with duodenal biopsy. *J Pediatr Gastroenterol Nutr,* 1985, vol. 4, 381-7
- **DAWSON DJ ; LOBLEY RW ; BURROWS PC ; MILLER V ; HOLMES R.** Lactose digestion by human jejunal biopsies: the relationship between hydrolysis and absorption. *Gut,* 1986, vol. 27, 521-7
- **FAN MZ ; STOLL B ; JIANG R ; BURRIN DG.** Enterocyte digestive enzyme activity along the crypt-villus and longitudinal axes in the neonatal pig small intestine. *J Anim Sci,* 2001, vol. 79, 371-81
- **FLEISS JL.** Statistical methods for rates and proportions. John Wiley, 38-46
- **HE Y ; CHUS S ; WALKER W.** A Nucleotide supplements alter proliferation and differentiation of cultured human (Caco-2) and rat (IEC-6) intestinal epithelial cells. *J Nutr,* 1993, vol. 123, 1017-17
- **HERMIDA C, GUERRA P ; MARTINEZ-COSTA OH ; SANCHEZ V ; SANCHEZ JJ ; SOLERA J ; FERNÁNDEZ-MAYORALAS A ; CODOCEO R ; FRIAS J ; ARAGÓN JJ.** Phase I and phase IB clinical trials for the noninvasive evaluation of intestinal lactase with 4-galactosylxylose (gaxilose). *J Clin Gastroenterol,* 2013, vol. 47, 501-8
- **HERMIDA C ; MARTINEZ-COSTA OH ; CORRALES G ; TERUEL C ; SANCHEZ V ; SANCHEZ JJ ; SARRION D ; ARIZA MJ ; CODOCEO R ; CALVO I.** Improvement and validation of D-Xylose determination in urine and serum as a new tool for the noninvasive evaluation of lactase activity in humans. *J Clin Lab Anal,* 2014, vol. 28, 478-86
- **HINNEBUSCH BF ; MA Q ; HENDERSON JW ; SIDDIQUE A ; ARCHER SY ; HODIN RA.** Enterocyte response to ischemia is dependent on differentiation state. *J Gastrointest Surg,* 2002, vol. 6, 403-9
- **JOHNSEN U ; SCHONHEIT P.** Novel xylose dehydrogenase in the halophilic archaeon Haloarcula marismortui. *J Bacteriol,* 2004, vol. 186, 6198-207

- **JOHNSEN U ; DAMBECK M ; ZAISS H ; FUHRER T ; SOPPA J ; SAUER U ; SCHONHEIT P.** D-Xylose degradation pathway in the halophilic archaeon Haloferax volcanii. *J Biol Chem,* 2009, vol. 284, 27290-303
- **KOETSE HA ; STELLAARD F ; BIJLEVELD CM ; ELZINGA H ; BOVERHOF R ; VAN DER MEER R ; VONK RJ ; SAUER PJ.** Non-invasive detection of low intestinal lactase activity in children by use of a combined CO2/H2 breath Test. Scand. *J Gastroenterol,* 1999, vol. 34, 35-40
- **LANDIS JR ; KOCH GG.** The measurement of observer agreement for categorical data. *Biometrics,* 1977, vol. 33, 159-74
- **LEVITT MD.** Production and excretion of hydrogen gas in man. *N Engl J Med,* 1969, vol. 281, 122-7
- **LI L ; LIANG B ; SHI J ; LI F ; MASCINI M ; LIU A.** A selective and sensitive D-xylose electrochemical biosensor based on xylose dehydrogenase displayed on the surface of bacteria and multi-walled carbon nanotubes modified electrode. *Biosens Bioelectron,* 2012, vol. 33, 100-5
- **LI L ; LI F ; SHI J ; MASCINI M ; LANG Q ; LIU A.** Co-immobilization of glucose oxidase and xylose dehydrogenase displayed whole cell on multiwalled carbon nanotube nanocomposite films modified electrode for simultaneous voltammetric detection of d-glucose and D-xylose. *Biosens Bioelectron,* 2013, vol. 42, 156-62
- **LIANG B ; LI L ; MASCIN M ; LIU A.** Construction of xylose dehydrogenase displayed on the surface of bacteria using ice nucleation protein for sensitive D-Xylose detection. *Anal Chem,* 2012, vol. 84, 275-82
- **LIU M. ; VALDEHUESA KN ; NISOLA GM ; RAMOS KR ; CHUNG WJ.** High yield production of D-xylonic acid from D-xylose using engineered Escherichia coli. *Bioresour Technol,* 2012, vol. 115, 244-8
- **LIFSHITZ CH ; BAUTISTA A ; GAPALACHRISHNA GS ; STUFF J ; GARZA C.** Absorption and tolerance of lactose in infants recovering from severe diarrhea. *J Pediatr Gastroenterol Nut.,* 1985, vol. 4, 942-8
- **MA J ; ZHONG L ; PENG X ; SUN R.** D-Xylonic acid: a solvent and an effective biocatalyst for a three-component reaction. *Green Chem,* 2016, vol. 18, 1738-50
- **MCGILL DB ; NEWCOMER AD.** Comparison of venous and capillary blood samples in lactose tolerance testing. *Gastroenterology,* 1967, vol. 53, 371-4
- **METZ G ; JENKINS DJ ; PETERS TJ ; NEWMAN A ; BLENDIS LM.** Breath hydrogen as a diagnostic method for hypolactasia. *Lancet,* 1975, vol. 1, 1155-7
- **MIHASAN M ; STEFAN M ; HRITCU L ; ARTENIE V ; BRANDSCH R.** Evidence of a plasmid-encoded oxidative xylose-catabolic pathway in Arthrobacter nicotinovorans pAO1. *Res Microbiol,* 2013, vol. 164, 22-30
- **NEWCOMER AD ; MCGILL DB.** Distribution of disaccharidase activity in the small bowel of normal and lactase-deficient subjects. *Gastroenterology,* 1966, vol. 51, 481-8
- **NEWCOMER AD ; MCGILL DB ; THOMAS PJ ; HOFMANN AF.** Prospective comparison of indirect methods for detecting lactase deficiency. *N Engl J Med,* 1975, vol. 293, 1232-6
- **NIEMINEN U ; KAHRI A ; SAVILAHTI E ; FÄRKKILÄ MA.** Duodenal disaccharidase activities in the follow-up of villous atrophy in coeliac disease. *Scand J Gastroenterol,* 2001, vol. 36, 507-10
- **NIU W ; MOLEFE MN ; FROST JW.** Microbial synthesis of the energetic material precursor 1,2,4-butanetriol. *J Am Chem Soc,* 2003, vol. 125, 12998-9
- **NYGÅRD Y ; TOIVARI MH ; PENTTILÄ M ; RUOHONEN L ; WIEBE MG.** Bioconversion of D-xylose to D-xylonate with Kluyveromyces lactis. *Metab Eng,* 2011, vol. 13, 383-91
- **NYGÅRD Y ; MAAHEIMO H ; MOJZITA D ; TOIVARI M ; WIEBE M ; RESNEKOV O ; GUSTAVO PESCE C ; RUOHONEN L ; PENTTILA M.** Single cell and in vivo analyses elucidate the effect of xylC lactonase during production of D-xylonate in Saccharomyces cerevisiae. *Metab Eng,* 2014, vol. 25, 238-47
- **PARFENOV AI ; AKHMADULLINA OV ; SABELN'NIKOVA EA ; BELOSTOSKII NI ; GUDKOVA RB ; KHOMERIKI SG.** Carbohydrase activities may serve as a marker for small intestinal mucosal recovery in patients with celiac disease. *Ter Arkh,* 2015, vol. 87, 24-9
- **SASAKI Y ; IIO M ; KAMEDA H ; UEDA H ; AOYAGI T.** Measurement of C-lactose absorption in the diagnosis of lactase deficiency. *J Lab Clin Med,* 1970, vol. 76, 824-35
- **SATO N ; NAKANO T ; KAWAKAMI H ; IDOTA T.** In vitro and in vivo effects of exogenous nucleotides on the proliferation and maturation of intestinal epithelial cells. *J Nutr Sci Vitaminol (Tokyo),* 1999, vol. 45, 107-18
- The Online Metabolic and Molecular Bases of Inherited Disease. **SEMENZA G ; AURICCHIO S ; MANTEI N.** Carbohydrates. Chapter 75: Small-Intestinal Disaccharidases. McGraw-Hill, 2006, 1-62
- Small-intestinal disaccharidases [Review. **SEMENZA G ; AURICCHIO S ; MANTEI N.** Scriver's Online Metabolic and Molecular Bases of Inherited Disease. McGraw-Hill, 2006

- **STEPHENS C ; CHRISTEN, B ; FUCHS T ; SUNDARAM V ; WATANABE K ; JENAL U.** Genetic analysis of a novel pathway for D-Xylose metabolism in C. crescentus. *J Bacteriol,* 2007, vol. 189, 2181-5
- **TOIVARI MH ; RUOHONEN L ; RICHARD P ; PENTTILÄ M ; WIEBE MG.** Saccharomyces cerevisiae engineered to produce D-xylonate. *Appl Microbiol Biotechnol,* 2010, vol. 88, 751-60
- **TOIVARI M ; NYGÅRD Y ; KUMPULA EP ; VEHKOMÄKI ML ; BENČINA M ; VALKONEN M. ; MAAHEIMO H. ; ANDBERG M ; KOIVULA A ; RUOHONEN L.** Metabolic engineering of Saccharomyces cerevisiae for bioconversion of D-xylose to D-xylonate. *Metab Eng,* 2012, vol. 14, 427-36
- **TOIVARI MH ; NYGÅRD Y ; PENTTILÄ M ; RUOHONEN L ; WIEBE MG.** Microbial D-xylonate production. *Appl Microbiol Biotechnol,* 2012, vol. 96, 1-8
- **TOIVARI MH ; VEHKOMÄKI ML ; NYGÅRD Y ; PENTTILÄ M ; RUOHONEN L ; WIEBE MG.** Low pH D-xylonate production with Pichia kudriavzevii. *Bioresource Technology,* 2013, vol. 133, 555-62
- **TRIADOU N ; BATAILLE J ; SCHMITZ J.** Longitudinal study of the human intestinal brush border membrane proteins distribution of the main disaccharidases and peptidases. *Gastroenterology,* 1983, vol. 85, 1326-32
- **UEHARA K ; TAKEDA M.** L-Xylose dehydrogenase in baker's yeast. *J Biochem.,* 1962, vol. 52, 461-3
- **VALDEHUESA KNG ; LIU H ; RAMOS KRM ; PARK SJ ; NISOLA W ; LEE K ; CHUNG J.** Direct bioconversion of D-xylose to 1, -2, -4-butanetriol in an engineered Escherichia coli. *Process Biochem,* 2014, vol. 49, 25-32
- **XIA L ; LIANG B ; LI L ; TANG X ; PALCHETTI I ; MASCINI M ; LIU A.** Direct energy conversion from xylose using xylose dehydrogenase surface displayed bacteria based enzymatic biofuel cell. *Biosens Bioelectron,* 2013, vol. 44, 160-3
- **ZAMORA F ; BUENO M ; MOLINA I ; IRIBARREN JI ; MUNOZ-GUERRA S ; GALBIS JA.** *Macromolecules,* 2000, vol. 33, 2030-38

SEQUENCE LISTING

<110> Venter Pharma

<120> ENZYMATIC METHOD FOR THE EVALUATION OF XYLOSE

<130> PCT-07448

<150> PCT/US62/270825
<151> 0028-06-06

<160> 8

<170> BiSSAP 1.3.6

<210> 1
<211> 660
<212> DNA
<213> Caulobacter crescentus NA1000

<220>
<223> xylB

<400> 1

```
atgtcctcag ccatctatcc cagcctgaag ggcaagcgcg tcgtcatcac cggcggcggc      60
tcgggcatcg gggccggcct caccgccggc ttcgcccgtc agggcgcgga ggtgatcttc     120
ctcgacatcg ccgacgagga ctccagggct cttgaggccg agctggccgg ctcgccgatc     180
ccgccggtct acaagcgctg cgacctgatg aacctcgagg cgatcaaggc ggtcttcgcc     240
gagatcggcg acgtcgacgt gctggtcaac aacgccggca atgacgaccg ccacaagctg     300
gccgacgtga ccggcgccta ttgggacgag cggatcaacg tcaacctgcg ccacatgctg     360
ttctgcaccc aggccgtcgc gccgggcatg aagaagcgtg gcggcggggc ggtgatcaac     420
ttcggttcga tcagctggca cctggggctt gaggacctcg tcctctacga aaccgccaag     480
gccggcatcg aaggcatgac ccgcgcgctg gcccgggagc tgggtcccga cgacatccgc     540
gtcacctgcg tggtgccggg caacgtcaag accaagcgcc aggagaagtg gtacacgccc     600
gaaggcgagg cccagatcgt ggcggcccaa tgcctgaagg gccgcatcgt cccggagaac     660
```

<210> 2
<211> 248
<212> PRT
<213> Caulobacter crescentus NA1000

<220>
<223> Xylose dehydrogenase XylB

<400> 2

```
Met Ser Ser Ala Ile Tyr Pro Ser Leu Lys Gly Lys Arg Val Val Ile
1               5                   10                  15
Thr Gly Gly Gly Ser Gly Ile Gly Ala Gly Leu Thr Ala Gly Phe Ala
            20                  25                  30
Arg Gln Gly Ala Glu Val Ile Phe Leu Asp Ile Ala Asp Glu Asp Ser


        35                  40                  45
Arg Ala Leu Glu Ala Glu Leu Ala Gly Ser Pro Ile Pro Pro Val Tyr
    50                  55                  60
Lys Arg Cys Asp Leu Met Asn Leu Glu Ala Ile Lys Ala Val Phe Ala
65                  70                  75                  80
Glu Ile Gly Asp Val Asp Val Leu Val Asn Asn Ala Gly Asn Asp Asp
                85                  90                  95
Arg His Lys Leu Ala Asp Val Thr Gly Ala Tyr Trp Asp Glu Arg Ile
            100                 105                 110
Asn Val Asn Leu Arg His Met Leu Phe Cys Thr Gln Ala Val Ala Pro
        115                 120                 125
Gly Met Lys Lys Arg Gly Gly Gly Ala Val Ile Asn Phe Gly Ser Ile
    130                 135                 140
Ser Trp His Leu Gly Leu Glu Asp Leu Val Leu Tyr Glu Thr Ala Lys
145                 150                 155                 160
Ala Gly Ile Glu Gly Met Thr Arg Ala Leu Ala Arg Glu Leu Gly Pro
                165                 170                 175
Asp Asp Ile Arg Val Thr Cys Val Val Pro Gly Asn Val Lys Thr Lys
            180                 185                 190
Arg Gln Glu Lys Trp Tyr Thr Pro Glu Gly Glu Ala Gln Ile Val Ala
        195                 200                 205
Ala Gln Cys Leu Lys Gly Arg Ile Val Pro Glu Asn Val Ala Ala Leu
    210                 215                 220
Val Leu Phe Leu Ala Ser Asp Asp Ala Ser Leu Cys Thr Gly His Glu
225                 230                 235                 240
Tyr Trp Ile Asp Ala Gly Trp Arg
                245
```

<210> 3
<211> 747
<212> DNA
<213> Artificial Sequence

<220>
<223> Gene xylB from C. crescentus strain NA1000 codon optimized

<400> 3

```
atgtcctctg cgatttatcc gagtctgaaa ggcaaacgtg tggtgattac gggtggtggt       60
tctggtattg gtgctggtct gacggcgggt tttgcgcgtc agggtgccga agtgattttc      120
ctggatatcg cggatgaaga cagtcgcgca ctggaagctg aactggcagg tagcccgatt      180
ccgccggtct ataaacgttg cgatctgatg aacctggaag ccattaaagc agtttttgct      240
gaaatcggtg atgtcgacgt gctggttaac aatgcaggca atgatgaccg ccataaactg      300
gcagatgtta ccggtgctta ctgggacgaa cgtatcaacg tcaatctgcg ccacatgctg      360
ttttgtacgc aggcggtggc cccgggcatg aaaaaacgtg gcggtggcgc cgttattaac      420
ttcggtagca tctcttggca tctgggcctg gaagatctgg tgctgtatga aaccgcaaaa      480
gctggtattg agggtatgac gcgtgcactg gcacgtgaac tgggtccgga tgacatccgt      540
gtcacctgcg tggttccggg caacgtgaaa accaaacgcc aagaaaaatg gtacacgccg      600
gaaggtgaag cccagattgt tgcggcccaa tgtctgaaag gccgcatcgt cccggaaaat      660
gtggcagctc tggttctgtt cctggcgtcg gatgatgcgt cgctgtgtac gggtcacgaa      720
tactggattg atgcgggctg gcgttga                                          747
```

<210> 4
<211> 764
<212> DNA
<213> Artificial Sequence

<220>
<223> xylB synthetic gene, including restriction sites for the endonucleases NcoI, NdeI and HindIII

<400> 4

ccatgggcca tatgtcctct gcgatttatc cgagtctgaa aggcaaacgt gtggtgatta 60
cgggtggtgg ttctggtatt ggtgctggtc tgacggcggg ttttgcgcgt cagggtgccg 120
aagtgatttt cctggatatc gcggatgaag acagtcgcgc actggaagct gaactggcag 180
gtagcccgat tccgccggtc tataaacgtt gcgatctgat gaacctggaa gccattaaag 240
cagtttttgc tgaaatcggt gatgtcgacg tgctggttaa caatgcaggc aatgatgacc 300
gccataaact ggcagatgtt accggtgctt actgggacga acgtatcaac gtcaatctgc 360
gccacatgct gttttgtacg caggcggtgg ccccgggcat gaaaaaacgt ggcggtggcg 420
ccgttattaa cttcggtagc atctcttggc atctgggcct ggaagatctg gtgctgtatg 480
aaaccgcaaa agctggtatt gagggtatga cgcgtgcact ggcacgtgaa ctgggtccgg 540
atgacatccg tgtcacctgc gtggttccgg gcaacgtgaa aaccaaacgc caagaaaaat 600
ggtacacgcc ggaaggtgaa gcccagattg ttgcggccca atgtctgaaa ggccgcatcg 660
tcccggaaaa tgtggcagct ctggttctgt tcctggcgtc ggatgatgcg tcgctgtgta 720
cgggtcacga atactggatt gatgcgggct ggcgttgaaa gctt 764

<210> 5
<211> 251
<212> PRT
<213> Artificial Sequence

<220>
<223> Sequence of the protein XylB-wt

<400> 5

Met Gly His Met Ser Ser Ala Ile Tyr Pro Ser Leu Lys Gly Lys Arg
1 5 10 15
Val Val Ile Thr Gly Gly Gly Ser Gly Ile Gly Ala Gly Leu Thr Ala
20 25 30
Gly Phe Ala Arg Gln Gly Ala Glu Val Ile Phe Leu Asp Ile Ala Asp
35 40 45
Glu Asp Ser Arg Ala Leu Glu Ala Glu Leu Ala Gly Ser Pro Ile Pro
50 55 60
Pro Val Tyr Lys Arg Cys Asp Leu Met Asn Leu Glu Ala Ile Lys Ala
65 70 75 80
Val Phe Ala Glu Ile Gly Asp Val Asp Val Leu Val Asn Asn Ala Gly
85 90 95
Asn Asp Asp Arg His Lys Leu Ala Asp Val Thr Gly Ala Tyr Trp Asp

```
                    100                     105                     110
Glu Arg Ile Asn Val Asn Leu Arg His Met Leu Phe Cys Thr Gln Ala
                115                     120                     125
Val Ala Pro Gly Met Lys Lys Arg Gly Gly Gly Ala Val Ile Asn Phe
            130                     135                     140
Gly Ser Ile Ser Trp His Leu Gly Leu Glu Asp Leu Val Leu Tyr Glu
145                     150                     155                     160
Thr Ala Lys Ala Gly Ile Glu Gly Met Thr Arg Ala Leu Ala Arg Glu
                    165                     170                     175
Leu Gly Pro Asp Asp Ile Arg Val Thr Cys Val Val Pro Gly Asn Val
                180                     185                     190
Lys Thr Lys Arg Gln Glu Lys Trp Tyr Thr Pro Glu Gly Glu Ala Gln
            195                     200                     205
Ile Val Ala Ala Gln Cys Leu Lys Gly Arg Ile Val Pro Glu Asn Val
        210                     215                     220
Ala Ala Leu Val Leu Phe Leu Ala Ser Asp Asp Ala Ser Leu Cys Thr
225                     230                     235                     240
Gly His Glu Tyr Trp Ile Asp Ala Gly Trp Arg
                245                     250
```

<210> 6
<211> 268
<212> PRT
<213> Artificial Sequence

<220>
<223> Sequence of the protein XylB-6His

<400> 6

```
Met Gly Ser Ser His His His His His His Ser Ser Gly Leu Val Pro
1               5                   10                  15
Arg Gly Ser His Met Ser Ser Ala Ile Tyr Pro Ser Leu Lys Gly Lys
            20                  25                  30
Arg Val Val Ile Thr Gly Gly Gly Ser Gly Ile Gly Ala Gly Leu Thr
        35                  40                  45
Ala Gly Phe Ala Arg Gln Gly Ala Glu Val Ile Phe Leu Asp Ile Ala
    50                  55                  60
Asp Glu Asp Ser Arg Ala Leu Glu Ala Glu Leu Ala Gly Ser Pro Ile
65                  70                  75                  80
Pro Pro Val Tyr Lys Arg Cys Asp Leu Met Asn Leu Glu Ala Ile Lys
                85                  90                  95
Ala Val Phe Ala Glu Ile Gly Asp Val Asp Val Leu Val Asn Asn Ala
            100                 105                 110
Gly Asn Asp Asp Arg His Lys Leu Ala Asp Val Thr Gly Ala Tyr Trp
        115                 120                 125
Asp Glu Arg Ile Asn Val Asn Leu Arg His Met Leu Phe Cys Thr Gln
    130                 135                 140
Ala Val Ala Pro Gly Met Lys Lys Arg Gly Gly Gly Ala Val Ile Asn
145                 150                 155                 160
Phe Gly Ser Ile Ser Trp His Leu Gly Leu Glu Asp Leu Val Leu Tyr
                165                 170                 175
Glu Thr Ala Lys Ala Gly Ile Glu Gly Met Thr Arg Ala Leu Ala Arg
            180                 185                 190
Glu Leu Gly Pro Asp Asp Ile Arg Val Thr Cys Val Val Pro Gly Asn
        195                 200                 205
Val Lys Thr Lys Arg Gln Glu Lys Trp Tyr Thr Pro Glu Gly Glu Ala
    210                 215                 220
Gln Ile Val Ala Ala Gln Cys Leu Lys Gly Arg Ile Val Pro Glu Asn
225                 230                 235                 240
Val Ala Ala Leu Val Leu Phe Leu Ala Ser Asp Asp Ala Ser Leu Cys
                245                 250                 255
Thr Gly His Glu Tyr Trp Ile Asp Ala Gly Trp Arg


                    260                 265
```

<210> 7
<211> 6004
<212> DNA
<213> Artificial Sequence

<220>
<223> pET-xylB-wt

<400> 7

```
tggcgaatgg gacgcgccct gtagcggcgc attaagcgcg gcgggtgtgg tggttacgcg      60
cagcgtgacc gctacacttg ccagcgccct agcgcccgct cctttcgctt tcttcccttc     120
ctttctcgcc acgttcgccg gctttccccg tcaagctcta aatcgggggc tccctttagg     180
gttccgattt agtgctttac ggcacctcga ccccaaaaaa cttgattagg gtgatggttc     240
acgtagtggg ccatcgccct gatagacggt ttttcgccct ttgacgttgg agtccacgtt     300
ctttaatagt ggactcttgt tccaaactgg aacaacactc aaccctatct cggtctattc     360
ttttgattta taagggattt tgccgatttc ggcctattgg ttaaaaaatg agctgattta     420
acaaaaattt aacgcgaatt ttaacaaaat attaacgttt acaatttcag gtggcacttt     480
tcggggaaat gtgcgcggaa cccctatttg tttatttttc taaatacatt caaatatgta     540
tccgctcatg aattaattct tagaaaaact catcgagcat caaatgaaac tgcaatttat     600
tcatatcagg attatcaata ccatattttt gaaaaagccg tttctgtaat gaaggagaaa     660
actcaccgag gcagttccat aggatggcaa gatcctggta tcggtctgcg attccgactc     720
gtccaacatc aatacaacct attaatttcc cctcgtcaaa aataaggtta tcaagtgaga     780
aatcaccatg agtgacgact gaatccggtg agaatggcaa aagtttatgc atttctttcc     840
agacttgttc aacaggccag ccattacgct cgtcatcaaa atcactcgca tcaaccaaac     900
cgttattcat tcgtgattgc gcctgagcga gacgaaatac gcgatcgctg ttaaaaggac     960
aattacaaac aggaatcgaa tgcaaccggc gcaggaacac tgccagcgca tcaacaatat    1020
tttcacctga atcaggatat tcttctaata cctggaatgc tgttttcccg gggatcgcag    1080
tggtgagtaa ccatgcatca tcaggagtac ggataaaatg cttgatggtc ggaagaggca    1140
taaattccgt cagccagttt agtctgacca tctcatctgt aacatcattg gcaacgctac    1200
ctttgccatg tttcagaaac aactctggcg catcgggctt cccatacaat cgatagattg    1260
tcgcacctga ttgcccgaca ttatcgcgag cccatttata cccatataaa tcagcatcca    1320
tgttggaatt taatcgcggc ctagagcaag acgtttcccg ttgaatatgg ctcataacac    1380
cccttgtatt actgtttatg taagcagaca gttttattgt tcatgaccaa aatcccttaa    1440
cgtgagtttt cgttccactg agcgtcagac cccgtagaaa agatcaaagg atcttcttga    1500
gatccttttt ttctgcgcgt aatctgctgc ttgcaaacaa aaaaaccacc gctaccagcg    1560
```

gtggtttgtt tgccggatca agagctacca actctttttc cgaaggtaac tggcttcagc 1620
agagcgcaga taccaaatac tgtccttcta gtgtagccgt agttaggcca ccacttcaag 1680
aactctgtag caccgcctac atacctcgct ctgctaatcc tgttaccagt ggctgctgcc 1740
agtggcgata agtcgtgtct taccgggttg gactcaagac gatagttacc ggataaggcg 1800
cagcggtcgg gctgaacggg gggttcgtgc acacagccca gcttggagcg aacgacctac 1860
accgaactga gatacctaca gcgtgagcta tgagaaagcg ccacgcttcc cgaagggaga 1920
aaggcggaca ggtatccggt aagcggcagg gtcggaacag gagagcgcac gagggagctt 1980
ccagggggaa acgcctggta tctttatagt cctgtcgggt ttcgccacct ctgacttgag 2040
cgtcgatttt tgtgatgctc gtcagggggg cggagcctat ggaaaaacgc cagcaacgcg 2100
gcctttttac ggttcctggc cttttgctgg ccttttgctc acatgttctt tcctgcgtta 2160
tcccctgatt ctgtggataa ccgtattacc gcctttgagt gagctgatac cgctcgccgc 2220
agccgaacga ccgagcgcag cgagtcagtg agcgaggaag cggaagagcg cctgatgcgg 2280
tattttctcc ttacgcatct gtgcggtatt tcacaccgca tatatggtgc actctcagta 2340
caatctgctc tgatgccgca tagttaagcc agtatacact ccgctatcgc tacgtgactg 2400
ggtcatggct gcgccccgac acccgccaac acccgctgac gcgccctgac gggcttgtct 2460
gctcccggca tccgcttaca gacaagctgt gaccgtctcc gggagctgca tgtgtcagag 2520
gttttcaccg tcatcaccga aacgcgcgag gcagctgcgg taaagctcat cagcgtggtc 2580
gtgaagcgat tcacagatgt ctgcctgttc atccgcgtcc agctcgttga gtttctccag 2640
aagcgttaat gtctggcttc tgataaagcg ggccatgtta agggcggttt tttcctgttt 2700
ggtcactgat gcctccgtgt aagggggatt tctgttcatg gggtaatga taccgatgaa 2760
acgagagagg atgctcacga tacgggttac tgatgatgaa catgcccggt tactggaacg 2820
ttgtgagggt aaacaactgg cggtatggat gcggcgggac cagagaaaaa tcactcaggg 2880
tcaatgccag cgcttcgtta atacagatgt aggtgttcca cagggtagcc agcagcatcc 2940
tgcgatgcag atccggaaca taatggtgca gggcgctgac ttccgcgttt ccagacttta 3000
cgaaacacgg aaaccgaaga ccattcatgt tgttgctcag gtcgcagacg ttttgcagca 3060
gcagtcgctt cacgttcgct cgcgtatcgg tgattcattc tgctaaccag taaggcaacc 3120
ccgccagcct agccgggtcc tcaacgacag gagcacgatc atgcgcaccc gtggggccgc 3180
catgccggcg ataatggcct gcttctcgcc gaaacgtttg gtggcgggac cagtgacgaa 3240
ggcttgagcg agggcgtgca agattccgaa taccgcaagc gacaggccga tcatcgtcgc 3300
gctccagcga aagcggtcct cgccgaaaat gacccagagc gctgccggca cctgtcctac 3360
gagttgcatg ataaagaaga cagtcataag tgcggcgacg atagtcatgc cccgcgccca 3420

ccggaaggag ctgactgggt tgaaggctct caagggcatc ggtcgagatc ccggtgccta 3480
atgagtgagc taacttacat taattgcgtt gcgctcactg cccgctttcc agtcgggaaa 3540
cctgtcgtgc cagctgcatt aatgaatcgg ccaacgcgcg gggagaggcg gtttgcgtat 3600
tgggcgccag ggtggttttt cttttcacca gtgagacggg caacagctga ttgcccttca 3660
ccgcctggcc ctgagagagt tgcagcaagc ggtccacgct ggtttgcccc agcaggcgaa 3720
aatcctgttt gatggtggtt aacggcggga tataacatga gctgtcttcg gtatcgtcgt 3780
atcccactac cgagatatcc gcaccaacgc gcagcccgga ctcggtaatg gcgcgcattg 3840
cgcccagcgc catctgatcg ttggcaacca gcatcgcagt gggaacgatg ccctcattca 3900
gcatttgcat ggtttgttga aaaccggaca tggcactcca gtcgccttcc cgttccgcta 3960
tcggctgaat ttgattgcga gtgagatatt tatgccagcc agccagacgc agacgcgccg 4020
agacagaact taatgggccc gctaacagcg cgatttgctg gtgacccaat gcgaccagat 4080
gctccacgcc cagtcgcgta ccgtcttcat gggagaaaat aatactgttg atgggtgtct 4140
ggtcagagac atcaagaaat aacgccggaa cattagtgca ggcagcttcc acagcaatgg 4200
catcctggtc atccagcgga tagttaatga tcagcccact gacgcgttgc gcgagaagat 4260
tgtgcaccgc cgctttacag gcttcgacgc cgcttcgttc taccatcgac accaccacgc 4320
tggcacccag ttgatcggcg cgagatttaa tcgccgcgac aatttgcgac ggcgcgtgca 4380
gggccagact ggaggtggca acgccaatca gcaacgactg tttgcccgcc agttgttgtg 4440
ccacgcggtt gggaatgtaa ttcagctccg ccatcgccgc ttccactttt tcccgcgttt 4500
tcgcagaaac gtggctggcc tggttcacca cgcgggaaac ggtctgataa gagacaccgg 4560
catactctgc gacatcgtat aacgttactg gtttcacatt caccaccctg aattgactct 4620
cttccgggcg ctatcatgcc ataccgcgaa aggttttgcg ccattcgatg gtgtccggga 4680
tctcgacgct ctcccttatg cgactcctgc attaggaagc agcccagtag taggttgagg 4740
ccgttgagca ccgccgccgc aaggaatggt gcatgcaagg agatggcgcc caacagtccc 4800
ccggccacgg ggcctgccac catacccacg ccgaaacaag cgctcatgag cccgaagtgg 4860
cgagcccgat cttccccatc ggtgatgtcg gcgatatagg cgccagcaac cgcacctgtg 4920
gcgccggtga tgccggccac gatgcgtccg gcgtagagga tcgagatctc gatcccgcga 4980
aattaatacg actcactata ggggaattgt gagcggataa caattcccct ctagaaataa 5040
ttttgtttaa ctttaagaag gagatatacc atgggccata tgtcctctgc gatttatccg 5100
agtctgaaag gcaaacgtgt ggtgattacg ggtggtggtt ctggtattgg tgctggtctg 5160
acggcgggtt ttgcgcgtca gggtgccgaa gtgattttcc tggatatcgc ggatgaagac 5220
agtcgcgcac tggaagctga actggcaggt agcccgattc cgccggtcta taaacgttgc 5280
gatctgatga acctggaagc cattaaagca gtttttgctg aaatcggtga tgtcgacgtg 5340

ctggttaaca atgcaggcaa tgatgaccgc cataaactgg cagatgttac cggtgcttac 5400

tgggacgaac gtatcaacgt caatctgcgc cacatgctgt tttgtacgca ggcggtggcc 5460

ccgggcatga aaaaacgtgg cggtggcgcc gttattaact tcggtagcat ctcttggcat 5520

ctgggcctgg aagatctggt gctgtatgaa accgcaaaag ctggtattga gggtatgacg 5580

cgtgcactgg cacgtgaact gggtccggat gacatccgtg tcacctgcgt ggttccgggc 5640

aacgtgaaaa ccaaacgcca agaaaaatgg tacacgccgg aaggtgaagc ccagattgtt 5700

gcggcccaat gtctgaaagg ccgcatcgtc ccggaaaatg tggcagctct ggttctgttc 5760

ctggcgtcgg atgatgcgtc gctgtgtacg ggtcacgaat actggattga tgcgggctgg 5820

cgttgaaagc ttgcggccgc actcgagcac caccaccacc accactgaga tccggctgct 5880

aacaaagccc gaaaggaagc tgagttggct gctgccaccg ctgagcaata actagcataa 5940

ccccttgggg cctctaaacg ggtcttgagg ggttttttgc tgaaaggagg aactatatcc 6000

ggat 6004

<210> 8
<211> 6055
<212> DNA
<213> Artificial Sequence

<220>
<223> Plasmid pET-xylB-6His

<400> 8

```
tggcgaatgg gacgcgccct gtagcggcgc attaagcgcg gcgggtgtgg tggttacgcg      60
cagcgtgacc gctacacttg ccagcgccct agcgcccgct cctttcgctt tcttcccttc     120
ctttctcgcc acgttcgccg gctttccccg tcaagctcta aatcgggggc tccctttagg     180
gttccgattt agtgctttac ggcacctcga ccccaaaaaa cttgattagg gtgatggttc     240
acgtagtggg ccatcgccct gatagacggt ttttcgccct ttgacgttgg agtccacgtt     300
ctttaatagt ggactcttgt tccaaactgg aacaacactc aaccctatct cggtctattc     360
ttttgattta taagggattt tgccgatttc ggcctattgg ttaaaaaatg agctgattta     420
acaaaaattt aacgcgaatt ttaacaaaat attaacgttt acaatttcag gtggcacttt     480
tcggggaaat gtgcgcggaa cccctatttg tttatttttc taaatacatt caaatatgta     540
tccgctcatg aattaattct tagaaaaact catcgagcat caaatgaaac tgcaatttat     600
tcatatcagg attatcaata ccatattttt gaaaaagccg tttctgtaat gaaggagaaa     660
actcaccgag gcagttccat aggatggcaa gatcctggta tcggtctgcg attccgactc     720
gtccaacatc aatacaacct attaatttcc cctcgtcaaa aataaggtta tcaagtgaga     780
aatcaccatg agtgacgact gaatccggtg agaatggcaa aagtttatgc atttctttcc     840
```

agacttgttc aacaggccag ccattacgct cgtcatcaaa atcactcgca tcaaccaaac 900
cgttattcat tcgtgattgc gcctgagcga gacgaaatac gcgatcgctg ttaaaaggac 960
aattacaaac aggaatcgaa tgcaaccggc gcaggaacac tgccagcgca tcaacaatat 1020
tttcacctga atcaggatat tcttctaata cctggaatgc tgttttcccg gggatcgcag 1080
tggtgagtaa ccatgcatca tcaggagtac ggataaaatg cttgatggtc ggaagaggca 1140
taaattccgt cagccagttt agtctgacca tctcatctgt aacatcattg gcaacgctac 1200
ctttgccatg tttcagaaac aactctggcg catcgggctt cccatacaat cgatagattg 1260
tcgcacctga ttgcccgaca ttatcgcgag cccatttata cccatataaa tcagcatcca 1320
tgttggaatt taatcgcggc ctagagcaag acgtttcccg ttgaatatgg ctcataacac 1380
cccttgtatt actgtttatg taagcagaca gttttattgt tcatgaccaa aatcccttaa 1440
cgtgagtttt cgttccactg agcgtcagac cccgtagaaa agatcaaagg atcttcttga 1500
gatccttttt ttctgcgcgt aatctgctgc ttgcaaacaa aaaaaccacc gctaccagcg 1560
gtggtttgtt tgccggatca agagctacca actctttttc cgaaggtaac tggcttcagc 1620
agagcgcaga taccaaatac tgtccttcta gtgtagccgt agttaggcca ccacttcaag 1680
aactctgtag caccgcctac atacctcgct ctgctaatcc tgttaccagt ggctgctgcc 1740
agtggcgata agtcgtgtct taccgggttg gactcaagac gatagttacc ggataaggcg 1800
cagcggtcgg gctgaacggg gggttcgtgc acacagccca gcttggagcg aacgacctac 1860
accgaactga gatacctaca gcgtgagcta tgagaaagcg ccacgcttcc cgaagggaga 1920
aaggcggaca ggtatccggt aagcggcagg gtcggaacag gagagcgcac gagggagctt 1980
ccagggggaa acgcctggta tctttatagt cctgtcgggt ttcgccacct ctgacttgag 2040
cgtcgatttt tgtgatgctc gtcagggggg cggagcctat ggaaaaacgc cagcaacgcg 2100
gcctttttac ggttcctggc cttttgctgg ccttttgctc acatgttctt tcctgcgtta 2160
tcccctgatt ctgtggataa ccgtattacc gcctttgagt gagctgatac cgctcgccgc 2220
agccgaacga ccgagcgcag cgagtcagtg agcgaggaag cggaagagcg cctgatgcgg 2280
tattttctcc ttacgcatct gtgcggtatt tcacaccgca tatatggtgc actctcagta 2340
caatctgctc tgatgccgca tagttaagcc agtatacact ccgctatcgc tacgtgactg 2400
ggtcatggct gcgccccgac acccgccaac acccgctgac gcgccctgac gggcttgtct 2460
gctcccggca tccgcttaca gacaagctgt gaccgtctcc gggagctgca tgtgtcagag 2520
gttttcaccg tcatcaccga aacgcgcgag gcagctgcgg taaagctcat cagcgtggtc 2580
gtgaagcgat tcacagatgt ctgcctgttc atccgcgtcc agctcgttga gtttctccag 2640
aagcgttaat gtctggcttc tgataaagcg ggccatgtta agggcggttt tttcctgttt 2700

ggtcactgat gcctccgtgt aaggggggatt tctgttcatg ggggtaatga taccgatgaa 2760

acgagagagg atgctcacga tacgggttac tgatgatgaa catgcccggt tactggaacg 2820

ttgtgagggt aaacaactgg cggtatggat gcggcgggac cagagaaaaa tcactcaggg 2880

tcaatgccag cgcttcgtta atacagatgt aggtgttcca cagggtagcc agcagcatcc 2940

tgcgatgcag atccggaaca taatggtgca gggcgctgac ttccgcgttt ccagacttta 3000

cgaaacacgg aaaccgaaga ccattcatgt tgttgctcag gtcgcagacg ttttgcagca 3060

gcagtcgctt cacgttcgct cgcgtatcgg tgattcattc tgctaaccag taaggcaacc 3120

ccgccagcct agccgggtcc tcaacgacag gagcacgatc atgcgcaccc gtggggccgc 3180

catgccggcg ataatggcct gcttctcgcc gaaacgtttg gtggcgggac cagtgacgaa 3240

ggcttgagcg agggcgtgca agattccgaa taccgcaagc gacaggccga tcatcgtcgc 3300

gctccagcga aagcggtcct cgccgaaaat gacccagagc gctgccggca cctgtcctac 3360

gagttgcatg ataaagaaga cagtcataag tgcggcgacg atagtcatgc cccgcgccca 3420

ccggaaggag ctgactgggt tgaaggctct caagggcatc ggtcgagatc ccggtgccta 3480

atgagtgagc taacttacat taattgcgtt gcgctcactg cccgctttcc agtcgggaaa 3540

cctgtcgtgc cagctgcatt aatgaatcgg ccaacgcgcg gggagaggcg gtttgcgtat 3600

tgggcgccag ggtggttttt cttttcacca gtgagacggg caacagctga ttgcccttca 3660

ccgcctggcc ctgagagagt tgcagcaagc ggtccacgct ggtttgcccc agcaggcgaa 3720

aatcctgttt gatggtggtt aacggcggga tataacatga gctgtcttcg gtatcgtcgt 3780

atcccactac cgagatatcc gcaccaacgc gcagcccgga ctcggtaatg gcgcgcattg 3840

cgcccagcgc catctgatcg ttggcaacca gcatcgcagt gggaacgatg ccctcattca 3900

gcatttgcat ggtttgttga aaaccggaca tggcactcca gtcgccttcc cgttccgcta 3960

tcggctgaat ttgattgcga gtgagatatt tatgccagcc agccagacgc agacgcgccg 4020

agacagaact taatgggccc gctaacagcg cgatttgctg gtgacccaat gcgaccagat 4080

gctccacgcc cagtcgcgta ccgtcttcat gggagaaaat aatactgttg atgggtgtct 4140

ggtcagagac atcaagaaat aacgccggaa cattagtgca ggcagcttcc acagcaatgg 4200

catcctggtc atccagcgga tagttaatga tcagcccact gacgcgttgc gcgagaagat 4260

tgtgcaccgc cgctttacag gcttcgacgc cgcttcgttc taccatcgac accaccacgc 4320

tggcacccag ttgatcggcg cgagatttaa tcgccgcgac aatttgcgac ggcgcgtgca 4380

gggccagact ggaggtggca acgccaatca gcaacgactg tttgcccgcc agttgttgtg 4440

ccacgcggtt gggaatgtaa ttcagctccg ccatcgccgc ttccactttt tcccgcgttt 4500

tcgcagaaac gtggctggcc tggttcacca cgcgggaaac ggtctgataa gagacaccgg 4560

catactctgc gacatcgtat aacgttactg gtttcacatt caccaccctg aattgactct 4620

```
cttccgggcg ctatcatgcc ataccgcgaa aggttttgcg ccattcgatg gtgtccggga      4680
tctcgacgct ctcccttatg cgactcctgc attaggaagc agcccagtag taggttgagg      4740
ccgttgagca ccgccgccgc aaggaatggt gcatgcaagg agatggcgcc caacagtccc      4800
ccggccacgg ggcctgccac catacccacg ccgaaacaag cgctcatgag cccgaagtgg      4860
cgagcccgat cttccccatc ggtgatgtcg gcgatatagg cgccagcaac cgcacctgtg      4920
gcgccggtga tgccggccac gatgcgtccg gcgtagagga tcgagatctc gatcccgcga      4980
aattaatacg actcactata ggggaattgt gagcggataa caattcccct ctagaaataa      5040
ttttgtttaa ctttaagaag gagatatacc atgggcagca gccatcatca tcatcatcac      5100
agcagcggcc tggtgccgcg cggcagccat atgtcctctg cgatttatcc gagtctgaaa      5160
ggcaaacgtg tggtgattac gggtggtggt tctggtattg gtgctggtct gacggcgggt      5220
tttgcgcgtc agggtgccga agtgattttc ctggatatcg cggatgaaga cagtcgcgca      5280
ctggaagctg aactggcagg tagcccgatt ccgccggtct ataaacgttg cgatctgatg      5340
aacctggaag ccattaaagc agtttttgct gaaatcggtg atgtcgacgt gctggttaac      5400
aatgcaggca atgatgaccg ccataaactg gcagatgtta ccggtgctta ctgggacgaa      5460
cgtatcaacg tcaatctgcg ccacatgctg ttttgtacgc aggcggtggc cccgggcatg      5520
aaaaaacgtg gcggtggcgc cgttattaac ttcggtagca tctcttggca tctgggcctg      5580
gaagatctgg tgctgtatga aaccgcaaaa gctggtattg agggtatgac gcgtgcactg      5640
gcacgtgaac tgggtccgga tgacatccgt gtcacctgcg tggttccggg caacgtgaaa      5700
accaaacgcc aagaaaaatg gtacacgccg gaaggtgaag cccagattgt tgcggcccaa      5760
tgtctgaaag gccgcatcgt cccggaaaat gtggcagctc tggttctgtt cctggcgtcg      5820
gatgatgcgt cgctgtgtac gggtcacgaa tactggattg atgcgggctg gcgttgaaag      5880
cttgcggccg cactcgagca ccaccaccac caccactgag atccggctgc taacaaagcc      5940
cgaaaggaag ctgagttggc tgctgccacc gctgagcaat aactagcata accccttggg      6000
gcctctaaac gggtcttgag gggttttttg ctgaaaggag gaactatatc cggat            6055
```